(19) Europäisches Patentamt
European Patent Office
Office européen des brevets

(11) Publication number: **0 234 119 B1**

(12) # EUROPEAN PATENT SPECIFICATION

(45) Date of publication of patent specification: **24.08.94**

(51) Int. Cl.$^5$: **A01N 43/56**, A61K 31/415, C07D 231/38, A01N 55/00, C07F 7/08, C07D 231/44, C07D 231/40, C07D 231/18, C07D 231/16, C07D 231/14, C07D 231/12

(21) Application number: **86309981.8**

(22) Date of filing: **19.12.86**

Divisional application 93115360.5 filed on 23/09/93.

(54) **Pesticidal method using N-phenylpyrazoles.**

(30) Priority: **20.12.85 GB 8531485**

(43) Date of publication of application:
**02.09.87 Bulletin 87/36**

(45) Publication of the grant of the patent:
**24.08.94 Bulletin 94/34**

(84) Designated Contracting States:
**AT BE CH DE ES FR GB GR IT LI LU NL SE**

(56) References cited:
**EP-A- 202 169**
**EP-A- 216 102**
**EP-A- 0 201 852**
**US-A- 2 998 419**
**US-A- 3 760 084**
**EP-A- 204 242**
**EP-A- 0 200 872**
**AU-A- 508 225**
**US-A- 3 423 424**
**US-A- 4 145 554**

(73) Proprietor: **RHONE-POULENC AGROCHIMIE**
**14-20, rue Pierre Baizet**
**F-69009 Lyon (FR)**

(72) Inventor: **Hatton, Leslie Roy**
**May & Baker Limited**
**Dagenham Essex RM10 7XS (GB)**
Inventor: **Hawkins, David William**
**May & Baker Limited**
**Dagenham Essex RM10 7XS (GB)**
Inventor: **Parnell, Edgar William**
**May & Baker Limited**
**Dagenham Essex RM10 7XS (GB)**
Inventor: **Pearson, Christopher John**
**May & Baker Limited**
**Dagenham Essex RM10 7XS (GB)**
Inventor: **Roberts, David Alan**
**May & Baker Limited**
**Dagenham Essex RM10 7XS (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

EP 0 234 119 B1

JOURNAL OF HETROCYCLIC CHEMISTRY, vol. 12, no. 12, December 1975, pages 1199-1205, Provo, UTAH, US; P.L. SOUTHWICK et al.: "Preparation of 4,6-diaminopyrazolo[3,4-d]pyrimidines with variations in substitution at the 1- and 3-positions(1)"

IL FARMACO ED. SCIENTIFICA, vol. XXXVIII, no. 4, April 1983, pages 274-282, Pavia, IT; P. GIORI et al.: "Synthesis and antifungal activity of pyrazole derivatives containing sulfurated functions"

CHEMICAL ABSTRACTS, vol. 91, no. 25, 17th December 1979, page 181, abstract no. 205651p, Columbus, Ohio, US; & JP-A-79 84 032 (ISHIHARA SANGYO KAISHA LTD)

(74) Representative: **Bentham, Stephen et al**
**J.A. KEMP & CO.**
**14 South Square**
**Gray's Inn**
**London WC1R 5LX (GB)**

## Description

This invention relates to the use of N-phenylpyrazole derivatives against arthropod, plant nematode and helminth pests, to compositions containing them and to novel N-phenylpyrazole derivatives.

In J. Heter. Chem., 12 (1975), 1199-1205, P.L. Southwick and B. Dhawan have described experiments for the preparation of 4,6-diamino-pyrazolo[3,4-d]pyrimidines in the expectation that such pyrimidine derivatives would have useful pharmacological properties. They employed as starting materials 5-amino-4-cyanopyrazoles carrying on the 1-position a hydrogen atom, a methyl group, a hydroxyethyl group or a phenyl group substituted by one or more chlorine atoms and/or methyl groups, and on the 3-position a hydrogen atom, a methyl group or a phenyl or benzyl group. This publication contains no suggestion that compounds of general formula I possess or would be expected to possess activity against arthropods, helminths or plant nematodes.

Apparently these pyrazole compounds did not lead (according to the authors of the article) to useful therapeutic (viz. antimalarial) 4,6-diaminopyrazolo[3,4-d]pyrimidines.

US Patent No. 3760084 describes certain 5-amino-1-phenyl-pyrazoles as being useful for ameliorating inflammation in warm-blooded animals: the compounds carry on the 3-position hydrogen or a lower alkyl group and on the 4-position a carbamoyl or cyano group.

US Patent No. 3869274 describes certain 4-nitropyrazoles as being useful for the induction of abscission of fruit from fruit-bearing plants.

US Patent No. 4066776 describes a very extensive group of 1,4-disubstituted-3-nitropyrazoles as having antimicrobial, parasiticidal and herbicidal properties: the great biological activity of the compounds is stated to be limited to the 3-nitropyrazoles disclosed, the characterising feature of the compounds being the 3-nitropyrazole nucleus.

Japanese Patent Publication No. 12644/64 describes a process for the preparation of 4-thiocyanatopyrazole derivatives which are stated to be useful as germicides.

Japanese Patent Publication No. 49-117502 describes certain pyrazole sulphonamides having anti-thrombogenic properties.

None of the foregoing publications describes or suggests that compounds of general formula I possess or would be expected to possess the activity against arthropods, helminths or plant nematodes which has been discovered by the Applicants.

EP-A-0 201 852 (Bayer) describes, as insecticides, acaricides and nematocides, 5-aminopyrazoles of the general formula:

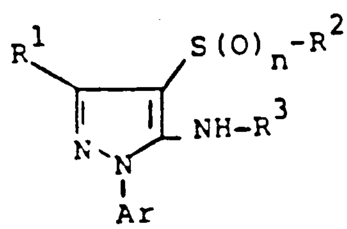

wherein $R^1$ represents hydrogen, alkyl or halogenoalkyl;

$R^2$ represents, inter alia, alkyl or halogenoalkyl;

$R^3$ represents hydrogen, alkyl or a group $-C(X)R^4$ in which $R^4$ represents hydrogen, alkyl, alkenyl and various other groups and X represents oxygen or sulphur.

EP-A-0 200872 (Bayer) describes, as herbicides and insecticides, compounds of the general formula:

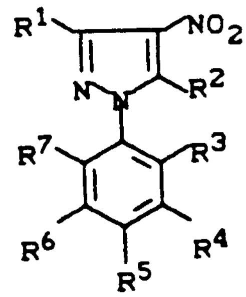

wherein $R^1$ and $R^2$ independently represent, inter alia, hydrogen, alkyl and cycloalkyl, and $R^3$, $R^4$, $R^5$, $R^6$ and $R^7$ represent, independently, hydrogen, halogen, cyano, nitro, alkyl, alkoxy, alkylthio, alkylsulphinyl, alkylsulphonyl, alkoxycarbonyl or a group $-(X)_n-R^8$ in which X represents oxygen, sulphur, sulphinyl or sulphonyl and n is 0 or 1 and $R^8$ represents halogenoalkyl.

EP-A-0 216102 describes, as insecticides, acaricides and nematocides compounds of the general formula:

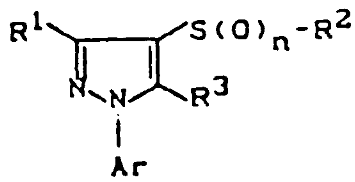

wherein $R^1$ represents hydrogen, alkyl or halogenoalkyl; $R^2$ represents alkyl, halogenoalkyl or optionally substituted aralkyl or aryl, $R^3$ represents hydrogen or halogen and Ar represents optionally substituted phenyl or pyridyl.

EP-A-0 201852 and EP-A-0 200872 were published before the present European application was filed but after the date from which it claims priority.

The present invention does not embrace the uses of chemical species disclosed and characterised in the applications from which EP-A-0 201852 and EP-A-0 200872 claim priority.

EP-A-0 216102 was filed before the present European application but after the date from which it claims priority. The present invention does not embrace the uses of chemical species disclosed and characterised in the application from which EP-A-0 216102 claims priority.

It has now unexpectedly been found after extensive research and experimentation that the N-phenylpyrazole derivatives of the general formula I depicted hereinafter wherein Y represents a halogen, i.e. fluorine, chlorine, bromine or iodine, atom, the cyano or nitro group or a group $RSO_2$, RSO or RS in which R represents a straight- or branched-chain alkyl group containing from 1 to 6 carbon atoms which is unsubstituted or substituted by one or more halogen atoms, a cycloalkyl group containing from 3 to 5 carbon atoms, a straight- or branched-chain alkenyl group containing from 2 to 6 carbon atoms, or Y represents the thiocyanato group, the sulphamoyl group which is unsubstituted or substituted by one or two straight- or branched-chain alkyl groups which may be the same or different and contain from 1 to 6 carbon atoms, the carbamoyl group which may be unsubstituted or substituted by one or two straight- or branched-chain alkyl groups which may be the same or different and contain from 1 to 6 carbon atoms, a straight- or branched-chain alkoxycarbonyl group containing from 2 to 7 carbon atoms, a straight- or branched-chain alkanoyl group containing from 2 to 7 carbon atoms, or a straight- or branched-chain alkyl group containing from 1 to 6 carbon atoms which is unsubstituted or substituted by one or more halogen atoms,
Z represents the hydrogen atom, or the amino group $-NR^1R^2$ wherein $R^1$ and $R^2$, which may be the same or different, each represents a hydrogen atom or a straight- or branched-chain alkyl group (containing from 1 to 6 carbon atoms, and which is unsubstituted or substituted by straight- or branched-chain alkoxycarbonyl of 2 to 5 carbon atoms), cycloalkyl group containing from 3 to 6 carbon atoms, formyl group, straight- or branched-chain alkanoyl group (which contains from 2 to 7 carbon atoms or together form a 5 or 6 membered cyclic imide with the nitrogen atom to which they are attached and themselves are unsubstituted or substituted by one or more halogen atoms) or cycloalkylcarbonyl group (which contains from 4 to 7 carbon atoms) or straight- or branched-chain alkoxycarbonyl group (which contains from 2 to 7 carbon

atoms and themselves are unsubstituted or substituted by one or more halogen atoms), or Z represents a straight- or branched-chain alkylsulphenylamino group containing from 1 to 4 carbon atoms, a straight- or branched-chain alkoxymethyleneamino group containing from 2 to 5 carbon atoms which is unsubstituted or substituted on methylene by a straight- or branched-chain alkyl group containing from 1 to 4 carbon atoms, or represents a halogen, i.e. fluorine, chlorine, bromine or iodine, atom, a straight- or branched-chain alkyl group containing from 1 to 4 carbon atoms, the carboxy group, or a straight- or branched-chain alkylthio, alkylsulphinyl or alkylsulphonyl group containing from 1 to 6 carbon atoms which is unsubstituted or substituted by one or more halogen atoms, or represents a straight- or branched-chain trialkylsilylmethyl group containing from 1 to 6 carbon atoms in each alkyl group which may be the same or different, a trialkylsilyl group containing from 1 to 6 carbon atoms in each alkyl group which may be the same or different or the cyano or nitro group, $R^3$ represents a halogen, i.e. fluorine, chlorine, bromine or iodine atom, a straight- or branched-chain alkyl or alkoxy group containing from 1 to 4 carbon atoms which is unsubstituted or substituted by one or more halogen atoms, (e.g. a trifluoromethyl or trifluoromethoxy group), a straight- or branched-chain alkylthio or alkylsulphinyl group containing from 1 to 4 carbon atoms which is substituted by one or more halogen atoms (e.g. a trifluoromethylthio or trifluoromethylsulphinyl group), the nitro or cyano group or a straight- or branched-chain alkylsulphonyl group containing from 1 to 4 carbon atoms which is unsubstituted or substituted by one or more halogen atoms (e.g. the trifluoromethyl-sulphonyl group), and $R^4$ represents a halogen, i.e. fluorine, chlorine, bromine or iodine, atom, a cyano or nitro group, and $\underline{n}$ represents an integer from 1 to 5 inclusive, and, when Z represents a carboxy group, salts thereof with pesticidally-acceptable bases provided that (A) $R^4$, Y and Z do not simultaneously represent three groups of the same genus selected from the genera (i) nitro, (ii) cyano and (iii) halogen have valuable activity against arthropod, plant nematode and helminth pests, more particularly by ingestion of the compound(s) of general formula I by the arthropods. When $\underline{n}$ represents an integer from 2 to 5 inclusive, atoms and groups represented by $R^3$ may be the same or different.

By the term 'salts with pesticidally acceptable bases' is meant salts the cations of which are known and accepted in the art for the formation of salts of pesticidally active acids for agricultural or horticultural use. When intended for application to vertebrates to combat infection or infestation by arthropods or helminths, the salts with bases used will be non-toxic. By the term 'non-toxic' is meant salts with bases the cations of which are innocuous to the vertebrates at the doses administered and which do not vitiate the beneficial effects produced by the anion.

Preferably, the salts are water-soluble. Suitable salts with bases include alkali metal (e.g. sodium and potassium), alkaline earth metal (e.g. calcium and magnesium), ammonium and amine (e.g. diethanolamine, triethanolamine, octylamine, morpholine and dioctylmethylamine) salts. It is to be understood that where reference is made in the present specification to the compounds of general formula I such reference is intended to include also the salts with pesticidally acceptable bases of compounds of general formula I where appropriate.

Preferred compounds of general formula I are those with phenyl substitution which is 2,4,6-trichloro, 2,3,5,6-tetrachloro, 2-chloro-4-trifluoromethyl, 2,3,5,6-tetrafluoro-4-trifluoromethyl, 2,6-dichloro-4-trifluoromethylthio, 2-chloro-3,5,6-trifluoro-4-trifluoromethyl, 2,6-dichloro-3,5-difluoro-4-trifluoromethyl, 2,6-dichloro-4-nitro, 2,6-dichloro-4-trifluoromethylsulphinyl, 2,6-dichloro-4-methanesulphonyl and 2,6-dichloro-4-trifluoromethanesulphonyl.

Compounds of general formula I wherein $(R^3)n$ represents 2,6-dichloro-4-trifluoromethyl or 2,6-dichloro-4-trifluoromethoxy substitution of the phenyl group are especially preferred.

Preferred compounds are those where

(a) Y and $R^4$ each represent a cyano group and Z represents the hydrogen atom, the amino group $-NR^1R^2$ or an alkylsulphenylamino group, an alkoxymethyleneamino group which may be unsubstituted or substituted on methylene by an alkyl group, a halogen atom, an alkyl group, the carboxy group, an alkylthio, alkylsulphinyl or alkylsulphonyl group which is optionally halogen substituted, a trialkylsilylmethyl group, a trialkylsilyl group or the nitro group;

(b) Y represents an alkylsulphonyl group which is optionally halogen substituted, a cycloalkylsulphonyl group or an alkenylsulphonyl group, Z represents the hydrogen atom, the amino group $-NR^1R^2$ or an alkylsulphenylamino group, an alkoxymethyleneamino group which is unsubstituted or substituted on methylene by an alkyl group, a halogen atom, an alkyl group, the carboxy group, an alkylthio, alkylsulphinyl or alkylsulphonyl group which is optionally halogen substituted, a trialkylsilylmethyl group, a trialkylsilyl group or the cyano or nitro group and $R^4$ represents a halogen atom or the cyano or nitro group;

(c) $R^4$ represents the nitro group, Y represents the cyano or nitro group, a carbamoyl group or an alkoxycarbonyl group and

Z represents the hydrogen atom, a halogen atom, an alkyl group, the carboxy group, an alkylthio, alkylsulphinyl or alkylsulphonyl group which is optionally halogen substituted,
a trialkylsilylmethyl group, a trialkylsilyl group or the nitro group;
(d) $R^4$ represents a halogen atom, Y represents the cyano or nitro group, a carbamoyl group or an alkoxycarbonyl group and
Z represents the hydrogen atom, the amino group $-NR^1R^2$ or an alkylsulphenylamino group, an alkoxymethyleneamino group which is unsubstituted or substituted on methylene by an alkyl group, a halogen atom, an alkyl group, the carboxy group, an alkylthio, alkylsulphinyl or alkylsulphonyl group which is optionally halogen substituted, a trialkylsilylmethyl group, a trialkylsilyl group or the nitro group; and

It will be appreciated that the groups listed above are as hereinbefore defined earlier in the specification.

Compounds of general formula I which are of particular interest against arthropods are:
1 5-Amino-3,4-dicyano-1-(2,4,6-trichlorophenyl)pyrazole
2 5-Amino-1-(2,6-dichloro-4-trifluoromethylphenyl)-3,4-dicyanopyrazole
3 5-Amino-3,4-dicyano-1-(2,3,5,6-tetrachlorophenyl)pyrazole
6 5-Amino-3-chloro-4-cyano-1-(2,6-dichloro-4-trifluoromethylphenyl)pyrazole
7 5-Amino-3-bromo-4-cyano-1-(2,6-dichloro-4-trifluoromethylphenyl)pyrazole
8 5-Amino-3-iodo-4-cyano-1-(2,6-dichloro-4-trifluoromethylphenyl)pyrazole
12 5-Acetamido-1-(2,6-dichloro-4-trifluoromethylphenyl)-3,4-dicyanopyrazole
13 5-Dichloroacetamido-1-(2,6-dichloro-4-trifluoromethylphenyl)-3,4-dicyanopyrazole
14 5-Cyclopropylcarbonamido-1-(2,6-dichloro-4-trifluoromethylphenyl)-3,4-dicyanopyrazole
15 5-Pentanamido-1-(2,6-dichloro-4-trifluoromethylphenyl)-3,4-dicyanopyrazole
16 5-Propionamido-1-(2,6-dichloro-4-trifluoromethylphenyl)-3,4-dicyanopyrazole
17 5-Amino-1-(2-chloro-4-trifluoromethylphenyl)-3,4-dicyanopyrazole
18 5-Amino-3,4-dicyano-1-(2,3,5,6-tetrafluoro-4-trifluoromethylphenyl)pyrazole
26 1-(2,6-Dichloro-4-trifluoromethylphenyl)-3,4-dicyano-5-(2,2-dimethylpropionamido)pyrazole
48 5-Amino-3,4-dicyano-1-(2,6-dichloro-4-trifluoromethylthiophenyl)pyrazole
49 5-Amino-1-(2-chloro-3,5,6-trifluoro-4-trifluoromethylphenyl)-3,4-dicyanopyrazole,
50 5-Amino-1-(2,6-dichloro-3,5-difluoro-4-trifluoromethylphenyl)-3,4-dicyanopyrazole,
51 5-Amino-1-(2,6-dichloro-4-trifluoromethoxyphenyl)-3,4-dicyanopyrazole,
64 5-Amino-1-(2,6-dichloro-4-nitrophenyl)-3,4-dicyanopyrazole
65 1-(2,6-Dichloro-4-trifluoromethylphenyl)-3,4-dicyano-5-methylaminopyrazole
66 1-(2,6-Dichloro-4-trifluoromethylphenyl)-3,4-dicyano-5-ethylaminopyrazole
72 3-Chloro-1-(2,6-dichloro-4-trifluoromethylphenyl)-4-cyano-5-trimethylacetylaminopyrazole
73 3-Chloro-1-(2,6-dichloro-4-trifluoromethylphenyl)-4-cyano-5-bis(ethoxycarbonyl)aminopyrazole
74 3-Chloro-1-(2,6-dichloro-4-trifluoromethylphenyl)-4-cyano-5-ethoxycarbonylaminopyrazole
77 1-(2,6-Dichloro-4-trifluoromethylphenyl)-5-bis(ethoxycarbonyl)amino-3,4-dicyanopyrazole
80 1-(2,6-Dichloro-4-trifluoromethylphenyl)-3,4-dicyano-5-ethoxycarbonylaminopyrazole
87 4-Cyano-1-(2,6-dichloro-4-trifluoromethylphenyl)-3-fluoropyrazole
92 1-(2,6-Dichloro-4-trifluoromethylphenyl)-4-cyano-3-nitropyrazole
93 1-(2,6-Dichloro-4-trifluoromethylphenyl)-3,4-dicyano-5-nitropyrazole
94 5-Amino-1-(2,6-dichloro-4-trifluoromethylphenyl)-4-cyano-3-fluoropyrazole
95 5-Amino-3-chloro-1-(2,6-dichloro-4-trifluoromethoxyphenyl)-4-cyanopyrazole
96 5-Amino-3-chloro-4-cyano-1-(2,6-dichloro-3,5-difluoro-4-trifluoromethylphenyl)pyrazole
106 5-Amino-3-cyano-1-(2,6-dichloro-4-trifluoromethylphenyl)-4-methanesulphonylpyrazole

The numbers 1 to 128 are assigned to the above compounds for identification and reference hereinafter.

Especially preferred compounds of general formula I are numbered:-
2; and 106.

According to a feature of the present invention, there is provided a method for the control of arthropod, plant nematode or helminth pests at a locus which comprises the treatment of the locus (e.g. by application or administration) with an effective amount of a compound of general formula I, or a pesticidally acceptable salt thereof, wherein the various symbols are as hereinbefore defined with the exclusion of a method for the treatment of the human or animal body carried out by a medical or veterinary practitioner as therapy. The compounds of general formula I may, in particular, be used in the fields of veterinary medicine and livestock husbandry and in the maintenance of public health against arthropods or helminths which are parasitic internally or externally upon vertebrates, particularly warm-blooded vertebrates, for example man

and domestic animals, e.g. cattle, sheep, goats, equines, swine, poultry, dogs, cats and fishes, for example Acarina, including ticks (e.g. Ixodes spp., Boophilus spp. e.g. Boophilus microplus, Amblyomma spp., Hyalomma spp., Rhipicephalus spp. e.g. Rhipicephalus appendiculatus, Haemaphysalis spp., Dermacentor spp., Ornithodorus spp. (e.g. Ornithodorus moubata) and mites (e.g. Damalinia spp., Dermahyssus gallinae, Sarcoptes spp. e.g. Sarcoptes scabiei, Psoroptes spp., Chorioptes spp., Demodex spp., Eutrombicula spp.,); Diptera (e.g. Aedes spp., Anopheles spp., Musca spp., Hypoderma spp., Gasterophilus spp., Simulium spp.); Hemiptera (e.g. Triatoma spp.); Phthiraptera (e.g. Damalinia spp., Linognathus spp.); Siphonaptera (e.g. Ctenocephalides spp.); Dictyoptera (e.g. Periplaneta spp., Blatella spp.); Hymenoptera - (e.g. Monomorium pharaonis); for example against infections of the gastro-intestinal tract caused by parasitic nematode worms, for example members of the family Trichostrongylidae, Nippostrongylus brasiliensis, Trichinella spiralis, Haemonchus contortus, Trichostrongylus colubriformis, Nematodirus battus, Ostertagia circumcincta, Trichostrongylus axei, Cooperia spp. and Hymenolepis nana; in the protection of stored products, for example cereals, including grain and flour, groundnuts, animal feedstuffs, timber and household goods, e.g. carpets and textiles, against attack by arthropods, more especially beetles, including weevils, moths and mites, for example Ephestia spp. (flour moths), Anthrenus spp. (carpet beetles), Tribolium spp. (flour beetles), Sitophilus spp. (grain weevils) and Acarus spp. (mites), in the control of cockroaches, ants and similar arthropod pests in infested domestic and industrial premises and in the control of mosquito larvae in waterways, wells, reservoirs or other running or standing water; in agriculture, against adults, larvae and eggs of Lepidoptera (butterflies and moths), e.g. Heliothis spp. such as Heliothis virescens (tobacco budworm), Heliothis armigera and Heliothis zea, Spodoptera spp. such as S.exempta, S.littoralis (Egyptian cotton worm), S.eridania (southern army worm), Mamestra configurata (bertha army worm); Earias spp. e.g. E.insulana (Egyptian bollworm), Pectinophora spp. e.g. Pectinophora gossypiella - (pink bollworm), Ostrinia spp. such as O.nubilalis (European cornborer), Trichoplusia ni (cabbage looper), Pieris spp. (cabbage worms), Laphygma spp. (army worms), Agrotis and Amathes spp. (cutworms), Wiseana spp. (porina moth), Chilo spp. (rice stem borer), Tryporyza spp. and Diatraea spp. (sugar cane borers and rice borers), Sparganothis pilleriana (grape berry moth), Cydia pomonella (codling moth), Archips spp. (fruit tree tortrix moths), Plutella xylostella (diamond back moth); against adults and larvae of Coleoptera (beetles) e.g. Hypothenemus hampei (coffee berry borer), Hylesinus spp. (bark beetles), Anthonomus grandis (cotton boll weevil), Acalymma spp. (cucumber beetles), Lema spp., Psylliodes spp., Leptinotarsa decemlineata (Colorado potato beetle), Diabrotica spp. (corn rootworms), Gonocephalum spp. (false wire worms), Agriotes spp. (wireworms), Dermolepida and Heteronychus spp. (white grubs), Phaedon cochleariae (mustard beetle), Lissorhoptrus oryzophilus (rice water weevil), Meligethes spp. (pollen beetles), Ceutorhynchus spp. Rhynchophorus and Cosmopolites spp. (root weevils); against Hemiptera e.g. Psylla spp., Bemisia spp., Aphis spp., Myzus spp., Megoura viciae, Phylloxera spp., Adelges spp., Phorodon humuli (hop damson aphid), Aeneolamia spp., Nephotettix spp. (rice leaf hoppers), Empoasca spp., Nilaparvata spp., Perkinsiella spp., Pyrilla spp., Aonidiella spp. (red scales), Coccus spp., Psuedococcus spp., Helopeltis spp. (mosquito bugs), Lygus spp., Dysdercus spp., Oxycarenus spp., Nezara spp.; Hymenoptera e.g. Athalia spp. and Cephus spp. (saw flies), Atta spp. (leaf cutting ants); Diptera e.g. Hylemyia spp. (root flies), Atherigona spp. and Chlorops spp. (shoot flies), Phytomyza spp. (leaf miners), Ceratitis spp. (fruit flies); Thysanoptera such as Thrips tabaci; Orthoptera such as Locusta and Schistocerca spp. (locusts) and crickets e.g. Gryllus spp. and Acheta spp., Collembola e.g. Sminthurus spp. and Onychiurus spp. (springtails), Isoptera e.g. Odontotermes spp. (termites), Dermaptera e.g. Forficula spp. (earwigs) and also other arthropods of agricultural significance such as Acari (mites) e.g. Tetranychus spp., Panonychus spp. and Bryobia spp. (spider mites), Eriophyes spp. (gall mites), Polyphagotarsonemus spp.; Blaniulus spp. (millipedes), Scutigerella spp. (symphilids), Oniscus spp. (woodlice) and Triops spp. (crustacea); nematodes which attack plants and trees of importance to agriculture, forestry, horticulture either directly or by spreading bacterial, viral, mycoplasma or , fungal diseases of the plants, root-knot nematodes such as Meloidogyne spp. (e.g. M. incognita); cyst nematodes such as Globodera spp. (e.g. G. rostochiensis); Heterodera spp. (e.g. H. avenae); Radopholus spp. (e.g. R. similis); lesion nematodes such as Pratylenchus spp. (e.g. P. pratensis); Belonolaimus spp. (e.g. B. gracilis); Tylenchulus spp. (e.g. T. semipenetrans); Rotylenchulus spp. (e.g. R. reniformis); Rotylenchus spp. (e.g. R. robustus); Helicotylenchus spp. (e.g. H. multicinctus); Hemicycliophora spp. (e.g. H. gracilis); Criconemoides spp. (e.g. C. similis); Trichodorus spp. (e.g. T. primitivus); dagger nematodes such as Xiphinema spp. (e.g. X. diversicaudatum), Longidorus spp. (e.g. L. elongatus); Hoplolaimus spp. (e.g. H. coronatus); Aphelenchoides spp. (e.g. A. ritzema-bosi, A. besseyi); stem and bulb eelworms such as Ditylenchus spp. (e.g. D. dipsaci).

The invention also provides a method for the control of arthropod or nematode pests of plants which comprises the application to the plants or to the medium in which they grow of an effective amount of a compound of general formula I or a pesticidally acceptable salt thereof.

The compounds of general formula I may be applied in solid or liquid compositions to the soil principally to control those nematodes dwelling therein but also to the foliage principally to control those nematodes attacking the aerial parts of the plants (e.g. Aphelenchoides spp. and Ditylenchus spp. listed above).

The compounds of general formula I are of value in controlling pests which feed on parts of the plant remote from the point of application, e.g. leaf feeding insects are killed by the subject compounds applied to roots.

In addition the compounds may reduce attacks on the plant by means of antifeeding or repellant effects.

The compounds of general formula I are of particular value in the protection of field, forage, plantation, glass house, orchard and vineyard crops, of ornamentals and of plantation and forest trees, for example, cereals (such as maize, wheat, rice, sorghum), cotton, tobacco, vegetables and salads (such as beans, cole crops, curcurbits, lettuce, onions, tomatoes and peppers), field crops (such as potato, sugar beet, ground nuts, soyabean, oil seed rape), sugar cane, grassland and forage (such as maize, sorghum, lucerne), plantations (such as of tea, coffee, cocoa, banana, oil palm, coconut, rubber, spices), orchards and groves (such as of stone and pip fruit, citrus, kiwifruit, avocado, mango, olives and walnuts), vineyards, ornamental plants, flowers and shrubs under glass and in gardens and parks, forest trees (both deciduous and evergreen) in forests, plantations and nurseries.

They are also valuable in the protection of timber (standing, felled, converted, stored or structural) from attack by sawflies (e.g. Urocerus) or beetles (e.g. scolytids, platypodids, lyctids, bostrychids, cerambycids, anobiids).

They have applications in the protection of stored products such as grains, fruits, nuts, spices and tobacco, whether whole, milled or compounded into products, from moth, beetle and mite attack. Also protected are stored animal products such as skins, hair, wool and feathers in natural or converted form (e.g. as carpets or textiles) from moth and beetle attack; also stored meat and fish from beetle, mite and fly attack.

The compounds of general formula I are of particular value in the control of arthropods or helminths which are injurious to, or spread or act as vectors of diseases in man and domestic animals, for example those hereinbefore mentioned, and more especially in the control of ticks, mites, lice, fleas, midges and biting, nuisance and myiasis flies. The compounds of general formula I are particularly useful in controlling arthropods or helminths which are present inside domestic host animals or which feed in or on the skin or suck the blood of the animal, for which purpose they may be administered orally, parenterally, percutaneously or topically.

The compositions hereinafter described for topical application to man and animals and in the protection of stored products, household goods, property and areas of the general environment may, in general, alternatively be employed for application to growing crops and crop growing loci and as a seed dressing.

Suitable means of applying the compounds of general formula I include:-

to persons or animals infested by or exposed to infestation by arthropods or helminths, by parenteral, oral or topical application of compositions in which the active ingredient exhibits an immediate and/or prolonged action over a period of time against the arthropods or helminths, for example by incorporation in feed or suitable orally-ingestible pharmaceutical formulations, edible baits, salt licks, dietary supplements, pour-on formulations, sprays, baths, dips, showers, jets, dusts, greases, shampoos, creams, wax-smears and livestock self-treatment systems;

to the environment in general or to specific locations where pests may lurk, including stored products, timber, household goods, and domestic and industrial premises, as sprays, fogs, dusts, smokes, wax-smears, lacquers, granules and baits, and in tricklefeeds to waterways, wells, reservoirs and other running or standing water;

to domestic animals in feed to control fly larvae feeding in their faeces.

The compounds of general formula I may be applied to control arthropods or helminths in compositions of any type known to the art suitable for internal or external administration to vertebrates or application for the control of arthropods in any premises or indoor or outdoor area, containing as active ingredient at least one compound of general formula I in association with one or more compatible diluents or adjuvants appropriate for the intended use. All such compositions may be prepared in any manner known to the art.

Compositions suitable for administration to vertebrates or man include preparations suitable for oral, parenteral, percutaneous, e.g. pour-on, or topical administration.

Compositions for oral administration comprise one or more of the compounds of general formula I in association with pharmaceutically acceptable carriers or coatings and include, for example, tablets, pills, capsules, pastes, gels, drenches, medicated feeds, medicated drinking water, medicated dietary supplements, slow-release boluses or other slow-release devices intended to be retained within the gastro-intestinal tract. Any of these may incorporate active ingredient contained within microcapsules or coated with acid-labile or alkali-labile or other pharmaceutically acceptable enteric coatings. Feed premixes and concentrates containing compounds of the present invention for use in preparation of medicated diets, drinking water or other materials for consumption by animals may also be used.

Compositions for parenteral administration include solutions, emulsions or suspensions in any suitable pharmaceutically acceptable vehicle and solid or semisolid subcutaneous implants or pellets designed to release active ingredient over a protracted period and may be prepared and made sterile in any appropriate manner known to the art.

Compositions for percutaneous and topical administration include sprays, dusts, baths, dips, showers, jets, greases, shampoos, creams, wax-smears, or pour-on preparations and devices (e.g. ear tags) attached externally to animals in such a way as to provide local or systemic arthropod control.

Solid or liquid baits suitable for controlling arthropods comprise one or more compounds of general formula I and a carrier or diluent which may include a food substance or some other substance to induce consumption by the arthropod.

Liquid compositions include water miscible concentrates, emulsifiable concentrates, flowable suspensions, wettable or soluble powders containing one or more compounds of general formula I which may be used to treat substrates or sites infested or liable to infestation by arthropods including premises, outdoor or indoor storage or processing areas, containers or equipment and standing or running water.

Solid homogenous or heterogenous compositions containing one or more compounds of general formula I, for example granules, pellets, briquettes or capsules, may be used to treat standing or running water over a period of time. A similar effect may be achieved using trickle or intermittent feeds of water dispersible concentrates as described herein.

Compositions in the form of aerosols and aqueous or non-aqueous solutions or dispersions suitable for spraying, fogging and low- or ultra-low volume spraying may also be used.

Suitable solid diluents which may be used in the preparation of compositions suitable for applying the compounds of general formula I include aluminium silicate, kieselguhr, corn husks, tricalcium phosphate, powdered cork, adsorbent carbon black, magnesium silicate, a clay such as kaolin, bentonite or attapulgite, and water soluble polymers and such solid compositions may, if desired, contain one or more compatible wetting, dispersing, emulsifying or colouring agents which, when solid, may also serve as diluent.

Such solid compositions, which may take the form of dusts, granules or wettable powders, are generally prepared by impregnating the solid diluents with solutions of the compound of general formula I in volatile solvents, evaporating the solvents and, if necessary, grinding the products so as to obtain powders and, if desired, granulating or compacting the products so as to obtain granules, pellets or briquettes or by encapsulating finely divided active ingredient in natural or synthetic polymers, e.g. gelatin, synthetic resins and polyamides.

The wetting, dispersing and emulsifying agents which may be present, particularly in wettable powders, may be of the ionic or non-ionic types, for example sulphoricinoleates, quaternary ammonium derivatives or products based upon condensates of ethylene oxide with nonyl- and octyl-phenol, or carboxylic acid esters of anhydrosorbitols which have been rendered soluble by etherification of the free hydroxy groups by condensation with ethylene oxide, or mixtures of these types of agents. Wettable powders may be treated with water immediately before use to give suspensions ready for application.

Liquid compositions for the application of the compounds of general formula I may take the form of solutions, suspensions and emulsions of the compounds of general formula I optionally encapsulated in natural or synthetic polymers, and may, if desired, incorporate wetting, dispersing or emulsifying agents. These emulsions, suspensions and solutions may be prepared using aqueous, organic or aqueous-organic diluents, for example acetophenone, isophorone, toluene, xylene, mineral, animal or vegetable oils, and water soluble polymers (and mixtures of these diluents), which may contain wetting, dispersing or emulsifying agents of the ionic or non-ionic types or mixtures thereof, for example those of the types described above. When desired, the emulsions containing the compounds of general formula I may be used in the form of self-emulsifying concentrates containing the active substance dissolved in the emulsifying agents or in solvents containing emulsifying agents compatible with the active substance, the simple addition of water to such concentrates producing compositions ready for use.

Compositions containing compounds of general formula I which may be applied to control arthropod, plant nematode or helminth pests, may also contain synergists (e.g. piperonyl butoxide or sesamex), stabilizing substances, other insecticides, acaricides, plant nematocides or anthelmintics, fungicides (agricultural or veterinary as appropriate e.g. benomyl, iprodione), bactericides, arthropod or vertebrate attractants or repellants or pheromones, reodorants, flavouring agents, dyes and auxiliary therapeutic

agents, e.g. trace elements. These may be designed to improve potency, persistence, safety, uptake where desired, spectrum of pests controlled or to enable the composition to perform other useful functions in the same animal or area treated.

Examples of other pesticidally-active compounds which may be included in, or used in conjuntion with, the compositions of the present invention are:-acephate, chlorpyrifos, demeton-S-methyl, disulfoton, ethoprofos, fenitrothion, malathion, monocrotophos, parathion, phosalone, pirimiphos-methyl, triazophos, cyfluthrin, cypermethrin, deltamethrin, fenpropathrin, fenvalerate, permethrin, aldicarb, carbosulfan, methomyl, oxamyl, pirimicarb, bendiocarb, teflubenzuron, dicofol, endosulfan, lindane, benzoximate, cartap, cyhexatin, tetradifon, avermectins, ivermectin, milbemycins, thiophanate, trichlorfon and dichlorvos.

The compositions for application to control arthropods usually contain from 0.00001% to 95%, more particularly from 0.0005% to 50%, by weight of one or more compounds of general formula I or of total active ingredients (that is to say the compound(s) of general formula I together with other substances toxic to arthropods and plant nematodes, anthelmintics, synergists, trace elements or stabilisers). The actual compositions employed and their rate of application will be selected to achieve the desired effects(s) by the farmer, livestock producer, medical or veterinary practitioner, pest control operator or other person skilled in the art. Solid and liquid compositions for application topically to animals, timber, stored products or household goods usually contain from 0.00005% to 90%, more particularly from 0.001% to 10%, by weight of one or more compounds of general formula I. For administration to animals orally or parenterally, including percutaneously, solid and liquid compositions normally contain from 0.1% to 90% by weight of one or more compounds of general formula I. Medicated feedstuffs normally contain from 0.001% to 3% by weight of one or more compounds of general formula I. Concentrates and supplements for mixing with feedstuffs normally contain from 5% to 90%, and preferably from 5% to 50%, by weight of one or more compounds of general formula I. Mineral salt licks normally contain from 0.1% to 10% by weight of one or more compounds of general formula I.

Dusts and liquid compositions for application to livestock, persons, goods, premises or outdoor areas may contain 0.0001% to 15%, and more especially 0.005% to 2.0%, by weight of one or more compounds of general formula I. Suitable concentrations in treated waters are between 0.0001 ppm and 20 ppm, and more especially 0.001 ppm to 5.0 ppm, of one or more compounds of general formula I and may also be used therapeutically in fish farming with appropriate exposure times. Edible baits may contain from 0.01% to 5% and preferably 0.01% to 1.0%, by weight of one or more compounds of general formula I.

When administered to vertebrates parenterally, orally or by percutaneous or other means, the dosage of compounds of general formula I will depend upon the species, age and health of the vertebrate and upon the nature and degree of its actual or potential infestation by arthropods. A single dose of 0.1 to 100 mg, preferably 2.0 to 20.0 mg, per kg body weight of the animal or doses of 0.01 to 20.0 mg, preferably 0.1 to 5.0 mg, per kg body weight of the animal per day for sustained medication are generally suitable by oral or parenteral administration. By use of sustained release formulations or devices, the daily doses required over a period of months may be combined and administered to animals on a single occasion. The present invention accordingly provides an arthropodical, plant nematocidal or anthelmintic composition which comprises at least one compound of general formula I, or a salt thereof, in association with one or more compatible diluents or carriers with the provisos that (1) when $R^4$ represents chloro, Y represents nitro, and Z represents methyl and $(R^3)_n$ represents 4-nitro, the composition is not a plant nematocidal composition and comprises a pharmaceutically acceptable adjuvant or a feedstuff or is substantially sterile and pyrogen-free or is in unit dosage form; and (2) excluding compositions comprising 1-(4-nitrophenyl)-3-nitro-4-cyano or -carboxamide pyrazole.

The invention also provides the use as an arthropodicide, plant nematocide or anthelmintic of such a composition.

Medicated feeds which comprise known compounds of general formula I and arthropodicidally- or anthelmintically-acceptable salts thereof and an edible carrier or diluent form a feature of the present invention.

In experiments on activity against arthropods carried out on representative compounds, the following results (wherein 'Dose mg/kg' indicates the dose of test compound administered in mg per kg animal body weight and ppm indicates the concentration of the compound in parts per million of the test solution applied) have been obtained:-

Test 1

One or more dilutions of the compounds to be tested were made in 50% aqueous acetone.

a)Test species: Plutella xylostella (Diamond-back Moth) and Phaedon cochleariae (Mustard Beetle).

Turnip leaf discs were set in agar in petri-dishes and infected with 10 larvae (2nd instar Plutella or 3rd instar Phaedon). Four replicate dishes were assigned to each treatment and were sprayed under a Potter Tower with the appropriate test dilution. Four or five days after treatment the dishes were removed from the constant temperature (25 °C) room in which they had been held and the mean percentage mortalities of larvae were determined. These data were corrected against the mortalities in dishes treated with 50% aqueous acetone alone which served as controls .

b)Megoura viciae (Vetch Aphid)

Potted tic bean plants previously infected with mixed stages of Megoura were sprayed to run-off using a laboratory turntable sprayer. Treated plants were held in a greenhouse for 2 days and were assessed for aphid mortality using a scoring system, judging the response in comparison with plants treated with 50% aqueous acetone alone, as controls .

| Score | |
|---|---|
| 3 | all aphids dead |
| 2 | few aphids alive |
| 1 | most aphids alive |
| 0 | no significant mortality |

According to the above method (a) an application of 500 ppm of the following compounds was totally effective against the larvae of Plutella xylostella, producing 100% mortality.

Compound No.

6, 7, 8, 72, 73, 80, 87, 94, 106.

According to the above method (a) an application of 5 ppm of the following compounds was totally effective against the larvae of Phaedon cochleariae, producing 100% mortality.

Compound No.

74, 80, 106.

According to the above method (b) an application of 50 ppm of the following compounds was totally effective against Megoura viciae producing 100% mortality, that is giving a score of 12 from 4 replicates.

Compound No

48, 92, 93, 106.

The data quoted in Tables 1 and 2 summarise the results from a number of different experiments carried out to the protocols a) and b) above.

Table 1

| No. | Plutella %m 500 ppm | Phaedon %m 10 ppm | Megoura score/12 50 ppm |
|---|---|---|---|
| 6 | | 100 | 9 |
| 7 | | 100 | 10 |

Table 2

| No. | Plutella %m 500 ppm | Phaedon %m 10 ppm |
|---|---|---|
| 8 | | 93 |
| 2 | 89 | 100 |

Test 2

Some 20 larvae of Rhipicephalus appendiculatus were placed in plastic capsules attached to the shaved flank of guinea-pigs. After 3 hours and then at 23 hourly intervals, the guinea-pigs were given a total of 4 subcutaneous injections of the test compound. Approximately 100 hours after infestation, the guinea-pigs were killed and the engorged tick larvae recovered, counted and kept at 23 °C in a humidity cabinet for 14 to 21 days. After this period, the percentage survival through moulting was assessed. Results obtained are given below in Table 4.

Table 4

| Compound No. | Dose (mg/kg at each repetition) | Result |
|---|---|---|
| 12 | 5 | No ticks recovered |
| | 4 | No ticks recovered |
| | 3 | Less than five engorged ticks recovered |
| | 2.5 | Ticks engorged normally but only 62.5 per cent survived |
| 26 | 10 | No ticks recovered |
| | 5 | Less than five engorged ticks recovered |

Test 3

The high activity of the compounds of general formula against the cockroach species Periplaneta americana is demonstrated by results from the following experiment.

0.2 microlitres of an acetone solution of the compound was injected through the soft cuticle between the leg and thorax of ten insects, to give a dose rate of 5 micrograms per g of insect body weight. Ten cockroaches were similarly injected with 0.2 microlitres of acetone alone to serve as controls. After treatment the insects were held in plastic boxes with appropriate food. Five days after treatment the numbers of dead and alive insects were counted and percentage mortalities calculated.

According to the above method a dose of 5 micrograms/g insect body weight of the following compounds was totally effective against the cockroach species Periplaneta americana producing 100% mortality.

Compound No.

2, 14, 17.

The following Examples illustrate compositions for use against arthropod, plant nematode or helminth pests which comprise, as active ingredient, compounds of general formula I.

Example A

A dusting powder may be prepared by intimately mixing:-

| | |
|---|---|
| Compound of general formula I | 1 to 10% w/w (weight/weight) |
| Talc superfine | to 100% by weight |

This powder may be applied to a locus of arthropod infestation, for example refuse tips or dumps, stored products or household goods or animals infested by, or at risk of infestation by, arthropods to control the arthropods by oral ingestion. Suitable means for distributing the dusting powder to the locus of arthropod infestation include mechanical blowers, handshakers and livestock self treatment devices.

Example B

An edible bait may be prepared by intimately mixing:-

| | |
|---|---|
| Compound of general formula I | 0.1 to 1.0% w/w |
| Wheat flour | 80% w/w |
| Molasses | to 100% w/w |

This edible bait may be distributed at a locus, for example domestic and industrial premises, e.g. kitchens, hospitals or stores, or outdoor areas, infested by arthropods, for example ants, locusts, cockroaches and flies, to control the arthropods by oral ingestion.

Example C

A solution may be prepared containing:-

| | |
|---|---|
| Compound of general formula I | 15% w/v (weight/volume) |
| Dimethylsulphoxide | to 100% by volume |

by dissolving the pyrazole derivative in a portion of the dimethylsulphoxide and then adding more dimethylsulphoxide to the desired volume. This solution may be applied to domestic animals infested by arthropods, percutaneously as a pour-on application or, after sterilisation by filtration through a polytetrafluoroethylene membrane (0.22 μm pore size), by parenteral injection, at a rate of application of from 1.2 to 12 ml of solution per 100 kg of animal body weight.

Example D

A wettable powder may be formed from:-

| | |
|---|---|
| Compound of general formula I | 50% w/w |
| Ethylan BCP (a nonylphenol/ethylene oxide condensate containing 9 moles of ethylene oxide per mol of phenol) | 5% w/w |
| Aerosil (silicon dioxide of microfine-particle size) | 5% w/w |
| Celite PF (synthetic magnesium silicate carrier) | 40% w/w |

by adsorbing the Ethylan BCP onto the Aerosil, mixing with the other ingredients and grinding the mixture in a hammer-mill to give a wettable powder, which may be diluted with water to a concentration of from 0.001% to 2% w/v of the pyrazole compound and applied to a locus of infestation by arthropods, for example dipterous larvae, or plant nematodes by spraying, or to domestic animals infested by, or at risk of infestation by, arthropods, by spraying or dipping, or by oral administration as drinking water, to control the arthropods or helminths.

Example E

A slow release bolus may be formed from granules containing a density agent, binder, slow-release agent and compound of general formula I at varying percentage compositions. By compressing the mixture a bolus with a specific gravity of 2 or more can be formed and may be administered orally to ruminant domestic animals for retention within the reticulo-rumen to give a continual slow release of pyrazole compound over an extended period of time to control infestation of the ruminant domestic animals by arthropods or helminths.

Example F

A slow release composition may be prepared from:-

| | |
|---|---|
| Compound of general formula I<br>polyvinylchloride base | 0.5 to 25% w/w<br>to 100% w/w |

by blending the polyvinylchloride base with the pyrazole compound and a suitable plasticiser, e.g. dioctyl phthalate, and melt-extruding or hot-moulding the homogenous composition into suitable shapes, e.g. granules, pellets, brickettes or strips, suitable, for example, for addition to standing water or, in the case of strips, fabrication into collars or ear-tags for attachment to domestic animals, to control insect pests by slow release of the pyrazole compound.

The compounds of general formula I can be prepared by the application or adaptation of known methods (i.e. methods heretofore used or described in the chemical literature), generally heterocycle formation followed where necessary by changing substituents with protection/deprotection of other substituents if necessary, for example as hereinafter described.

In the following description when symbols appearing in formulae are not specifically defined it is to be understood that they are "as hereinbefore defined" in accordance with the first definition of each symbol in this specification.

Compounds of general formula I conforming to general formula IA wherein Y' represents the cyano or nitro group or a group $RSO_2$, RSO or RS, a straight- or branched-chain alkoxycarbonyl group containing from 2 to 7 carbon atoms, or a straight- or branched-chain alkyl group containing from 1 to 6 carbon atoms which may be unsubstituted or substituted by one or more halogen atoms, Z' represents the unsubstituted amino group or a straight- or branched-chain alkyl group containing from 1 to 4 carbon atoms, and $R^5$ represents a fluorine, chlorine or bromine atom or the cyano group may be prepared by the process which comprises

(i) the reaction of a compound of the general formula II, or an acid addition salt thereof, e.g. the hydrochloride, with (1), when $R^5$ in the compound of general formula IA represents a fluorine, chlorine or bromine atom, a compound of the general formula III, wherein $R^6$ represents the cyano group or a straight- or branched-chain alkanoyl group containing from 2 to 5 carbon atoms and $R^8$ represents a straight- or branched-chain alkoxy group containing from 1 to 4 carbon atoms, preferably ethoxy, the hydroxy group or a fluorine, chlorine or bromine atom, or (2), when $R^5$ in the compound of general formula IA represents the cyano group (and Y' represents the cyano group and Z' represents the unsubstituted amino group), tetracyanoethylene.

The reaction of a compound of general formula II with a compound of general formula III (optionally prepared in situ) or tetracyanoethylene may be effected in the presence of an inert organic solvent, for example an alkanol containing from 1 to 4 carbon atoms, e.g. ethanol, acetic acid, ethoxyethanol or an ether, and at a temperature from ambient temperature to the reflux temperature of the reaction mixture and optionally in the presence of an alkali metal, e.g. sodium or potassium, acetate, carbonate or bicarbonate or organic base e.g. triethylamine. When an acid addition salt of the compound of general formula II is used, the reaction with the compound of general formula III is effected in the presence of an alkali metal, e.g. sodium or potassium, acetate, carbonate or bicarbonate.

(ii) Compounds of general formula IA wherein Z′ represents the unsubstituted amino group may alternatively be prepared directly by reacting a compound of general formula $Y'CH_2CN$ with a compound of general formula II in the presence of a compound of general formula $R^7C(R^o)_3$ wherein $R^7$ represents a straight- or branched-chain alkyl group containing from 1 to 4 carbon atoms which may be unsubstituted or substituted by one or more halogen atoms or a cycloalkyl group containing from 3 to 6 carbon atoms and $R^o$ represents an alkoxy group which may be straight- or branched-chain and preferably contains from 1 to 4 carbon atoms, in an inert organic solvent, preferably ethanol, at a temperature from ambient to reflux.

(iii) Compounds of general formula IA wherein Z′ represents the unsubstituted amino group and $R^5$ represents the cyano group may be obtained by the reaction of a compound of the general formula IV with a molar equivalent of compound of general formula $Y'CH_2CN$, i.e. malononitrile when Y′ represents the cyano group, generally in the presence of an anhydrous inert organic solvent, e.g. ethanol, and a molar equivalent of a base, e.g. sodium hydride, and at a temperature from 0 to 50 °C.

The compounds of general formula IA may be prepared by reaction of a compound of general formula II with a compound of general formula III or tetracyanoethylene with isolation of an intermediate compound

of general formula V from the reaction mixture. When the reaction of a compound of general formula II with a compound of general formula III is effected in acetic acid, in the absence or presence of an alkali metal, e.g. sodium or potassium, acetate, the intermediate compound of general formula V may separate from the reaction mixture, depending upon its solubility in the reaction medium, and may, if desired, be isolated before being cyclised as hereinbefore described to a compound of general formula IA. The cyclisation of a compound of general formula V, which constitutes a feature of the invention may be effected in the presence of an inert organic solvent, for example an alkanol containing from 1 to 4 carbon atoms, e.g. ethanol, acetic acid or ethoxyethanol, at a temperature of from ambient temperature up to the reflux temperature of the reaction mixture, and optionally in the presence of sodium ethoxide when the solvent is ethanol.

It will be appreciated that in the preparation of compounds of general formula I the following subsidiary processes or adaptations thereof may be performed in an appropriate combination to achieve the compound sought.

Compounds of general formula I which conform to general formula IB wherein $R^1$ represents an $R^9C(=O)$- group, wherein $R^9$ represents a straight- or branched-chain alkyl or alkoxy group containing from 1 to 6 carbon atoms, or a cycloalkyl group containing from 3 to 6 carbon atoms, and $R^2$ represents a hydrogen atom or an $R^9C(=O)$- group which is identical to the group $R^9C(=O)$- represented by $R^1$ or $-NR^1R^2$ represents a cyclic imide as hereinbefore defined, may be prepared by the reaction of a compound of general formula I wherein Z represents the unsubstituted amino group, or an alkali metal salt thereof, with a compound of the general formula:-

$R^9COX$ VI

wherein X represents a chlorine or bromine atom, or with a compound of the general formula:-

$(R^9CO)_2O$ VII

or with a dicarboxylic acid derivative. The reaction may be conducted in the absence or presence of an inert organic solvent, for example acetonitrile, tetrahydrofuran, a ketone, e.g. acetone, an aromatic hydrocarbon, e.g. benzene or toluene, chloroform, dichloromethane or dimethylformamide, and optionally in the presence of an acid-binding agent, for example pyridine, triethylamine or an alkali metal, e.g. sodium or potassium, carbonate or bicarbonate, at a temperature from 0°C to the reflux temperature of the reaction medium, to give a compound of general formula IB wherein $R^1$ represents an $R^9C(=O)$- group wherein $R^9$ is as hereinbefore defined and $R^2$ represents a hydrogen atom or an $R^9C(=O)$- group, depending upon the reaction conditions chosen and/or the use of an excess of the compound of general formula VI or VII.

Compounds of general formula IB wherein $R^1$ represents a formula group and $R^2$ represents a hydrogen atom may be prepared by reaction of a compound of general formula I, wherein Z represents the unsubstituted amino group with formic acid. The reaction may be conducted in an inert organic solvent, for example a ketone e.g. methylisobutyl ketone, or an aromatic hydrocarbon, e.g. benzene or toluene, at the reflux temperature of the reaction mixture.

Compounds of general formula IB wherein $R^1$ represents a formyl group and $R^2$ represents a hydrogen atom or a formyl group, may be prepared by the reaction of a compound of general formula I, wherein Z represents the unsubstituted amino group with formylacetic anhydride. Formylacetic anhydride may be prepared from formic acid and acetic anhydride and the reaction with the compound of general formula I may be conducted in the absence of presence of an inert organic solvent, for example a ketone, e.g. acetone, or an aromatic hydrocarbon, e.g. benzene or toluene, and optionally in the presence of an acid-binding agent, for example pyridine, triethylamine or an alkali metal, e.g. sodium or potassium, carbonate or bicarbonate, at a temperature from 0°C to the reflux temperature of the reaction mixture, to give a compound of general formula IB wherein $R^1$ represents a formyl group and $R^2$ represents a hydrogen atom or a formyl group, depending upon the reaction conditions chosen and/or the use of an excess of formylacetic anhydride.

Compounds of general formula IB wherein $R^1$ represents a formyl group or a group $R^9C(=O)$- and $R^2$ represents a hydrogen atom may be prepared by the selective removal by hydrolysis of an $R^9C(=O)$- group or a formyl group from a compound of general formula IB wherein $R^1$ and $R^2$ both represent a $R^9C(=O)$ group or a formyl group. Hydrolysis is effected under mild conditions, for example by treatment with an aqueous-ethanolic solution or suspension of an alkali metal, e.g. sodium or potassium, bicarbonate, or with aqueous ammonia.

Compounds of general formula IB wherein $R^1$ represents a straight- or branched-chain alkoxycarbonyl group containing from 2 to 7 carbon atoms which is unsubstituted or substituted by one or more halogen atoms, and $R^2$ represents a hydrogen atom may be prepared by the reaction of a compound of the general formula VIII, wherein $R^{10}$ represents an alkoxycarbonyl group $R^{11}C(=O)$, wherein $R^{11}$ represents a straight- or branched-chain alkoxy group containing from 1 to 6 carbon atoms (which is unsubstituted or substituted by one or more halogen atoms) or a phenoxy group, with a compound of the general formula:-

$R^{11}H$ IX

to replace a first group represented by the symbol $R^{10}$ by a hydrogen atom, and to replace the second group represented by the symbol $R^{10}$ by an alkoxycarbonyl group when $R^{10}$ represents a phenoxycarbonyl group, or, if desired, to replace the second group represented by the symbol $R^{10}$ by another alkoxycarbonyl group when $R^{10}$ in formula VIII represents an alkoxycarbonyl group. As will be apparent to those skilled in the art, the desired compound of general formula IB is obtained by selection of the appropriate compounds of general formulae VIII and IX. The reaction may be effected in water or an inert aqueous-organic or organic solvent, for example an alkanol containing 1 to 4 carbon atoms, e.g. ethanol, or an aromatic hydrocarbon, e.g. benzene or toluene, or which is preferably an excess of the compound of general formula IX, at a temperature from ambient temperature to the reflux temperature of the reaction mixture and, if necessary, at elevated pressure, and optionally in the presence of a base, for example an alkali metal alkoxide, e.g. of the compound of general formula IX.

Compounds of general formula IB wherein $R^1$ and $R^2$, which may be the same or different, each represents a formyl group or a $R^9C(=O)$- group, may be prepared by the reaction of an alkali metal, e.g. sodium or potassium, derivative of a compound of general formula IB wherein $R^1$ represents a group $R^9C(=O)$- as hereinbefore defined, or a formyl group, and $R^2$ represents a hydrogen atom with formic acid, formylacetic anhydride or a compound of general formula VI. Reaction may be effected in an inert aprotic solvent, e.g. dimethylformamide, at a temperature from laboratory temperature to the reflux temperature of the reaction mixture.

Alkali metal derivatives of compounds of general formula I (wherein Z represents the unsubstituted amino group) or IB wherein $R^1$ represents a group $R^9C(=O)$- and $R^2$ represents a hydrogen atom may be prepared in situ by reaction with an alkali metal, e.g. sodium or potassium, hydride, in an inert aprotic solvent, e.g. dimethylformamide, at a temperature from laboratory temperature to the reflux temperature of the reaction mixture.

Compounds of general formula VIII wherein $R^{10}$ represents a group $R^{11}C(=O)$-, may be prepared as hereinbefore described. Compounds of general formula VIII wherein $R^{10}$ represents a phenoxycarbonyl group may be prepared by the reaction of a compound of general formula I (wherein Z represents the unsubstituted amino group), with a compound of the general formula:-

$R^{12}COX$ VIA

, wherein $R^{12}$ represents a phenoxy group, or with a compound of the general formula:-

$(R^{12}CO)_2O$ VIIA

using the reaction conditions hereinbefore described for the reaction of a compound of general formula I with a compound of formula VI or VII.

Compounds of general formula IB wherein $R^1$ represents a group $R^{13}$ which represents a straight- or branched-chain alkyl group containing from 1 to 6 carbon atoms (which may be unsubstituted or substituted by alkoxycarbonyl groups containing from 2 to 5 carbon atoms) or a cycloalkyl group containing from 3 to 6 carbon atoms, and $R^2$ represents a hydrogen atom may be prepared by the removal of the group $R^9C(=O)$- of a compound of the general formula IB, wherein $R^1$ represents a group $R^{13}$ and $R^2$ represents a group $R^9C(=O)$- . Removal of the group $R^9C(=O)$- may be effected by selective hydrolysis under mild conditions, for example by treatment with an alkali metal, e.g. sodium or potassium, hydroxide in water or an inert organic or aqueous-organic solvent, for example a lower alkanol, e.g. methanol, or a mixture of water and lower alkanol, at a temperature from laboratory temperature up to the reflux temperature of the reaction mixture.

Compounds of general formula IB, wherein $R^1$ represents a group $R^{13}$ and $R^2$ represents a group $R^9C(=O)$-, may be prepared by reaction of a compound of general formula IB wherein $R^1$ represents a hydrogen atom , or an alkali metal, e.g., sodium or potassium, derivative thereof, with a compound of the

general formula:-

$R^{13}X^1$ X

, wherein $X^1$ represents a chlorine, bromine or iodine atom. Reaction may be effected in an inert organic solvent, e.g. dichloromethane, tetrahydrofuran, or dimethylformamide, at a temperature from laboratory temperature up to the reflux temperature of the reaction mixture and, when a compound of general formula IB is used, in the presence of a base, e.g. Triton B; or by reaction of a compound of general formula IB wherein $R^1$ represents the hydrogen atom and $R^2$ represents a group $R^{13}$ with a compound of general formula VI or VII.

Compounds of general formula I wherein Z represents an N-(alkyl or cycloalkyl)-N-formylamino group as hereinbefore described may be prepared in a similar manner to the process above using, where appropriate, formylacetic anhydride instead of a compound of general formula VI or VII.

Compounds of general formula IB wherein one of $R^1$ and $R^2$ or both of $R^1$ and $R^2$ represent a straight- or branched- chain alkyl group containing from 1 to 6 carbon atoms or cycloalkyl group containing from 3 to 6 carbon atoms, groups represented by $R^1$ and $R^2$ being identical, may be prepared by reaction of a compound of general formula I, wherein Z represents the unsubstituted amino group, or an alkali metal, e.g. sodium or potassium, derivative thereof, with a compound of general formula X, in the absence or presence of an inert organic solvent, for example an aromatic hydrocarbon, e.g. benzene or toluene, chloroform, dichloromethane, tetrahydrofuran or dimethylformamide, and optionally in the presence of an acid-binding agent, for example pyridine, triethylamine or an alkali metal, e.g. sodium or potassium, bicarbonate, at a temperature from 0°C up to the reflux temperature of the reaction mixture.

Alkali metal derivatives of compounds of formulae IB (wherein $R^1$ represents a hydrogen atom) and I (wherein Z represents the unsubstituted amino group) may be prepared in situ by the reaction of the compounds, with an alkali metal, e.g. sodium or potassium, hydride, at a temperature from laboratory temperature to the reflux temperature of the reaction mixture.

Compounds of general formula I wherein Z represents a straight- or branched-chain alkoxymethyleneamino group containing from 2 to 5 carbon atoms which may be unsubstituted or substituted on methylene by a straight- or branched-chain alkyl group containing from 1 to 4 carbon atoms may be prepared by the reaction of a compound of general formula I (wherein Z represents the unsubstituted amino group) with a trisalkoxyalkane in the presence of an acidic catalyst, e.g. p-toluenesulphonic acid, at a temperature from ambient temperature to the reflux temperature of the reaction mixture.

Compounds of general formula I, wherein Z represents a straight- or branched-chain alkylsulphenylamino group containing from 1 to 4 carbon atoms, may be prepared by the reaction of compounds of general formula I (wherein Z represents the unsubstituted amino group) with an alkanesulphenyl chloride in the presence of a base, e.g. sodium hydride, and optionally in the presence of a crown ether catalyst, e.g. 15-crown-5.

The reaction may be performed in a solvent, e.g. tetrahydrofuran, at a temperature from 0°C to the reflux temperature of the reaction mixture.

Compounds of general formula I wherein Z represents $-NHCH_2R^{14}$ wherein $R^{14}$ represents the hydrogen atom or a straight- or branched-chain alkyl group containing from 1 to 4 carbon atoms may be prepared by reaction of a compound of general formula I wherein Z represents $-N=C(OR^{15})R^{14}$ wherein $R^{15}$ represents a straight- or branched-chain alkyl group containing from 1 to 4 carbon atoms with a reducing agent, preferably sodium borohydride. The reaction may be effected in an inert organic solvent, ethanol or methanol being preferred, at a temperature from 0°C to the reflux temperature of the reaction mixture.

Compounds of general formula I wherein Y represents $-C(=O)NH_2$ may be prepared by partial hydrolysis of a compound of general formula I wherein Y represents -CN preferably with sulphuric acid at a temperature from ambient temperature to 100°C.

Compounds of general formula I wherein Y represents the chlorine, bromine or iodine atom may be prepared by reaction of a compound of general formula XI with a halogenating agent, preferably N-halosuccinimide in an inert solvent, preferably carbon tetrachloride, at a temperature from ambient temperature to the reflux temperature of the reaction mixture.

Compounds of general formula I wherein Z represents the chlorine, bromine or iodine atom may be prepared by diazotisation of a compound of general formula I wherein Z represents $-NH_2$ with an alkyl nitrite, preferably tert-butyl nitrite, in the presence of a halogenating agent preferably bromoform, iodine or anhydrous copper chloride at a temperature from 0°C to 100°C.

Compounds of general formula I wherein Y represents the nitro group may be prepared by reacting a compound of general formula XI with a nitrating agent, preferably nitric acid optionally in the presence of

sulphuric acid or nitric acid in a solvent such as acetic acid or acetic anhydride at a temperature from 0 ° C to 100 ° C.

Compounds of general formula I wherein Y represents $-SO_2NR^{16}R^{17}$ wherein $R^{16}$ and $R^{17}$, which may be the same or different, each represent the hydrogen atom or a straight- or branched-chain alkyl group containing from 1 to 6 carbon atoms may be prepared by reacting a compound of general formula XIV with an amine of the general formula $R^{16}R^{17}NH$ in a solvent such as toluene or water at a temperature from 0 ° C to the reflux temperature of the reaction mixture.

Compounds of general formula I wherein Y represents $-CONR^{16}R^{17}$ may be prepared by reacting a compound of general formula XV wherein $X^2$ represents a chlorine or bromine atom or activated ester moiety e.g. 4-nitrophenoxy group, especially the chlorine atom, with an amine of the general formula $R^{16}R^{17}NH$, in a solvent such as toluene or water, at a temperature from 0 ° C to the reflux temperature of the reaction mixture.

Intermediates of general formula XI may be prepared by decarboxylation of a compound of general formula XVI, performed by heating at a temperature from 100 ° C to 250 ° C optionally in the presence of an inert organic solvent, particularly N,N-dimethylaniline.

Alternatively intermediates of general formula XI may be prepared directly from esters of compounds of general formula XVI by heating in an inert organic solvent preferably acetic acid at a temperature from 50 ° C to reflux, in the presence of a strong acid preferably hydrobromic acid.

Intermediates of general formula XVI may be prepared by hydrolysis of esters of general formula I wherein Y represents $-COOR^{18}$ wherein $R^{18}$ represents a straight- or branched- chain alkyl group containing from 1 to 6 carbon atoms, preferably with an alkali metal hydroxide in a solvent such as an aqueous alcohol at a temperature from 0 ° C to the reflux temperature of the reaction mixture.

Intermediates of general formula XIV may be prepared by reacting a compound of general formula XI with chlorosulphonic acid at a temperature from 0 ° C to 150 ° C.

Intermediates of general formula XV are prepared by reacting a compound of general formula XVI with a chlorinating or brominating agent or e.g. 4-nitrophenol (preferably thionyl chloride) at a temperature from ambient temperature to the reflux temperature of the reaction mixture.

Compounds of general formula I wherein Y represents $-C(=O)R^{18}$ wherein $R^{18}$ represents a straight- or branched-chain alkyl group containing from 1 to 6 carbon atoms may be prepared by the reaction of a compound of general formula XI with an acylating agent such as $R^{18}COCl$ in the presence of a catalyst such as aluminium chloride and in an inert organic solvent such as 1,1,2,2-tetrachloroethane and at a temperature from 0 ° C to the reflux temperature of the reaction mixture.

When Z is an amino group it may also be acylated and subsequent hydrolysis using an acid such as hydrochloric or hydrobromic acid in a solvent such as dioxan or acetic acid may be necessary.

Compounds of general formula I wherein Y represents $-C(=O)R^{18}$ may also be prepared by the reaction of nitriles of the general formula I wherein Y represents -CN with an organometallic reagent such as a compound of general formula $R^{18}MgX^1$ in an inert organic solvent such as diethyl ether or tetrahydrofuran, at a temperature from 0 ° C to reflux.

Compounds of general formula IC may be prepared by the reaction of a compound of general formula I wherein Y represents the thiocyanato group with an organometallic reagent such as a compound of general formula $RMgX^1$ in an inert organic solvent such as diethyl ether or tetrahydrofuran, and at a temperature from ambient temperature to the reflux temperature of the reaction mixture.

Compounds of general formula IC wherein RS is other than a 1-alkenylthio group may also be prepared by reacting a compound of general formula I wherein Y represents the thiocyanato group with a base, preferably sodium hydroxide, or a reducing agent, preferably sodium borohydride, in the presence of a reagent of general formula $R'X^1$ wherein $R'$ is as hereinbefore defined for R with the exclusion of 1-alkenyl groups, for example methyl iodide in an inert organic or aqueous-organic solvent such as an alcohol e.g. ethanol or a mixture of an alcohol and water, the reaction being performed at a temperature from ambient to reflux.

Alternatively compounds of general formula IC wherein RS is other than a 1-alkenylthio group may be prepared by reductive alkylation of disulphides of general formula XVII employing a reducing agent preferably sodium dithionite or sodium borohydride, in the presence of a base, preferably sodium hydroxide or sodium carbonate, and of a reagent of general formula $R'X^1$ such as methyl iodide in an inert organic or aqueous-organic solvent such as an alcohol e.g. ethanol or a mixture of an alcohol and water, at a temperature from ambient to reflux.

Alternatively compounds of general formula IC may be prepared from a halide of general formula I wherein Y represents a bromine or iodine atom by metal exchange using a strong base, preferably butyl lithium, and subsequent addition of the appropriate disulphide of general formula R-S-S-R in an inert

organic solvent such as tetrahydrofuran, and the reaction is performed at a temperature from -78 °C to ambient.

Alternatively, compounds of general formula IC wherein RS represents a straight- or branched-chain alkylthio group containing from 1 to 6 carbon atoms which may be unsubstituted or substituted by one or more halogen atoms, may be prepared by reacting a compound of general formula XI with an alkanesulphenyl halide (which may be optionally substituted by one or more halogen atoms) in an inert organic solvent, preferably chloroform, in the presence of a base such as pyridine, and at temperatures from 0 ° to reflux.

Compounds of general formula IC wherein RS represents a methylthio group which is substituted by three halogen atoms which may be the same or different may also be prepared by the reaction of a compound of general formula I wherein Y represents the thiocyanato group with a source of halogenocarbene, such as chloroform and sodium hydroxide, preferably with phase transfer catalysis using for example benzyltriethylammonium chloride or tetrabutylammonium chloride.

Compounds of general formula IC wherein RS represents a straight- or branched-chain alkylthio group containing from 1 to 6 carbon atoms which is substituted by one or more fluorine atoms may also be prepared by a halogen exchange reaction of a compound of general formula IC wherein RS represents a straight- or branched-chain alkylthio group containing from 1 to 6 carbon atoms which is substituted by one or more chlorine atoms with a fluorinating agent such as a mixture of antimony trifluoride and antimony pentachloride, KF or CsF in an aprotic solvent such as sulfolane at a temperature from 50 °C to reflux.

Compounds of general formula I wherein Y represents the thiocyanato group may be prepared by the reaction of a compound of general formula XI with a thiocyanating agent such as alkali metal or ammonium salts of thiocyanic acid (e.g. NaSCN) and bromine in an inert organic solvent such as methanol, and at a temperature from 0 °C to 100 °C.

Intermediates of general formula XVII may be prepared by hydrolysis of thiocyanates of general formula I wherein Y represents the thiocyanato group, preferably using hydrochloric acid in the presence of ethanol at a temperature from ambient to reflux temperature; they may also be prepared by reduction of the thiocyanates by sodium borohydride in an alcohol preferably ethanol at a temperature from ambient to reflux.

Compounds of general formula I wherein Y represents a group RSO may be prepared by the oxidation of compounds of general formula IC by an oxidising reagent preferably 3-chloroperbenzoic acid in an inert organic solvent such as dichloromethane or by hydrogen peroxide in acetic acid at a temperature from 0 °C to the reflux temperature of the reaction medium.

Compounds of general formula I wherein Y represents a group $RSO_2$ may also be prepared by the above process, by employing an excess of the oxidising agent.

Compounds of general formula I wherein Y represents a group $RSO_2$ wherein R represents a straight- or branched-chain alkyl group containing from 1 to 6 carbon atoms which is substituted by one or more fluorine atoms may also be prepared by a halogen exchange reaction of a compound of general formula I wherein Y represents a group $RSO_2$ wherein R represents a straight- or branched-chain alkyl group containing from 1 to 6 carbon atoms which is substituted by one or more chlorine atoms with a fluorinating agent such as a mixture of antimony trifluoride and antimony pentachloride, KF or CsF at a temperature from 50 °C to 200 °C.

Compounds of general formula I wherein Y represents a group $RSO_2$ may also be prepared by reaction of a compound of general formula XI with the appropriate sulphonic anhydride of general formula $(RSO_2)_2O$ for example trifluoromethanesulphonic or methanesulphonic anhydride and in the presence of aluminum chloride as catalyst, and employing an inert organic solvent such as 1,1,2,2-tetrachloroethane at a temperature from ambient to 150 °C.

Compounds of general formula I wherein Z represents a straight- or branched-chain alkyl group containing from 1 to 4 carbon atoms, the carboxy group, a group $R^{19}S$ wherein $R^{19}$ represents a straight- or branched-chain alkyl group containing from 1 to 6 carbon atoms which may be unsubstituted or substituted by one or more halogen atoms or Z represents a trialkylsilyl group containing from 1 to 6 carbon atoms in each alkyl group which may be the same or different may be prepared by the reaction of a compound of general formula I wherein Z represents a hydrogen, bromine or iodine atom with a lithiating agent preferably lithium diisopropylamide or n-butyl lithium, and reaction with the appropriate substrate from alkyl halide, carbon dioxide, dialkylsulphides or trialkylsilyl halides respectively at a temperature from -78 °C to ambient temperature, and in an inert solvent, preferably tetrahydrofuran.

Compounds of general formula I wherein Z represents a hydrogen atom may be prepared by diazotisation of an amine of general formula I wherein Z represents the unsubstituted amino group using an alkyl nitrite, preferably tert-butyl nitrite, in an inert solvent preferably tetrahydrofuran, at a temperature from

ambient temperature to the reflux temperature of the reaction mixture.

Compounds of general formula I wherein Z represents a group $R^{19}SO$ may be prepared by the reaction of a compound of general formula I wherein Z represents a group $R^{19}S$ with an oxidising agent, preferably 3-chloroperbenzoic acid in a solvent such as dichloromethane, or by hydrogen peroxide in acetic acid at a temperature from 0°C to the reflux temperature of the reaction mixture.

Compounds of general formula I wherein Z represents a group $R^{19}SO_2$ may also be prepared by the above process, by employing an excess of the oxidising agent.

Compounds of general formula I wherein Z represents the fluorine atom or the cyano group may be prepared by the reaction of a halide of general formula I wherein Z represents the chlorine or bromine atom with an alkali metal fluoride, preferably caesium fluoride, or with an alkali metal cyanide preferably KCN, under anhydrous conditions in an inert solvent, preferably sulfolane, and at a temperature from ambient temperature to 150°C.

Compounds of general formula I wherein Z represents the nitro group may be prepared by oxidation of amines of general formula I wherein Z represents the unsubstituted amino group with an oxidant, preferably trifluoroperacetic acid or m-chloroperbenzoic acid and in an inert organic solvent preferably dichloromethane at a temperature from 0°C to reflux.

Compounds of general formula I wherein Z represents the cyano group may be prepared by dehydration of the corresponding amide preferably by heating with phosphorous pentoxide at a temperature from 50°C to 250°C.

The amides may be prepared (i) by reacting a carboxylic acid of general formula I wherein Z represents a carboxy group with a chlorinating agent preferably thionyl chloride, and (ii) reacting the resultant acid chloride of general formula XVIII with ammonia:-

(i) the reaction with a chlorinating agent preferably thionyl chloride is generally conducted at a temperature from ambient temperature to the reflux temperature of the reaction mixture;

(ii) the reaction with ammonia is generally conducted in a solvent which may be inert, preferably toluene, or in the presence of water, and at a temperature from 0°C to 100°C.

Compounds of general formula I wherein Y represents a group $RSO_2$ is other than a 1-alkenylsulphonyl group may be prepared alternatively by reaction of sulphinate metal (e.g. sodium) salts with a reagent of general formula $R'X^1$ or preferably a sulphate of general formula $(R')_2SO_4$, in a solvent such as water and in the presence of sodium bicarbonate at a temperature from 0°C to 100°C.

The intermediate sulphinate sodium salts may be prepared by reaction of sulphonyl chlorides of general formula XIV with sodium sulphite in the presence of sodium bicarbonate and water as solvent, at a temperature from 50°C to reflux.

Intermediates of general formula XIV may also be prepared from the thiocyanates of general formula I wherein Y represents a thiocyanato group by chlorination using chlorine in a solvent, preferably water, at a temperature from ambient to reflux.

Compounds of general formula I wherein $R^3$ represents a haloalkylsulphinyl group may be prepared by oxidation of a haloalkylthio derivative of general formula I, preferably with m-chloroperbenzoic and in an inert organic solvent preferably dichloromethane, at a temperature from 0°C to reflux.

Compounds of general formula I wherein $R^3$ represents a haloalkylsulphonyl group may be prepared in a similar manner, by employing two molar equivalents of oxidant.

Compounds of general formula I wherein Y represents the fluorine atom may be prepared by diazotisation of corresponding amines using sodium nitrite in tetrafluoroboric acid and sulphuric acid at a temperature from -10°C to +10°C, followed by photolysis in the presence of excess sodium tetrafluoroboric acid at a temperature from -30°C to ambient.

Intermediate amines above may be prepared by reduction of nitro compounds of general formula I wherein Y represents a nitro group, preferably with zinc in ethanol at a temperature from ambient to reflux.

Compounds of general formula I wherein Y represents the methyl group may be prepared by reduction of an acid of general formula XVI using a reducing agent, preferably borane-tetrahydrofuran complex in a solvent preferably tetrahydrofuran at a temperature from -30°C to reflux.

Compounds of general formula I wherein Z represents a trialkylsilylmethyl group as hereinbefore defined may be prepared by the reaction of a compound of general formula I wherein Z represents the methyl group with a lithiating agent preferably lithium diisopropylamide or n-butyl lithium, and reaction with a trialkylsilyl halide at a temperature from -78°C to ambient, and in an inert organic solvent preferably tetrahydrofuran, optionally in an inert atmosphere.

The following processes optionally followed by the subsidiary processes hereinbefore described permit the preparation of the remaining compounds of general formula I not described above, as well as some whose preparation is described above.

Compounds of general formula I wherein $R^4$ represents a chlorine, bromine or iodine atom and Z represents the unsubstituted amino group, may be prepared by the diazotisation of the (diamino) compounds of general formula I in which Z represents and $R^4$ is replaced by amino, using a molar equivalent of sodium nitrite in a mineral acid, for example a mixture of concentrated sulphuric acid and acetic acid, at a temperature from 0 to 60 °C, and by subsequent reaction with the appropriate copper salt and appropriate mineral acid or with an aqueous solution of potassium iodide (when $R^4$ represents an iodine atom) at a temperature from 0 to 100 °C.

The diamino compounds above wherein Y represents the cyano group may be prepared by the reaction of potassium cyanoform $KC(CN)_3$ with a phenylhydrazine of general formula II in the presence of hydrochloric acid, at a temperature from 50 °C to the reflux temperature of the reaction mixture.

Intermediates of general formula XII may be prepared by reduction of compounds of general formula XIX preferably with lithium borohydride in an inert organic solvent e.g. tetrahydrofuran at a temperature from 0 °C to the reflux temperature of the reaction mixture.

Intermediates of general formulae XIX (wherein $R^{20}$ represents an alkyl group) and XX wherein Z represents the unsubstituted amino group may be obtained by the reaction of a compound of the general formula XIII (wherein $R^o$ represents an alkoxy group) with a molar equivalent of compound of general formula $Y'CH_2CN$, i.e. malononitrile when Y represents the cyano group, in the presence of an anhydrous solvent, e.g. ethanol, and a molar equivalent of a base, e.g. sodium hydride, and at a temperature from 0 to 50 °C followed, if desired, by hydrolysis of the esters of general formula XIX with an aqueous base, e.g. sodium hydroxide, with a co-solvent, e.g. ethanol, at a temperature from 0 °C to the reflux temperature of the reaction mixture.

Intermediates of general formulae IV and XIII may be prepared by chlorination of the appropriate unsubstituted compound using chlorine or other chlorinating agent.

Intermediates of general formulae IV and XIII may be prepared by diazotisation of the appropriate aniline with a solution of a molar equivalent of sodium nitrite in a mineral acid, e.g. a mixture of concentrated sulphuric acid and acetic acid at a temperature from 0 to 60 °C, and then reacting with the compound of formula $CH_3COCH(Cl)CN$ or a compound of general formula $CH_3COCH(Cl)COR^o$ wherein $R^o$ represents an alkoxy group in the presence of an inert solvent, e.g. a mixture of water and ethanol, buffered, e.g. with excess sodium acetate, and at a temperature from 0 to 50 °C.

Compounds of general formula I wherein $R^4$ represents the nitro group may be prepared by oxidation of the corresponding amine with an oxidant, preferably trifluoroperacetic acid or m-chloroperbenzoic acid in an inert organic solvent preferably dichloromethane at a temperature from 0 °C to reflux. By employing known protecting agents in this process compounds of general formula I wherein Z represents the amino group may be prepared.

Compounds of general formula I wherein $R_4$ represents the fluorine atom may be prepared by the diazotisation of the corresponding amine of general formula I in which $R^4$ is replaced by $-NH_2$ using for example a solution of sodium nitrite in a mineral acid, for example sulphuric acid and in the presence of fluoroboric acid or its sodium salt and subsequent thermolysis or photolysis of the diazonium fluoroborate derivative by methods known per se.

Amine intermediates above wherein Z represents the hydrogen atom may be prepared by performing a Curtius rearrangement of the corresponding acid azide by heating in an inert organic solvent such as toluene at a temperature from 50 ° to 150 °C to give an isocyanate which is then reacted with for example tert-butanol to give a carbamate, which in turn is hydrolysed using dilute acid preferably hydrochloric acid in ethanol at a temperature from ambient to reflux.

Intermediate acid azides may be prepared by reaction of a carboxylic acid of general formula XX wherein Z represents the hydrogen atom with a chlorinating agent, preferably thionyl chloride at temperatures from ambient to reflux, followed by reaction of the intermediate acid chloride with sodium azide in a polar solvent, preferably acetone and water at a temperature from 0 °C to ambient.

Compounds of general formula I wherein $R^4$ represents the cyano group may also be prepared by reacting a carboxylic acid of general formula XX with a chlorinating agent, preferably thionyl chloride at ambient to reflux temperature, followed by reaction of the intermediate acid chloride with ammonia to give an intermediate amide which is then dehydrated by heating with a dehydrating reagent, preferably phosphorus pentoxide at a temperature from 50-250 °C.

Intermediates of general formula XX may be prepared by hydrolysis of the corresponding esters of general formula XIX preferably using a base such as sodium hydroxide and a solvent such as aqueous alcohol, and at a temperature from 0 °C to the reflux temperature of the reaction mixture.

Compounds of general formula I wherein Y represents a 1,1-difluoroalkyl group which may be substituted by one or more additional halogen atoms may be prepared by the reaction of a compound of

general formula I wherein Y represents a straight- or branched-chain alkanoyl group containing from 2 to 6 carbon atoms or the corresponding compound in which Y is replaced by the formyl group or a straight- or branched-chain alkanoyl group containing from 2 to 6 carbon atoms which is substituted by one or more halogen atoms with a fluorinating agent, preferably diethylaminosulphur trifluoride or sulphur tetrafluoride in an inert organic solvent, preferably dichloromethane, at a temperature from -78 °C to ambient.

Compounds of general formula I wherein Y represents the trifluoromethyl group or a trifluoromethylalkyl group containing from 2 to 6 carbon atoms which may be substituted by one or more additional halogen atoms may be prepared by the reaction of a fluorinating agent, e.g.sulphur tetrafluoride, with an acid of general formula XVI or the corresponding carboxyalkyl compound (it being understood that the carboxy group may be attached to any position of the alkyl moiety) at a temperature from ambient to 150 °C).

Salts with pesticidally-acceptable bases of compounds of general formula I wherein Z represents the carboxy group may be prepared from the corresponding compounds of general formula I by methods known per se, for example by reacting stoichiometric quantities of the compound of general formula I and the appropriate base, for example an alkali metal hydroxide, carbonate or bicarbonate, an alkaline earth metal hydroxide or carbonate, ammonia or an amine (e.g. diethanolamine, triethanolamine, octylamine, morpholine or dioctylamine), in a suitable solvent. The salts may, if necessary, be purified by recrystallisation from one, two or more suitable solvents.

Compounds of general formula I not hitherto disclosed or described in the chemical literature, together with their processes of preparation form further features of the present invention.

The present invention accordingly provides the compounds of general formula I, wherein the various symbols are as hereinbefore defined, and salts thereof, with the exclusion of the compounds wherein:

$R^4$ represents chloro, Y represents nitro, and Z represents methyl and $(R^3)_n$ represents 4-nitro; and $R^4$ represents nitro, Y represents cyano or $CONH_2$, Z represents hydrogen and $(R^3)_n$ represents 4-nitro substitution;

$R^4$ and Y both represent cyano, Z represents amino and $(R^3)_n$ represents 3- or 4-nitro or 4-chloro substitution;

$R^4$ represents bromine, Y represents methyl and Z represents hydrogen or $R^4$ and Y represent bromine and Z represents methyl and $(R^3)_n$ represents 2,4-dinitrophenyl or 2,4,6-trinitrophenyl substitution;

$R^4$ and Y represent bromine and Z represents hydrogen, $R^4$ and Y represent chlorine and Z represents methyl, and $(R^3)_n$ represents 2,4-dinitrophenyl substitution;

$R^4$ represents chlorine, Y represents bromine and Z represents methyl and $(R^3)_n$ represents 2,4-dinitrophenyl substitution;

and with the exclusion of compounds of formula (I) wherein

$R^4$, Y and Z represent, respectively, nitro, methyl and hydrogen and $(R^3)_n$ represents 2,4,6-trinitrophenyl;

$R^4$, Y and Z represent, respectively, bromine, bromine and hydrogen and $(R^3)_n$ represents 4-bromophenyl;

$(R^3)_n$ represents 2,4-dinitrophenyl and $R^4$, Y and Z represent, respectively, chlorine, methyl and hydrogen; chlorine, bromine and hydrogen;

$(R^3)_n$ represents 4-nitrophenyl and $R^4$, Y and Z represent, respectively, bromine, methyl and hydrogen ; nitro, methyl and hydrogen ; and cyano, methoxycarbonyl and amino

$R^4$, Y and Z represent, respectively, nitro, methyl and nitro and $(R^3)_n$ represents 2,4-dinitrophenyl.

Intermediates for the preparation of certain compounds of general formula I comprise compounds of that formula for which in their alternative meanings Y represents the hydrogen atom, the formyl or carboxy group, a straight- or branched-chain alkanoyl group containing from 2 to 6 carbon atoms which is substituted by one or more halogen atoms, the dithio group (which joins two pyrazole rings), the amino group, the $-SO_2Cl$ group, a straight- or branched-chain carboxyalkyl group containing from 2 to 6 carbon atoms, Z represents the carbamoyl group or a straight- or branched-chain alkoxycarbonyl group containing from 2 to 7 carbon atoms or the diphenoxycarbonylamino group, $(R^3)_n$ substitution is a preferred combination given earlier in the specification or $R^4$ represents the amino, hydroxymethyl, carboxy or carbamoyl group or a straight- or branched-chain alkoxycarbonyl or alkoxycarbonylamino group containing from 2 to 7 carbon atoms.

The following Examples and Reference Examples illustrate the preparation of compounds of general formula I according to the present invention:-

EXAMPLE 1

Compound No.1

A mixture of 2,4,6-trichlorophenylhydrazine (21.1 g) and tetracyanoethylene (13.3 g) in ethanol (100 ml) was heated at reflux for 15 minutes. The reaction mixture was cooled and the solid precipitate was filtered off and washed with diethyl ether to give 5-amino-3,4-dicyano-1-(2,4,6-trichlorophenyl)pyrazole (13 g), as a buff coloured solid, m.p. 267-271 °C.

EXAMPLE 2

Compounds Nos.2 and 3

Tetracyanoethylene (1.9 g) and 2,6-dichloro-4-trifluoromethylphenylhydrazine (3.7 g) was added to a magnetically-stirred solution of sodium acetate (0.6 g) in glacial acetic acid (15 ml) at laboratory temperature. After stirring for 15 minutes, a colourless solid precipitated from the solution and stirring was continued overnight. The mixture was then filtered. The solid obtained was washed successively with acetic acid, water, aqueous sodium bicarbonate solution and water, to give 5-amino-1-(2,6-dichloro-4-trifluoromethylphenyl)-3,4-dicyanopyrazole (2.5 g), as beige crystals, m.p. 221-222 °C.

By proceeding in a similar manner, but replacing the 2,6-dichloro-4-trifluoromethylphenylhydrazine by 2,3,5,6-tetrachlorophenylhydrazine, there was obtained:-

5-Amino-3,4-dicyano-1-(2,3,5,6-tetrachlorophenyl)pyrazole, m.p. greater than 330 °C, in the form of a buff-coloured powder.

REFERENCE EXAMPLE 1

Phenylhydrazines used as starting materials in Examples 1, 2 and 11, not hitherto described in the chemical literature were prepared as follows:-

2,6-Dichloro-4-trifluoromethylphenylaniline (4.3 g) was dissolved with stirring, in glacial acetic acid (23 ml). A solution of sodium nitrite (1.5 g) in concentrated sulphuric acid (11 ml) was then added at 55-60 °C. The solution thus obtained was cooled to 0-5 °C and a solution of stannous chloride (16.4 g) in concentrated hydrochloric acid (14 ml) was added with vigorous stirring. A cream-coloured solid precipitated. The mixture was filtered and the solid obtained was added to a mixture of aqueous ammonia solution and ice. The mixture thus obtained was extracted with diethyl ether (6 x 500 ml) and the combined ethereal extracts were dried over sodium sulphate, filtered and evaporated to dryness to give 2,6-dichloro-4-trifluoromethylphenylhydrazine (3.7 g) m.p. 54-56 °C, in the form of a colourless crystalline solid.

By proceeding in a similar manner, but replacing the 2,6-dichloro-4-trifluoromethylaniline by the hereinafter indicated aniline there were prepared:-

2-Chloro-4-trifluoromethylphenylhydrazine, m.p. 38-39 °C, in the form of a colourless solid, from 2-chloro-4-trifluoromethylaniline.

EXAMPLE 3

Compounds Nos.6, 7 and 8

3,5-Diamino-4-cyano-1-(2,6-dichloro-4-trifluoromethylphenyl)pyrazole (3.9g; prepared as described below) was dissolved with stirring in glacial acetic acid (60ml) at 15 °C. A solution of sodium nitrite (0.88g) in concentrated sulphuric acid (5.85ml) was then added over 5 minutes, maintaining at 15 °C. After 15 minutes longer at this temperature, the dark red oil solution was poured during 1 minute onto a stirred solution of cuprous chloride (2.32g) in concentrated hydrochloric acid (36ml). After 15 minutes at laboratory temperature, by which time the evolution of nitrogen had completely subsided, the reaction mixture was poured onto excess ice and water, and extracted with dichloromethane (3 x 50ml). The combined extracts were washed with water (2 x 50ml), then with sodium bicarbonate solution (50ml), dried over anhydrous magnesium sulphate, and evaporated in vacuo to give a brown semi-solid (4.1g). Chromatography on silica (Merck, 230-400 mesh, 0.7 kg $cm^{-2}$) using a mixture of dichloromethane and ethyl acetate (98:2) as eluent gave after evaporation of the eluate and recrystallisation of the residue from a mixture of dichloromethane and petroleum ether (b.p. 60-80 °C) 5-amino-3-chloro-4-cyano-1-(2,6-dichloro-4-trifluoromethylphenyl)-pyrazole (0.95g), m.p. 189-191 °C, in the form of white crystals.

By proceeding in a similar manner but replacing the cuprous chloride and concentrated hydrochloric acid by cuprous bromide and 48% w/v hydrobromic acid respectively there was prepared:-

5-Amino-3-bromo-4-cyano-1-(2,6-dichloro-4-trifluoromethylphenyl)pyrazole, m.p. 182-183 °C, in the form of white crystals.

By replacing the cuprous chloride and concentrated hydrochloric acid by a solution of potassium iodide in water there was prepared:-

5-Amino-3-iodo-4-cyano-1-(2,6-dichloro-4-trifluoromethylphenyl)pyrazole, m.p. 208-210 °C, in the form of white crystals.

REFERENCE EXAMPLE 2

A suspension of 2,6-dichloro-4-trifluoromethylphenylhydrazine (14.7g) in water (40ml) was stirred with concentrated hydrochloric acid (5.2ml), and potassium cyanoform (8.52g) added. The suspension was stirred and heated under reflux for 16 hours, and left to cool overnight. The mixture was washed into a separating funnel with the aid of ethyl acetate and water, and the organic phase collected. The aqueous phase was re-extracted with ethyl acetate (2 x 80ml), and the combined organic solutions washed with water (2 x 50ml), dried over anhydrous magnesium sulphate, and evaporated in vacuo to give an orange solid (20.9g). Two recrystallisations from a mixture of ethyl acetate and petroleum ether (b.p. 60-80 °C) gave 3,5-diamino-4-cyano-1-(2,6-dichloro-4-trifluoromethylphenyl)pyrazole (7.75g), m.p. 208-210 °C in the form of white crystals.

EXAMPLE 4

Compounds Nos.12, 13, 14, 15, 16 and 26

A suspension of 5-amino-1-(2,6-dichloro-4-trifluoromethylphenyl)-3,4-dicyanopyrazole (15.0g) in chloroform (250ml) was treated with acetyl chloride (42.8ml) with mechanical stirring at 0 °C. A solution of dry pyridine (7.0ml) in chloroform (30ml) was added dropwise during 30 minutes, keeping at 0 °C. The mixture was stirred overnight at laboratory temperature, and then heated under reflux conditions in order to complete the reaction. After cooling, the solution was poured onto a mixture of ice and dilute hydrochloric acid, and the chloroform layer separated. The aqueous solution was re-extracted with chloroform (2 x 100ml), and the combined organic extracts were washed with water (100ml), dried over anhydrous magnesium sulphate, and evaporated in vacuo to give a buff-coloured solid (23.0g). Recrystallisation from a mixture of ethyl acetate and petroleum ether (b.p. 60-80 °C) gave 5-acetamido-1-(2,6-dichloro-4-trifluoromethylphenyl)-3,4-dicyanopyrazole, m.p. 208-209 °C, in the form of white crystals.

By proceeding in a similar manner, the following phenylpyrazoles were obtained by acylation of 5-amino-1-(2,6-dichloro-4-trifluoromethylphenyl)-3,4-dicyanopyrazole with the appropriate acid chloride:-

5-Dichloroacetamido-1-(2,6-dichloro-4-trifluoromethylphenyl)-3,4-dicyanopyrazole, m.p. 186-187 °C after purification by trituration with carbon tetrachloride and subsequent recrystallisation from a mixture of ethanol and water, in the form of an off-white solid. The reaction was performed at laboratory temperature.

5-Cyclopropylcarbonamido-1-(2,6-dichloro-4-trifluoromethylphenyl)-3,4,dicyanopyrazole, m.p. 217-218 °C after recrystallisation from a mixture of ethanol and water, in the form of an off-white solid. The reaction was performed at laboratory temperature.

5-Pentanamido-1-(2,6-dichloro-4-trifluoromethylphenyl)-3,4-dicyanopyrazole in the form of a pale yellow glass. Infra-Red Absorption bands: 3260, 3100, 2960, 2940, 2880, 2240, 1730, 1700, 1315, 880, 820 $cm^{-1}$ (liquid film). The reaction was performed at 0 °C during the addition, and at laboratory temperature thereafter.

5-Propionamido-1-(2,6-dichloro-4-trifluoromethylphenyl)-3,4-dicyanopyrazole, m.p. 188-189 °C after purification by chromatography on silica (Merck, 230-400 mesh, 0.7 kg $cm^{-2}$) using a mixture of acetone and hexane (2:3) as eluent, and subsequent trituration with toluene, in the form of a white powder. The reaction was performed at laboratory temperature.

By proceeding in a similar manner; but replacing the solvent by acetonitrile, the following phenylpyrazole was obtained by acylation of 5-amino-1-(2,6-dichloro-4-trifluoromethylphenyl)-3,4-dicyanopyrazole with trimethylacetyl chloride:-

1-(2,6-Dichloro-4-trifluoromethylphenyl)-3,4-dicyano-5-(2,2-dimethylpropionamido)pyrazole as white crystals, m.p. 202-203 °C from toluene-hexane, and after purification by chromatography on silica (Merck, 230-400 mesh, 0.7 kg $cm^{-2}$) using a mixture of dichloromethane and ethyl acetate (9:1) as eluent.

EXAMPLE 5

Compounds Nos.17 and 18

Anhydrous sodium acetate (1.0g) was dissolved in stirred acetic acid (40ml), and tetracyanoethylene (3.5g) was added at laboratory temperature. 2-Chloro-4-trifluoromethylphenylhydrazine (5.25g) was added in one portion, and the mixture was stirred overnight. After dilution with water, the precipitated solid was filtered off to give, after drying, 5-amino-1-(2-chloro-4-trifluoromethylphenyl)-3,4-dicyano pyrazole, m.p. 209-210 °C in the form of a white powder.

By proceeding in a similar manner but replacing the 2-chloro-4-trifluoromethylphenylhydrazine by 2,3,5,6-tetrafluoro-4-trifluoromethylphenylhydrazine and with cooling during addition of the phenylhydrazine to the tetracyanoethylene solution, there was prepared:-

5-amino-3,4-dicyano-1-(2,3,5,6-tetrafluoro-4-trifluoromethylphenyl)pyrazole, m.p. 262-263 °C in the form of a buff powder.

REFERENCE EXAMPLE 6

Sodium hydride (80%, 0.25 g) was added to a stirred solution of 5-amino-4-cyano-1-(2,6-dichloro-4-trifluoromethylphenyl)-3-trifluoromethylpyrazole (2.9 g) in dry tetrahydrofuran (50 ml). After 3 hours at room temperature, 15-crown-5 (1 drop) and methyl iodide (2 g) was added at 0 °C, and the mixture left overnight at room temperature. The solution was evaporated in vacuo, and the residue was dissolved in dichloromethane (50 ml), washed with water, dilute hydrochloric acid and water. After drying over anhydrous magnesium sulphate, filtration, and evaporation in vacuo a yellow oil was obtained. Purification by chromatography using Merck silica (230-400 mesh, 0.7 kg $cm^{-2}$) with dichloromethane as eluent gave 4-cyano-1-(2,6-dichloro-4-trifluoromethylphenyl)-5-dimethylamino-3-trifluoromethylpyrazole as a white solid, m.p. 105-107 °C.

REFERENCE EXAMPLE 7

Sodium hydride (80%, 0.3 g) was added to a stirred solution of 5-amino-4-cyano-1-(2,6-dichloro-4-trifluoromethylphenyl)-3-trifluoromethylpyrazole (2.9 g) in dry tetrahydrofuran (50 ml). After 3 hours, 15-crown-5 (1 drop) and trimethylacetyl chloride (1.8 g) was added, and the mixture stirred overnight. Evaporation in vacuo gave a buff semisolid, which was dissolved in dichloromethane. This solution was washed with water, dilute hydrochloric acid and with water again and finally dried over anhydrous magnesium sulphate. Filtration followed by evaporation in vacuo gave a yellow oil, which was purified by chromatography on silica (Merck, 40-230 mesh, 0.7 kg $cm^{-2}$). Elution with dichloromethane gave after evaporation 4-cyano-1-(2,6-dichloro-4-trifluoromethylphenyl)-5-(2,2-dimethylpropionamido)-3-trifluoromethylpyrazole as a white solid, m.p. 198-200 °C.

REFERENCE EXAMPLE 8

A solution of 4-cyano-1-(2,6-dichloro-4-trifluoromethylphenyl)-5-bis(cyclopropanecarbonyl)amino-3-trifluoromethylpyrazole (1.0 g) in ethanol (50 ml) was heated under reflux with saturated sodium bicarbonate solution (25 ml) for 45 minutes. After cooling, and evaporation of the solvent in vacuo, the residue was diluted with water and extracted with dichloromethane. The extract was dried over anhydrous magnesium sulphate, filtered, and evaporated in vacuo to give 4-cyano-1-(2,6-dichloro-4-trifluoromethylphenyl)-5-cyclopropanecarbonamido-3-trifluoromethylpyrazole as a white solid m.p. 210-212 °C.

REFERENCE EXAMPLE 9

5-Amino-4-cyano-1-(2,6-dichloro-4-trifluoromethylphenyl)-3-hydroxymethylpyrazole was prepared as follows:-

A solution of 5-amino-4-cyano-1-(2,6-dichloro-4-trifluoromethylphenyl)-3-ethoxycarbonylpyrazole (1.0 g) in dry tetrahydrofuran (15 ml) was treated under nitrogen with lithium borohydride (0.06 g) with stirring at room temperature for 18 hours. Ethyl acetate (5 ml) followed by saturated sodium chloride solution (5 ml) was added, and the mixture was acidified with dilute hydrochloric acid and extracted with dichloromethane. The extract was dried over anhydrous magnesium sulphate, filtered, and evaporated in vacuo. The residual oil was purified by chromatography on silica (Merck, 40-230 mesh, 0.7 kg $cm^{-2}$) eluting with a mixture of

dichloromethane and ethyl acetate (1:1), and the pure fractions were evaporated in vacuo and recrystallised from ethyl acetate-petroleum ether to give the title compound as a white solid m.p. 159-161 °C

5-Amino-4-cyano-1-(2,6-dichloro-4-trifluoromethylphenyl)-3-ethoxycarbonylpyrazole was prepared as follows:-

To sodium hydride (80%, 0.9 g) in dry ethanol (30 ml) was added, with stirring, malononitrile (1.98 g). Ethyl chloro-(2,6-dichloro-4-trifluoromethylphenyl)hydrazono-acetate (11.0 g) was then added with stirring and cooling. The internal temperature quickly rose to 20 °C and was kept at that for 1 hour, before filtration of the pale yellow solid. The filtrate was evaporated in vacuo to give an orange solid. The combined solids were dissolved in ethyl acetate, washed twice with water, dried over anhydrous magnesium sulphate, filtered and evaporated in vacuo to give an orange solid (11.0 g). Recrystallisation from ethyl acetate-petroleum ether gave the title compound as fawn crystals (8.3 g) m.p. 208-209 °C.

Ethyl chloro-(2,6-dichloro-4-trifluoromethylphenyl)hydrazonoacetate was prepared as follows:-

Sodium nitrite (3.04 g) was added during 15 minutes to stirred concentrated sulphuric acid (24 ml) at 30-50 °C. The solution was cooled to 20 °C, and added dropwise during 15 minutes to a solution of 2,6-dichloro-4-trifluoromethylaniline (9.2 g) in acetic acid (90 ml), maintaining at 35-40 °C. This solution was then cooled to +10°, and added dropwise to a stirred solution of anhydrous sodium acetate (54 g) and ethyl chloroacetoacetate (7.0 g) in a mixture of water (72 ml) and ethanol (48 ml) during 45 minutes with cooling such that the temperature was kept at 10 °C. After 1 hour at room temperature the mixture was diluted with water, filtered, and the solid dissolved in dichloromethane. This solution was dried over anhydrous magnesium sulphate, filtered, and evaporated in vacuo to give the title compound as a white solid (11.9 g) m.p. 96-98 °C.

REFERENCE EXAMPLE 9

To a solution of 5-amino-4-cyano-1-(2,6-dichloro-4-trifluoromethylphenyl)-3-trifluoromethylpyrazole (2.33 g) in dry tetrahydrofuran (30 ml) was added with stirring at room temperature, a solution of tert-butyl nitrite (1.36 ml) in dry tetrahydrofuran (5 ml) during 2 minutes. The solution was then heated under reflux for 1 hour and cooled, and additional tert-butyl nitrite (2.72 ml) was added. The solution was heated under reflux for 30 minutes, and left to cool overnight. Evaporation in vacuo gave an orange oil, which was purified by chromatography on silica (Merck, 40-230 mesh, 0.7 kg $cm^{-2}$) eluting with dichloromethane- hexane (1:1). The product was finally recrystallised from hexane to give 4-cyano-1-(2,6-dichloro-4-trifluoromethylphenyl)-3-trifluoromethylpyrazole, m.p. 121-123 °C, as white crystals.

EXAMPLE 6

Compounds Nos. 48, 49 and 50

By proceeding in a similar manner to that hereinbefore described in Example 1, but replacing 2,4,6-trichlorophenylhydrazine by 2,6-dichloro-4-trifluoromethylthiophenylhydrazine, there was obtained:-

5-Amino-3,4-dicyano-1-(2,6-dichloro-4-trifluoromethylthiophenyl)pyrazole, m.p. 226-227 °C, in the form of an off-white solid, after recrystallisation from toluene.

By employing 2-chloro-3,5,6-trifluoro-4-trifluoromethylphenylhydrazine there was prepared:-

5-Amino-1-(2-chloro-3,5,6-trifluoro-4-trifluoromethylphenyl)-3,4-dicyanopyrazole, m.p. 242-243 °C, in the form of an orange solid, after recrystallisation from a mixture of ethanol and water.

By employing 2,6-dichloro-3,5-difluoro-4-trifluoromethylphenylhydrazine there was prepared:-

5-Amino-1-(2,6-dichloro-3,5-difluoro-4-trifluoromethylphenyl)-3,4-dicyanopyrazole, m.p. 245-247 °C, in the form of an off-white solid.

REFERENCE EXAMPLE 10

2,6-Dichloro-4-trifluoromethylthiophenylhydrazine was prepared by following the procedure of Reference Example 1, by proceeding in a similar manner, but replacing the 2,6-dichloro-4-trifluoromethylaniline by 2,6-dichloro-4-trifluoromethylthioaniline.

REFERENCE EXAMPLE 11

2-Chloro-3,5,6-trifluoro-4-trifluoromethylphenylhydrazine was prepared as follows:-

3-Chloro-2,4,5,6-tetrafluorobenzotrifluoride (12.1 g) and hydrazine hydrate (3.4 g) were heated under

reflux with ethanol (50 ml) for 3.5 hours. The mixture was poured onto ice/water mixture (500 ml), stirred, and the product was filtered. After washing with water and drying in a desiccator the title compound was obtained in the form of white crystals, m.p. 91-92 °C.

By proceeding in a similar manner but replacing 3-chloro-2,4,5,6-tetrafluorobenzotrifluoride by 3,5-dichloro-2,4,6-trifluorobenzotrifluoride there was prepared 2,6-dichloro-3,5-difluoro-4-trifluoromethylphenylhydrazine in the form of pale yellow crystals, m.p. 78-80 °C.

EXAMPLE 7

Compound No. 51

By proceeding in a similar manner to that hereinbefore described in Example 2, but employing 2,6-dichloro-4-trifluoromethoxyphenylhydrazine there was obtained:-

5-Amino-1-(2,6-dichloro-4-trifluoromethoxyphenyl)-3,4-dicyanopyrazole, m.p. 231-232 °C in the form of a brown solid, after recrystallisation from toluene.

REFERENCE EXAMPLE 12

2,6-Dichloro-4-trifluoromethoxyphenylhydrazine used in the above Example 7 was prepared by following the procedure of Reference Example 1, by proceeding in a similar manner, but replacing the 2,6-dichloro-4-trifluoromethylaniline by 2,6-dichloro-4-trifluoromethoxyaniline. The title compound was obtained as fawn crystals, m.p. 64-65 °C.

EXAMPLE 8

Compound No. 64

By proceeding in a similar manner to that hereinbefore described in Example 5, but replacing the 2-chloro-4-trifluoromethylphenylhydrazine by 2,6-dichloro-4-nitrophenylhydrazine there was prepared:-

5-Amino-1-(2,6-dichloro-4-nitrophenyl)-3,4-dicyanopyrazole, in the form of a pale brown solid, m.p. 289-290 °C.

EXAMPLE 9

Compounds Nos. 65 and 66

By proceeding in a similar manner to that hereinbefore described in Reference Example 6, but replacing the 5-amino-4-cyano-1-(2,6-dichloro-4-trifluoromethylphenyl)-3-trifluoromethylpyrazole by 5-amino-1-(2,6-dichloro-4-trifluoromethylphenyl)-3,4-dicyanopyrazole and using an appropriate quantity of methyl iodide there was prepared:-

1-(2,6-Dichloro-4-trifluoromethylphenyl)-3,4-dicyano-5-methylaminopyrazole in the form of a pale yellow solid, m.p. 165-166 °C, after recrystallisation from toluene.

By proceeding as above, but employing ethyl iodide, there was prepared:-

1-(2,6-Dichloro-4-trifluoromethylphenyl)-3,4-dicyano-5-ethylaminopyrazole in the form of an off-white solid, m.p. 245-246 °C, after purification by chromatography on silica (Merck 230-400 mesh, 0.7 kg $cm^{-2}$) using a mixture of ethyl acetate and petroleum ether (15:85).

EXAMPLE 10

Compound Nos. 72, 73, 74 and 77.

By proceeding in a similar manner to that hereinbefore described in Reference Example 7, there were prepared:-

3-Chloro-1-(2,6-dichloro-4-trifluoromethylphenyl)-4-cyano-5-trimethylacetylaminopyrazole, in the form of a white solid, m.p. 203-204 °C;

3-Chloro-1-(2,6-dichloro-4-trifluoromethylphenyl)-4-cyano-5-bis(ethoxycarbonyl)aminopyrazole, in the form of an orange crystalline solid, m.p. 67-69 °C;

and 3-Chloro-1-(2,6-dichloro-4-trifluoromethylphenyl)-4-cyano-5-ethoxycarbonylaminopyrazole, in the form of

a yellow solid, m.p. 175-179 °C;

[The latter three compounds were obtained by reaction of 5-amino-3-chloro-1-(2,6-dichloro-4-trifluoromethylphenyl)-4-cyanopyrazole with the appropriate acyl chlorides]

1-(2,6-Dichloro-4-trifluoromethylphenyl)-5-bis(ethoxycarbonyl)amino-3,4-dicyanopyrazole was prepared in a similar manner to the procedure described in Reference Example 7, but replacing the 5-amino-4-cyano-1-(2,6-dichloro-4-trifluoromethylphenyl)-3-trifluoromethylpyrazole by 5-amino-1-(2,6-dichloro-4-trifluoromethylphenyl)-3,4-dicyanopyrazole The trimethylacetyl chloride was replaced by the appropriate quantity of ethyl chloroformate (two equivalents) and 2 equivalents of sodium hydride were also used. The product was white crystals with m.p. 74-76 °C.

EXAMPLE 11

Compounds Nos. 80

By proceeding in a similar manner to that hereinbefore described in Reference Example 8, but replacing the 4-cyano-1-(2,6-dichloro-4-trifluoromethylphenyl)-5-bis(cyclopropanecarbonyl)amino-3-trifluoromethylpyrazole by 1-(2,6-dichloro-4-trifluoromethylphenyl)-5-bis(ethoxycarbonyl)amino-3,4-dicyanopyrazole there was obtained:-

1-(2,6-Dichloro-4-trifluoromethylphenyl)-3,4-dicyano-5-ethoxycarbonylaminopyrazole in the form of a white solid, m.p. 161-163 °C.

EXAMPLE 12

Compound No. 87

By proceeding in a similar manner to that hereinbefore described in Reference Example 9, but replacing the 5-amino-4-cyano-1-(2,6-dichloro-4-trifluoromethylphenyl)-3-trifluoromethylpyrazole by 5-amino-4-cyano-1-(2,6-dichloro-4-trifluoromethylphenyl)-3-fluoropyrazole, there was obtained 4-cyano-1-(2,6-dichloro-4-trifluoromethylphenyl)-3-fluoropyrazole in the form of white crystals, m.p. 120-121 °C.

EXAMPLE 13

Compounds Nos. 92 and 93

Trifluoroacetic anhydride (3.5 ml) was added dropwise to a stirred mixture of 85% w/v hydrogen peroxide solution (0.56 ml) in dichloromethane (15 ml) maintaining at 0-10 °C. After warming to 20 °C during 5 minutes, a solution of 3-amino-1-(2,6-dichloro-4-trifluoromethyl-phenyl)-4-cyanopyrazole (1.0 g; hereinafter described in Reference Example 13 in dichloromethane (10 ml) was added dropwise over 5 minutes. A temperature rise of 10 °C was observed during the addition, and the mixture heated under reflux for 1.5 hours. After cooling, the solution was poured onto excess water, and the organic solution washed in turn with solutions of sodium bicarbonate and sodium bisulphite. Drying over anhydrous magnesium sulphate, followed by evaporation in vacuo gave a buff solid, which was purified by chromatography on silica (Merck, 40-230 mesh, 0.7 kg $cm^{-2}$) eluting with dichloromethane. The resultant white solid was recrystallised from a mixture of dichloromethane and hexane to give

1-(2,6-dichloro-4-trifluoromethylphenyl)-4-cyano-3-nitropyrazole as white crystals (0.7 g), m.p. 163-165 °C.

By proceeding in a similar manner but replacing 3-amino-1-(2,6-dichloro-4-trifluoromethylphenyl)-4-cyanopyrazole by 5-amino-1-(2,6-dichloro-4-trifluoromethylphenyl)-3,4-dicyanopyrazole (hereinbefore described in Example 2), there was obtained:-

1-(2,6-Dichloro-4-trifluoromethylphenyl)-3,4-dicyano-5-nitropyrazole as orange crystals, m.p. 138-140 °C, after recrystallisation from cyclohexane.

REFERENCE EXAMPLE 13

3-Amino-1-(2,6-dichloro-4-trifluoromethylphenyl)-4-cyanopyrazole was prepared as follows:-

A solution of 3-tert-butoxycarbonylamino-1-(2,6-dichloro-4-trifluoromethylphenyl)-4-cyanopyrazole (2.8 g) in ethanol (100 ml) was treated with 50% v/v hydrochloric acid (10 ml), and the mixture heated under reflux for 1 hour. After standing overnight at room temperature, sodium carbonate was added to pH 8, and the mixture extracted three times with dichloromethane. The extract was washed with water, dried over

anhydrous magnesium sulphate, and evaporated in vacuo to give a buff solid. Recrystallisation from a mixture of ethyl acetate and petroleum ether gave the title compound (1.4 g) in the form of white crystals, m.p. 159-160 °C.

3-tert-Butoxycarbonylamino-1-(2,6-dichloro-4-trifluoromethylphenyl)-4-cyanopyrazole was prepared as follows:-

A mixture of 3-carboxy-4-cyano-1-(2,6-dichloro-4-trifluoromethylphenyl)pyrazole (11 g) and thionyl chloride (35 ml) and N,N-dimethylformamide (3 drops) was heated under reflux for 2 hours. The solvent was evaporated in vacuo, and re-evaporated in vacuo after addition of dry toluene (20 ml). The resultant gum was dissolved in dry acetone (50 ml) and stirred, whilst a solution of sodium azide (2.9 g) in water (15 ml) was added during 5 minutes keeping at 10-15 °C. After 30 minutes the mixture was poured onto water (250 ml) and extracted with dichloromethane (3 x 80 ml). The combined extract was washed with water, dried over anhydrous magnesium sulphate, and evaporated in vacuo at equal to or below 40 °C to give a buff solid (13 g).

The resulting azide was dissolved in dry toluene (200 ml) and heated under reflux for half-an-hour, with smooth evolution of nitrogen. After cooling, this was treated with tert-butanol (40 g), and the mixture heated under reflux overnight. After evaporation in vacuo, the resulting brown oil (15 g) was purified by chromatography on silica (Merck, 230-400 mesh, 0.7 kg $cm^2$) eluting with dichloromethane and ethyl acetate (98:2) to give the title compound (8.0 g) as a white solid, m.p. 154-155 °C.

3-Carboxy-4-cyano-1-(2,6-dichloro-4-trifluoromethylphenyl)pyrazole was prepared as follows:-

A suspension of 4-cyano-1-(2,6-dichloro-4-trifluoromethylphenyl)-3-ethoxycarbonylpyrazole (5.0 g) in ethanol (100 ml) was treated with a solution of sodium hydroxide (0.63 g) in water (15 ml) and stirred at room temperature for 1.5 hours. After evaporation in vacuo at equal to or below 40 °C, the residue was dissolved in water (150 ml) and extracted with dichloromethane (1 x 100 ml). This extract was back-washed with water (2 x 50 ml), and the combined aqueous solutions brought to pH 1 with dilute hydrochloric acid, and then extracted with ethyl acetate (3 x 50 ml). This extract was dried over anhydrous magnesium sulphate, and evaporated in vacuo to give a buff solid (4.6 g). Recrystallisation from a mixture of toluene and hexane gave the title compound in the form of buff crystals (4.4 g), m.p. 203-205 °C.

4-Cyano-1-(2,6-dichloro-4-trifluoromethylphenyl)-3-ethoxycarbonylpyrazole was prepared by following the method described in Reference Example 9, and replacing 5-amino-4-cyano-1-(2,6-dichloro-4-trifluoromethylphenyl)-3-trifluoromethylpyrazole by 5-amino-4-cyano-1-(2,6-dichloro-4-trifluoromethylphenyl)-3-ethoxycarbonylpyrazole. The title compound was obtained in the form of buff crystals, m.p: 198-199 °C.

EXAMPLE 14

Compounds Nos. 94, 95 and 96

Silver (I) fluoride (5 g) was added in portions during 40 minutes to a vigorously stirred solution of 1,1-dichloro-2,2-dicyanoethylene in acetonitrile (15 ml), maintained at 0-10 °C by external cooling. The stirring was continued at room temperature for 1 hour, and the solid filtered off. The filtrate containing 1,1-difluoro-2,2-dicyanoethylene was stirred and cooled whilst a solution of 2,6-dichloro-4-trifluoromethylphenylhydrazine (4.9 g) in acetonitrile (15 ml) was added dropwise at 5 °C. After stirring overnight the solid was filtered off and the filtrate evaporated in vacuo to give a dark orange oil (6 g). This was purified by chromatography or silica (Merck, 230-400 mesh, 10 lb $in^{-2}$) eluting with dichloromethane to give a white solid. Recrystallisation from a mixture of cyclohexane and ethyl acetate gave 5-amino-1-(2,6-dichloro-4-trifluoromethylphenyl)-4-cyano-3-fluoropyrazole (0.9 g) as a white solid, m.p. 193-194 °C.

By proceeding in a similar manner but replacing the 2,6-dichloro-4-trifluoromethylphenyl- hydrazine by 2,6-dichloro-4-trifluoromethoxyphenyl- hydrazine and by employing 1,1-dichloro- 2,2-dicyanoethylene instead of 1,1-difluoro- 2,2-dicyanoethylene, and by using diethyl ether as solvent, there was prepared 5-amino-3-chloro-1-(2,6-dichloro-4-trifluoromethoxyphenyl)-4-cyanopyrazole in the form of a yellow solid, m.p. 175-177 °C.

By proceeding as immediately above but replacing the 2,6-dichloro-4-trifluoromethoxyphenylhydrazine by 2,6-dichloro-3,5-difluoro-4-trifluoromethylphenylhydrazine, there was prepared 5-amino-3-chloro-4-cyano-1-(2,6-dichloro-3,5-difluoro-4-trifluoromethylphenyl)pyrazole, in the form of yellow crystals, m.p. 206-208 °C.

REFERENCE EXAMPLE 14

A stirred solution of 4-cyano-1-(2,6-dichloro-4-trifluoromethylphenyl)-3-trifluoromethylpyrazole (1.5 g) in dry tetrahydrofuran cooled to -78 °C was treated with a solution of n-butyl lithium (2.6 M in hexane, 1.71 ml)

dropwise under nitrogen. The temperature was kept below -65°C during the addition, and the resultant solution kept at -78°C for 1 hour. A solution of trimethylsilyl chloride (0.56 ml) in dry tetrahydrofuran (2 ml) was then added, dropwise, during 2 minutes. The mixture was allowed to reach room temperature over 2 hours, left overnight and evaporated in vacuo to give a pale yellow solid. This was dissolved in dichloromethane, washed with water, dried over anhydrous magnesium sulphate, and evaporated in vacuo. The product was recrystallised from hexane to give 4-cyano-1-(2,6-dichloro-4-trifluoromethylphenyl)-3-trifluoromethyl-5-trimethylsilylpyrazole as white crystals, m.p. 108-110°C.

EXAMPLE 15

Compounds Nos. 106

5-Carbamoyl-4-cyano-1-(2,6-dichloro-4-trifluoromethylphenyl)-3-trifluoromethylpyrazole (3.57 g) was heated to 200°C with phosphorus pentoxide (2.82 g) with stirring. After 3 hours, the cooled product was treated with ice, and extracted with dichloromethane (3 x 50 ml). The organic solution was washed with water, dried over anhydrous magnesium sulphate, and evaporated in vacuo to give a solid. Recrystallisation from hexane gave 1-(2,6-dichloro-4-trifluoromethylphenyl)-4,5-dicyano-3-trifluoromethylpyrazole in the form of white crystals (1.8 g), m.p. 80°C.

By proceeding in a similar manner but replacing the 5-carbamoyl-4-cyano-1-(2,6-dichloro-4-trifluoromethylphenyl)-3-trifluoromethylpyrazole by 5-amino-3-carbamoyl-1-(2,6-dichloro-4-trifluoromethylphenyl)-4-methanesulphonylpyrazole there was prepared:-

5-Amino-3-cyano-1-(2,6-dichloro-4-trifluoromethylphenyl)-4-methanesulphonylpyrazole in the form of a white solid, m.p. 214°C.

REFERENCE EXAMPLE 15

5-Carbamoyl-4-cyano-1-(2,6-dichloro-4-trifluoromethylphenyl)-3-trifluoromethylpyrazole used in the above Example 15, was prepared as follows:-

5-Carboxy-4-cyano-1-(2,6-dichloro-4-trifluoromethylphenyl)-3-trifluoromethylpyrazole (6.0 g; hereinbefore described in Reference Example 14) was added to thionyl chloride (30 ml) and the stirred solution heated to reflux for 4 hours. The solvent was evaporated in vacuo, and re-evaporated after addition of dry toluene (30 ml). The resultant orange oil was dissolved in dry ether (10 ml) and added dropwise to a stirred solution of ammonia (0.88, 20 ml) cooled by an ice bath. After stirring overnight, water (150 ml) was added, and the mixture extracted with dichloromethane (3 x 50 ml). The combined extract was washed with water, dried over anhydrous magnesium sulphate, and evaporated in vacuo to give a white solid (7.0 g). Recrystallisation from a mixture of ethyl acetate and petroleum ether gave the title compound (4.3 g), in the form of white crystals, m.p. 180-181°C.

5-Amino-3-carbamoyl-1-(2,6-dichloro-4-trifluoromethylphenyl)-4-methanesulphonylpyrazole used in the above Example 15 was prepared by the same procedure, but by replacing the 5-carboxy-4-cyano-1-(2,6-dichloro-4-trifluoromethylphenyl)-3-trifluoromethylpyrazole by 5-amino-3-carboxy-1-(2,6-dichloro-4-trifluoromethylphenyl)-4-methanesulphonylpyrazole. The title compound was obtained in the form of an off-white solid, m.p. 223-224°C.

5-Amino-3-carboxy-1-(2,6-dichloro-4-trifluoromethylphenyl)-4-methanesulphonylpyrazole used above was prepared as follows:-

5-Amino-1-(2,6-dichloro-4-trifluoromethylphenyl)-3-ethoxycarbonyl-4-methanesulphonylpyrazole (8.15 g) was added to stirred 80% sulphuric acid (80 ml), and heated at 100°C for 5 hours. After cooling, the solution was poured onto ice, the solid filtered off and dried over phosphorus pentoxide in a vacuum desiccator. Recrystallisation from a mixture of methanol and petroleum ether gave the title compound as a white solid, m.p. 203-205°C.

5-Amino-1-(2,6-dichloro-4-trifluoromethylphenyl)-3-ethoxycarbonyl-4-methanesulphonylpyrazole, used above, was prepared by the procedure of Reference Example 9, by replacing malononitrile by methanesulphonylacetonitrile. The title compound was obtained in the form of a white solid, m.p. 255°C, after recrystallisation from ethanol.

In the following formulae it is to be understood that:

$A^a$ represents

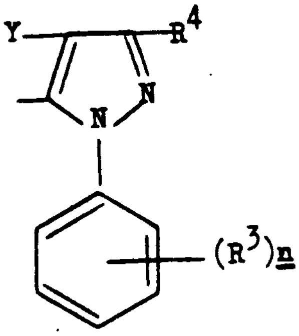

$A^b$ represents

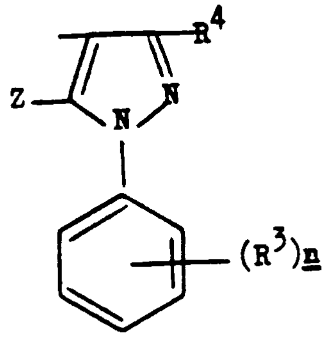

$A^c$ represents

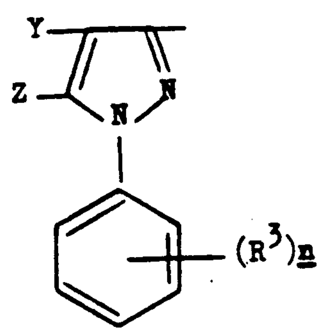

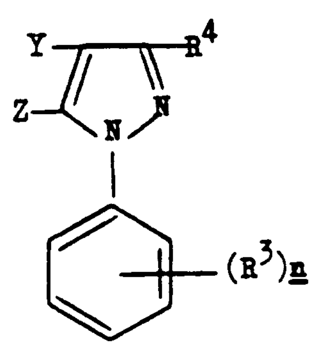

I

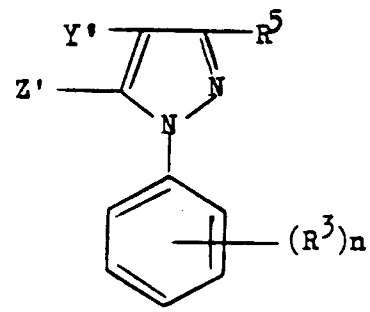

I A

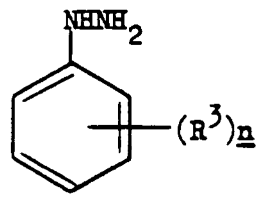

II

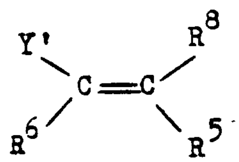

III

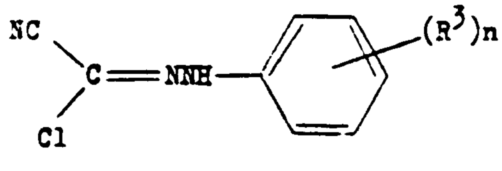

IV

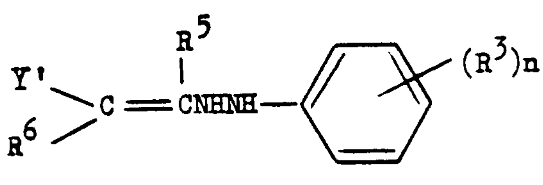

V

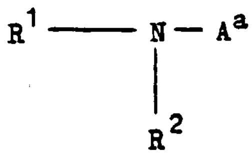

I B

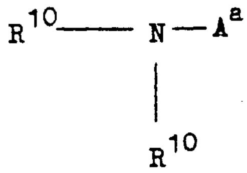 VIII

$H—A^b$ XI

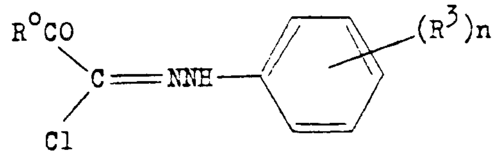 XIII

$ClO_2S—A^b$ XIV

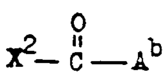 XV

$HOOC—A^b$ XVI

$RS—A^b$ I C

$A^b—S—S—A^b$ XVII

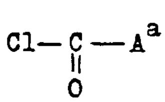 XVIII

$A^c—COOR^{20}$ XIX

$A^c—COOH$ XX

## Claims

### Claims for the following Contracting States : BE, CH, DE, FR, GB, GR, IT, LI, LU, NL, SE

1. A method for the control of arthropod, plant nematode or helminth pests at a locus which comprises treatment of the locus with an effective amount of a compound of the general formula:

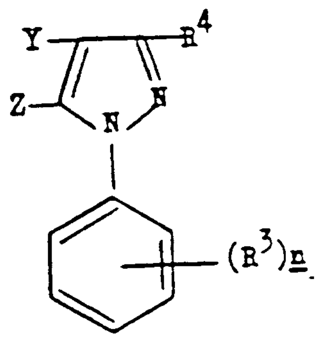

I

wherein Y represents a halogen atom, the cyano or nitro group or a group $RSO_2$, RSO or RS in which R represents a straight- or branched-chain alkyl group containing from 1 to 6 carbon atoms which is unsubstituted or substituted by one or more halogen atoms, a cycloalkyl group containing from 3 to 5 carbon atoms, a straight- or branched-chain alkenyl group containing from 2 to 6 carbon atoms, or Y represents the thiocyanato group, the sulphamoyl group which is unsubstituted or substituted by one or two straight- or branched-chain alkyl groups which may be the same or different and contain from 1 to 6 carbon atoms, the carbamoyl group which may be unsubstituted or substituted by one or two straight- or branched-chain alkyl groups which may be the same or different and contain from 1 to 6 carbon atoms, a straight- or branched-chain alkoxycarbonyl group containing from 2 to 7 carbon atoms, a straight- or branched-chain alkanoyl group containing from 2 to 7 carbon atoms, or a straight- or branched-chain alkyl group containing from 1 to 6 carbon atoms which is unsubstituted or substituted by one or more halogen toms, Z represents the hydrogen atom, or the amino group $-NR^1R^2$ wherein $R^1$ and $R^2$, which may be the same or different, each represents a hydrogen atom or a straight- or branched-chain alkyl group (containing from 1 to 6 carbon atoms, and which is unsubstituted or substituted by straight- or branched-chain alkoxycarbonyl of 2 to 5 carbon atoms), cycloalkyl group containing from 3 to 6 carbon atoms, formyl group, straight- or branched-chain alkanoyl group (which contains from 2 to 7 carbon atoms or together form a 5 or 6 membered cyclic imide with the nitrogen atom to which they are attached and themselves are unsubstituted or substituted by one or more halogen atoms) or cycloalkylcarbonyl group (which contains from 4 to 7 carbon atoms) or straight- or branched-chain alkoxycarbonyl group (which contains from 2 to 7 carbon atoms and themselves are unsubstituted or substituted by one or more halogen atoms), or Z represents a straight- or branched-chain alkylsulphenylamino group containing from 1 to 4 carbon atoms, a straight- or branched-chain alkoxymethyleneamino group containing from 2 to 5 carbon atoms which is unsubstituted or substituted on methylene by a straight- or branched-chain alkyl group containing from 1 to 4 carbon atoms, or represents a halogen atom, a straight-or branched-chain alkyl group containing from 1 to 4 carbon atoms, the carboxy group, or a straight- or branched-chain alkylthio, alkylsulphinyl or alkylsulphonyl group containing from 1 to 6 carbon atoms which is unsubstituted or substituted by one or more halogen atoms, or represents a straight- or branched-chain trialkylsilylmethyl group containing from 1 to 6 carbon atoms in each alkyl group which may be the same or different, a trialkylsilyl group containing from 1 to 6 carbon atoms in each alkyl group which may be the same or different or the cyano or nitro group, $R^3$ represents a halogen atom, a straight- or branched-chain alkyl or alkoxy group containing from 1 to 4 carbon atoms which is unsubstituted or substituted by one or more halogen atoms, a straight- or branched-chain alkylthio or alkylsulphinyl group containing from 1 to 4 carbon atoms which is substituted by one or more halogen atoms, the nitro or cyano group or a straight- or branched-chain alkylsulphonyl group containing from 1 to 4 carbon atoms which is unsubstituted or substituted by one or more halogen atoms, and $R^4$ represents a halogen atom, a cyano or nitro group, and

n represents an integer from 1 to 5 inclusive,

and, when Z represents a carboxy group, salts thereof with pesticidally-acceptable bases,

provided that

(A) $R^4$ , Y and Z do not simultaneously represent three groups of the same genus selected from the genera
(i) nitro,
(ii) cyano, and
(iii) halogen and
(B) with the exclusion of a method for the treatment of the human or animal body carried out by a medical or veterinary practitioner as therapy.

**2.** A method according to claim 1 wherein in general formula I $(R^3)_n$ represents
2,4,6-trichloro,
2,3,5,6-tetrachloro,
2-chloro-4-trifluoromethyl,
2,3,5,6-tetrafluoro-4-trifluoromethyl,
2,6-dichloro-4-trifluoromethylthio,
2-chloro-3,5,6-trifluoro-4-trifluoromethyl,
2,6-dichloro-3,5-difluoro-4-trifluoromethyl,
2,6-dichloro-4-nitro,
2,6-dichloro-4-trifluoromethylsulphinyl,
2,6-dichloro-4-methanesulphonyl or
2,6-dichloro-4-trifluoromethanesulphonyl substitution.

**3.** A method according to claim 1 wherein in general formula I $(R^3)$n represents
2,6-dichloro-4-trifluoromethyl or
2,6-dichloro-4-trifluoromethoxy substitution.

**4.** A method according to claim 1 or 3 wherein the compound of general formula I is
5-amino-1-(2,6-dichloro-4-trifluoromethylphenyl)-3,4-dicyanopyrazole or
5-amino-3-cyano-1-(2,6-dichloro-4-trifluoromethylphenyl)-4-methanesulphonylpyrazole.

**5.** Use as an arthropodicide, plant nematocide or anthelmintic of a composition which comprises at least one compound of general formula I wherein the various symbols are as defined in claim 1, or a pesticidally acceptable salt thereof, in association with one or more compatible diluents or carriers with the provisos that
(1) when
$R^4$ represents chloro,
Y represents nitro, and
Z represents methyl and
$(R^3)_n$ represents 4-nitro,
the composition comprises a pharmaceutically acceptable adjuvant or a feedstuff or is substantially sterile and pyrogen-free or is in unit dosage form ; and
(2) excluding compositions comprising 1-(4-nitrophenyl)-3-nitro-4-cyano or -carboxamide pyrazole ,

**6.** An arthropodicidal, plant nematocidal or anthelmintic composition which comprises at least one compound of general formula I wherein the various symbols are as defined in claim 1 , or a pesticidally acceptable salt thereof, in association with one or more compatible diluents or carriers with the provisos that
(1) when
$R^4$ represents chloro,
Y represents nitro, and
Z represents methyl and
$(R^3)_n$ represents 4-nitro,
the composition is not a plant nematocidal composition and comprises a pharmaceutically acceptable adjuvant or a feedstuff or is substantially sterile and pyrogen-free or is in unit dosage form;
and (2)
excluding compositions comprising 1-(4-nitrophenyl)-3-nitro-4-pyrazole-carbonitrile or carboxamide.

**7.** A compound of general formula I, wherein the various symbols are as defined in claim 1, and salts thereof, with the exclusion of the compounds wherein:

$R^4$ represents chloro,
Y represents nitro, and
Z represents methyl and $(R^3)_n$ represents 4-nitro;
and
$R^4$ represents nitro,
Y represents cyano or $CONH_2$,
Z represents hydrogen and
$(R^3)_n$ represents 4-nitro substitution;

$R^4$ and Y both represent cyano, Z represents amino and $(R^3)_n$ represents 3- or 4-nitro or 4-chloro substitution;

$R^4$ represents bromine, Y represents methyl and Z represents hydrogen or $R^4$ and Y represent bromine and Z represents methyl and $(R^3)_n$ represents 2,4-dinitrophenyl or 2,4,6-trinitrophenyl substitution;

$R^4$ and Y represent bromine and Z represents hydrogen, $R^4$ and Y represent chlorine and Z represents methyl, and $(R^3)_n$ represents 2,4-dinitrophenyl substitution;

$R^4$ represents chlorine, Y represents bromine and Z represents methyl and $(R^3)_n$ represents 2,4-dinitrophenyl substitution;

and with the exclusion of compounds of formula (I) wherein

$R^4$, Y and Z represent, respectively, nitro, methyl and hydrogen and $(R^3)_n$ represents 2,4,6-trinitrophenyl;

$R^4$, Y and Z represent, respectively, bromine, bromine and hydrogen and $(R^3)_n$ represents 4-bromophenyl;

$(R^3)_n$ represents 2,4-dinitrophenyl and $R^4$, Y and Z represent, respectively,
chlorine, methyl and hydrogen; chlorine, bromine and hydrogen;

$(R^3)_n$ represents 4-nitrophenyl and $R^4$, Y and Z represent, respectively, bromine, methyl and hydrogen ; nitro, methyl and hydrogen ; and cyano, methoxycarbonyl and amino

$R^4$, Y and Z represent, respectively, nitro, methyl and nitro and $(R^3)_n$ represents 2,4-dinitrophenyl.

**8.** A compound according to claim 7 wherein $(R^3)_n$ represents
2,4,6-trichloro,
2,3,5,6-tetrachloro,
2-chloro-4-trifluoromethyl,
2,3,5,6-tetrafluoro-4-trifluoromethyl,
2,6-dichloro-4-trifluoromethylthio,
2-chloro-3,5,6-trifluoro-4-trifluoromethyl,
2,6-dichloro-3,5-difluoro-4-trifluoromethyl,
2,6-dichloro-4-nitro,
2,6-dichloro-4-trifluoromethylsulphinyl,
2,6-dichloro-4-methanesulphonyl or
2,6-dichloro-4-trifluoromethanesulphonyl substitution.

**9.** A compound according to claim 7 wherein $(R^3)_n$ represents
2,6-dichloro-4-trifluoromethyl or
2,6-dichloro-4-trifluoromethoxy substitution.

**10.** A compound according to claim 7 which is
5-amino-1-(2,6-dichloro-4-trifluoromethyl-phenyl)-3,4-dicyanopyrazole, or
5-amino-3-cyano-1-(2,6-dichloro-4-trifluoromethylphenyl)-4-methane sulphonylpyrazole.

**11.** A process for the preparation of a compound of general formula I depicted in claim 1 wherein the various symbols are as defined in claim 7 which comprises:

(A) when the compound of general formula I conforms to the general formula (IA)

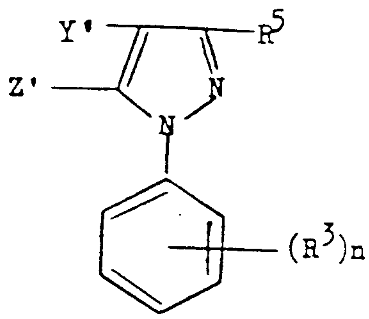

IA

wherein Y' represents the cyano or nitro group or a group $RSO_2$, RSO or RS, wherein R is as defined in claim 1, a straight- or branched-chain alkoxycarbonyl group containing from 2 to 7 carbon atoms, or a straight- or branched-chain alkyl group containing from 1 to 6 carbon atoms which is unsubstituted or substituted by one or more halogen atoms, Z' represents the unsubstituted amino group or a straight- or branched-chain alkyl group containing from 1 to 4 carbon atoms, and $R^5$ represents a fluorine, chlorine or bromine atom, the cyano group,

(i) the reaction of a compound of the general formula:

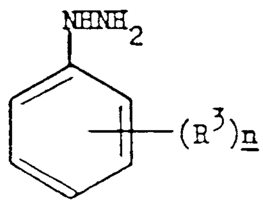

II

wherein $R^3$ and n are as defined in claim 1, or an acid addition salt thereof with (1), when $R^5$ in the compound of general formula IA represents a fluorine, chlorine or bromine atom, a compound of the general formula:

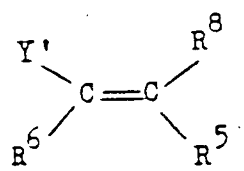

III

wherein Y' and $R^5$ are as hereinbefore defined, $R^6$ represents the cyano group or a straight- or branched-chain alkanoyl group containing from 2 to 5 carbon atoms and $R^8$ represents a straight- or branched-chain alkoxy group containing from 1 to 4 carbon atoms, the hydroxy group or a fluorine, chlorine or bromine atom, or

(2) when $R^5$ in the compound of general formula IA represents the cyano group, Y' represents the cyano group and Z' represents the unsubstituted amino group, with tetracyanoethylene;

optionally isolating, before its cyclisation to the compound of general formula IA, the intermediate of general formula:

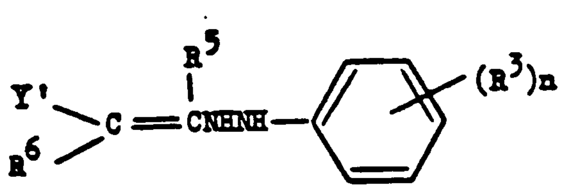

V

wherein $R^3$, n, $R^5$, $R^6$ and Y' are as hereinbefore defined, formed by reaction of a compound of general formula II with a compound of general formula III or tetracyano-ethylene;

(B) when Z' in general formula IA represents the unsubstituted amino group, by reacting a compound of general formula $Y'CH_2CN$ with a compound of general formula II in the presence of a compound of the general formula $R^7C(R^0)_3$ wherein $R^7$ represents a straight- or branched-chain alkyl group containing from 1 to 4 carbon atoms which is unsubstituted or substituted by one or more halogen atoms or a cycloalkyl group containing from 3 to 6 carbon atoms and $R^0$ represents an alkoxy group in an inert organic solvent at a temperature from ambient to reflux;

(C) when Z' in general formula IA represents the unsubstituted amino group and $R^5$ represents the cyano group by the reaction of a compound of the general formula

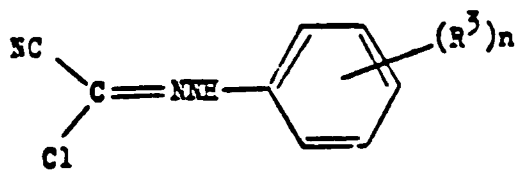

IV

wherein $R^3$ and n are as defined in claim 1, with a compound of general formula $Y'CH_2CN$, wherein Y' is as hereinbefore defined;

and preparing other compounds of general formula I by the conversion, as hereinbefore described, of one or more substituents Y, Z, $R^3$ and $R^4$, or substituents corresponding thereto, into substituents Y, Z, $R^3$ and $R^4$ as defined in claim 1; and, when Z represents the carboxy group, optionally converting a compound of general formula I into a salt thereof.

**12.** Intermediate compounds of general formula I wherein Y represents the hydrogen atom, the formyl or carboxy group, a straight- or branched-chain alkanoyl group containing from 2 to 6 carbon atoms which is substituted by one or more halogen atoms, the dithio group (which joins two pyrazole rings), the amino group, the $-SO_2Cl$ group, a straight- or branched-chain carboxyalkyl group containing from 2 to 6 carbon atoms, Z represents the carbamoyl group or a straight- or branched-chain alkoxycarbonyl group containing from 2 to 7 carbon atoms or the diphenoxycarbonylamino group, $(R^3)_n$ substitution is as defined in claim 2 or 3 and $R^4$ represents the amino, hydroxymethyl, carboxy or carbamoyl group or a straight- or branched-chain alkoxycarbonyl or alkoxycarbonylamino group containing from 2 to 7 carbon atoms.

**13.** A compound of general formula I as defined in claim 1, or a pesticidally acceptable salt thereof, for use in the manufacture of a medicament for the treatment of an arthropod or helminth infection.

**Claims for the following Contracting States : AT, ES**

**1.** A process for the preparation of a compound of the general formula:

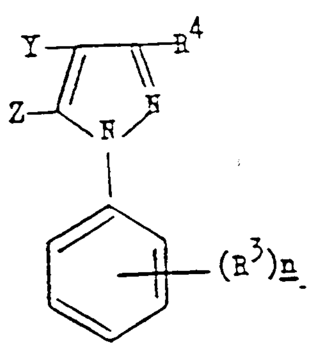

I

wherein Y represents a halogen atom, the cyano or nitro group or a group $RSO_2$, RSO or RS in which R represents a straight- or branched-chain alkyl group containing from 1 to 6 carbon atoms which is

unsubstituted or substituted by one or more halogen atoms, a cycloalkyl group containing from 3 to 5 carbon atoms, a straight- or branched-chain alkenyl group containing from 2 to 6 carbon atoms, or Y represents the thiocyanato group, the sulphamoyl group which is unsubstituted or substituted by one or two straight- or branched-chain alkyl groups which may be the same or different and contain from 1 to 6 carbon atoms, the carbamoyl group which may be unsubstituted or substituted by one or two straight- or branched-chain alkyl groups which may be the same or different and contain from 1 to 6 carbon atoms, a straight- or branched-chain alkoxycarbonyl group containing from 2 to 7 carbon atoms, a straight- or branched-chain alkanoyl group containing from 2 to 7 carbon atoms, or a straight- or branched-chain alkyl group containing from 1 to 6 carbon atoms which is unsubstituted or substituted by one or more halogen atoms, Z represents the hydrogen atom, or the amino group $-NR^1R^2$ wherein $R^1$ and $R^2$, which may be the same or different, each represents a hydrogen atom or a straight- or branched-chain alkyl group (containing from 1 to 6 carbon atoms, and which is unsubstituted or substituted by straight- or branched-chain alkoxycarbonyl of 2 to 5 carbon atoms), cycloalkyl group containing from 3 to 6 carbon atoms, formyl group, straight- or branched-chain alkanoyl group (which contains from 2 to 7 carbon atoms or together form a 5 or 6 membered cyclic imide with the nitrogen atom to which they are attached and themselves are unsubstituted or substituted by one or more halogen atoms) or cycloalkylcarbonyl group (which contains from 4 to 7 carbon atoms) or straight- or branched-chain alkoxycarbonyl group (which contains from 2 to 7 carbon atoms and themselves are unsubstituted or substituted by one or more halogen atoms), or Z represents a straight- or branched-chain alkylsulphenylamino group containing from 1 to 4 carbon atoms, a straight- or branched-chain alkoxymethyleneamino group containing from 2 to 5 carbon atoms which is unsubstituted or substituted on methylene by a straight- or branched-chain alkyl group containing from 1 to 4 carbon atoms, or represents a halogen atom, a straight- or branched-chain alkyl group containing from 1 to 4 carbon atoms, the carboxy group, or a straight- or branched-chain alkylthio, alkylsulphinyl or alkylsulphonyl group containing from 1 to 6 carbon atoms which is unsubstituted or substituted by one or more halogen atoms, or represents a straight- or branched-chain trialkylsilylmethyl group containing from 1 to 6 carbon atoms in each alkyl group which may be the same or different, a trialkylsilyl group containing from 1 to 6 carbon atoms in each alkyl group which may be the same or different or the cyano or nitro group, $R^3$ represents a halogen atom, a straight- or branched-chain alkyl or alkoxy group containing from 1 to 4 carbon atoms which is unsubstituted or substituted by one or more halogen atoms, a straight- or branched-chain alkylthio or alkylsulphinyl group containing from 1 to 4 carbon atoms which is substituted by one or more halogen atoms, the nitro or cyano group or a straight- or branched-chain alkylsulphonyl group containing from 1 to 4 carbon atoms which is unsubstituted or substituted by one or more halogen atoms, and $R^4$ represents a halogen atom, a cyano or nitro group, and
n represents an integer from 1 to 5 inclusive,

and, when Z represents a carboxy group, salts thereof with pesticidally-acceptable bases,

provided that

(A) $R^4$ , Y and Z do not simultaneously represent three groups of the same genus selected from the genera

(i) nitro,
(ii) cyano, and
(iii) halogen;

with the exclusion of the compounds wherein:

$R^4$ represents chloro,
Y represents nitro, and
Z represents methyl and $(R^3)_n$ represents 4-nitro;
and
$R^4$ represents nitro,
Y represents cyano or $CONH_2$,
Z represents hydrogen and
$(R^3)_n$ represents 4-nitro substitution;

$R^4$ and Y both represent cyano, Z represents amino and $(R^3)_n$ represents 3- or 4-nitro or 4-chloro substitution;

$R^4$ represents bromine, Y represents methyl and Z represents hydrogen or $R^4$ and Y represent bromine and Z represents methyl and $(R^3)_n$ represents 2,4-dinitrophenyl or 2,4,6-trinitrophenyl substitution;

$R^4$ and Y represent bromine and Z represents hydrogen, $R^4$ and Y represent chlorine and Z represents methyl, and $(R^3)_n$ represents 2,4-dinitrophenyl substitution;

$R^4$ represents chlorine, Y represents bromine and Z represents methyl and $(R^3)_n$ represents 2,4-

dinitrophenyl substitution;
and with the exclusion of compounds of formula (I) wherein
$R^4$, Y and Z represent, respectively, nitro, methyl and hydrogen and $(R^3)_n$ represents 2,4,6-trinitrophenyl;
$R^4$, Y and Z represent, respectively, bromine, bromine and hydrogen and $(R^3)_n$ represents 4-bromophenyl;
$(R^3)_n$ represents 2,4-dinitrophenyl and $R^4$, Y and Z represent, respectively, chlorine, methyl and hydrogen; chlorine, bromine and hydrogen;
$(R^3)_n$ represents 4-nitrophenyl and $R^4$, Y and Z represent, respectively, bromine, methyl and hydrogen ; nitro, methyl and hydrogen ; and cyano, methoxycarbonyl and amino
$R^4$, Y and Z represent, respectively, nitro, methyl and nitro and $(R^3)_n$ represents 2,4-dinitrophenyl;
which process comprises:

(A) when the compound of general formula I conforms to the general formula (IA)

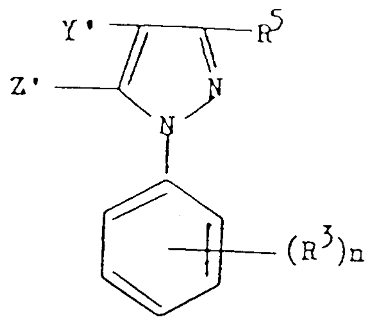

IA

wherein Y' represents the cyano or nitro group or a group $RSO_2$, RSO or RS, wherein R is as hereinbefore defined, a straight- or branched-chain alkoxycarbonyl group containing from 2 to 7 carbon atoms, or a straight- or branched-chain alkyl group containing from 1 to 6 carbon atoms which is unsubstituted or substituted by one or more halogen atoms, Z' represents the unsubstituted amino group or a straight- or branched-chain alkyl group containing from 1 to 4 carbon atoms, and $R^5$ represents a fluorine, chlorine or bromine atom, the cyano group,

(i) the reaction of a compound of the general formula:

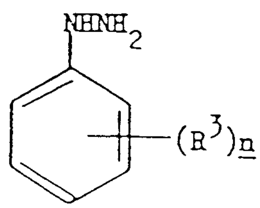

II

wherein $R^3$ and n are as hereinbefore defined, or an acid addition salt thereof with (1), when $R^5$ in the compound of general formula IA represents a fluorine, chlorine or bromine atom, a compound of the general formula:

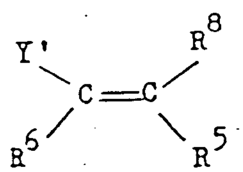

III

wherein Y' and $R^5$ are as hereinbefore defined, $R^6$ represents the cyano group or a straight- or branched-chain alkanoyl group containing from 2 to 5 carbon atoms and $R^8$ represents a straight- or branched-chain alkoxy group containing from 1 to 4 carbon atoms, the hydroxy group or a fluorine, chlorine or bromine atom, or

(2) when $R^5$ in the compound of general formula IA represents the cyano group, Y' represents the cyano group and Z' represents the unsubstituted amino group, with tetracyanoethylene;
optionally isolating, before its cyclisation to the compound of general formula IA, the intermediate of general formula:

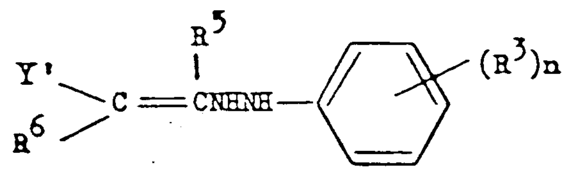

V

wherein $R^3$, n, $R^5$, $R^6$ and Y' are as hereinbefore defined, formed by reaction of a compound of general formula II with a compound of general formula III or tetracyano-ethylene;
(B) when Z' in general formula IA represents the unsubstituted amino group, by reacting a compound of general formula $Y'CH_2CN$ with a compound of general formula II in the presence of a compound of the general formula $R^7C(R^o)_3$ wherein $R^7$ represents a straight- or branched-chain alkyl group containing from 1 to 4 carbon atoms which is unsubstituted or substituted by one or more halogen atoms or a cycloalkyl group containing from 3 to 6 carbon atoms and $R^0$ represents an alkoxy group in an inert organic solvent at a temperature from ambient to reflux;
(C) when Z' in general formula IA represents the unsubstituted amino group and $R^5$ represents the cyano group by the reaction of a compound of the general formula

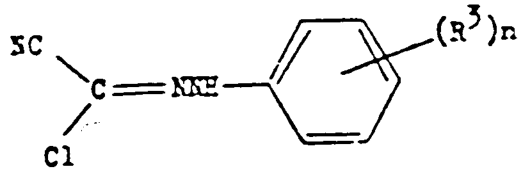

IV

wherein $R^3$ and n are as hereinbefore defined, with a compound of general formula $Y'CH_2CN$, wherein Y' is as hereinbefore defined;
and preparing other compounds of general formula I by the conversion, as hereinbefore described, of one or more substituents Y, Z, $R^3$ and $R^4$, or substituents corresponding thereto, into substituents Y, Z, $R^3$ and $R^4$ as hereinbefore defined; and, when Z represents the carboxy group, optionally converting a compound of general formula I into a salt thereof.

**2.** A process according to claim 1 wherein in general formula I $(R^3)_n$ represents
2,4,6-trichloro,
2,3,5,6-tetrachloro,
2-chloro-4-trifluoromethyl,
2,3,5,6-tetrafluoro-4-trifluoromethyl,
2,6-dichloro-4-trifluoromethylthio,
2-chloro-3,5,6-trifluoro-4-trifluoromethyl,
2,6-dichloro-3,5-difluoro-4-trifluoromethyl,
2,6-dichloro-4-nitro,
2,6-dichloro-4-trifluoromethylsulphinyl,
2,6-dichloro-4-methanesulphonyl or
2,6-dichloro-4-trifluoromethanesulphonyl substitution.

**3.** A process according to claim 1 wherein in general formula I $(R^3)_n$ represents
2,6-dichloro-4-trifluoromethyl or
2,6-dichloro-4-trifluoromethoxy substitution.

**4.** A process according to claim 1 or 3 wherein the compound of general formula I is
5-amino-1-(2,6-dichloro-4-trifluoromethylphenyl)-3,4-dicyanopyrazole or

5-amino-3-cyano-1-(2,6-dichloro-4-trifluoromethylphenyl)-4-methanesulphonylpyrazole.

**5.** An arthropodicidal, plant nematocidal or anthelmintic composition which comprises at least one compound of general formula I
wherein Y represents a halogen atom, the cyano or nitro group or a group $RSO_2$, RSO or RS in which R represents a straight- or branched-chain alkyl group containing from 1 to 6 carbon atoms which is unsubstituted or substituted by one or more halogen atoms, a cycloalkyl group containing from 3 to 5 carbon atoms, a straight- or branched-chain alkenyl group containing from 2 to 6 carbon atoms, or Y represents the thiocyanato group, the sulphamoyl group which is unsubstituted or substituted by one or two straight- or branched-chain alkyl groups which may be the same or different and contain from 1 to 6 carbon atoms, the carbamoyl group which may be unsubstituted or substituted by one or two straight- or branched-chain alkyl groups which may be the same or different and contain from 1 to 6 carbon atoms, a straight- or branched-chain alkoxycarbonyl group containing from 2 to 7 carbon atoms, a straight- or branched-chain alkanoyl group containing from 2 to 7 carbon atoms, or a straight- or branched-chain alkyl group containing from 1 to 6 carbon atoms which is unsubstituted or substituted by one or more halogen atoms, Z represents the hydrogen atom, or the amino group $-NR^1R^2$ wherein $R^1$ and $R^2$, which may be the same or different, each represents a hydrogen atom or a straight- or branched-chain alkyl group (containing from 1 to 6 carbon atoms, and which is unsubstituted or substituted by straight- or branched-chain alkoxycarbonyl of 2 to 5 carbon atoms), cycloalkyl group containing from 3 to 6 carbon atoms, formyl group, straight- or branched-chain alkanoyl group (which contains from 2 to 7 carbon atoms or together form a 5 or 6 membered cyclic imide with the nitrogen atom to which they are attached and themselves are unsubstituted or substituted by one or more halogen atoms) or cycloalkylcarbonyl group (which contains from 4 to 7 carbon atoms) or straight- or branched-chain alkoxycarbonyl group (which contains from 2 to 7 carbon atoms and themselves are unsubstituted or substituted by one or more halogen atoms), or Z represents a straight- or branched-chain alkylsulphenylamino group containing from 1 to 4 carbon atoms, a straight- or branched-chain alkoxymethyleneamino group containing from 2 to 5 carbon atoms which is unsubstituted or substituted on methylene by a straight- or branched-chain alkyl group containing from 1 to 4 carbon atoms, or represents a halogen atom, a straight- or branched-chain alkyl group containing from 1 to 4 carbon atoms, the carboxy group, or a straight- or branched-chain alkylthio, alkylsulphinyl or alkylsulphonyl group containing from 1 to 6 carbon atoms which is unsubstituted or substituted by one or more halogen atoms, or represents a straight- or branched-chain trialkylsilylmethyl group containing from 1 to 6 carbon atoms in each alkyl group which may be the same or different, a trialkylsilyl group containing from 1 to 6 carbon atoms in each alkyl group which may be the same or different or the cyano or nitro group, $R^3$ represents a halogen atom, a straight- or branched-chain alkyl or alkoxy group containing from 1 to 4 carbon atoms which is unsubstituted or substituted by one or more halogen atoms, a straight- or branched-chain alkylthio or alkylsulphinyl group containing from 1 to 4 carbon atoms which is substituted by one or more halogen atoms, the nitro or cyano group or a straight- or branched-chain alkylsulphonyl group containing from 1 to 4 carbon atoms which is unsubstituted or substituted by one or more halogen atoms, and $R^4$ represents a halogen atom, a cyano or nitro group, and
n represents an integer from 1 to 5 inclusive,
and, when Z represents a carboxy group, salts thereof with pesticidally-acceptable bases,
provided that
(A) $R^4$ , Y and Z do not simultaneously represent three groups of the same genus selected from the genera
(i) nitro,
(ii) cyano, and
(iii) halogen;
or a pesticidally acceptable salt thereof, in association with one or more compatible diluents or carriers with the provisos that
(1) when
$R^4$ represents chloro,
Y represents nitro, and
Z represents methyl and
$(R^3)_n$ represents 4-nitro,
the composition is not a plant nematocidal composition and comprises a pharmaceutically acceptable adjuvant or a feedstuff or is substantially sterile and pyrogen-free or is in unit dosage form;
and (2)

excluding compositions comprising
1-(4-nitrophenyl)-3-nitro-4-pyrazole-carbonitrile or carboxamide.

**6.** A method for the control of arthropod, plant nematode or helminth pests at a locus which comprises treatment of the locus with an effective amount of a compound of general formula I wherein the various symbols are as defined in claim 5, or a pesticidally acceptable salt thereof, in association with one or more compatible diluents or carriers with the provisos that

(1) when

| | |
|---|---|
| $R^4$ | represents chloro, |
| Y | represents nitro, and |
| Z | represents methyl and |
| $(R^3)_n$ | represents 4-nitro, |

the composition comprises a pharmaceutically acceptable adjuvant or a feedstuff or is substantially sterile and pyrogen-free or is in unit dosage form ; and

(2) excluding compositions comprising 1-(4-nitrophenyl)-3-nitro-4-cyano or -carboxamide pyrazole ,

(3) with the exclusion of a method for the treatment of the human or animal body carried out by a medical or veterinary practitioner as therapy.

**7.** A process according to claim 1 wherein the starting material is prepared from an intermediate compound of general formula I
wherein Y represents the hydrogen atom, the formyl or carboxy group, a straight- or branched-chain alkanoyl group containing from 2 to 6 carbon atoms which is substituted by one or more halogen atoms, the dithio group (which joins two pyrazole rings), the amino group, the $-SO_2Cl$ group, a straight- or branched-chain carboxyalkyl group containing from 2 to 6 carbon atoms, Z represents the carbamoyl group or a straight- or branched-chain alkoxycarbonyl group containing from 2 to 7 carbon atoms or the diphenoxycarbonylamino group, $(R^3)_n$ substitution is as defined in claim 2 or 3 and $R^4$ represents the amino, hydroxymethyl, carboxy or carbamoyl group or a straight- or branched-chain alkoxycarbonyl or alkoxycarbonylamino group containing from 2 to 7 carbon atoms.

**Patentansprüche**

**Patentansprüche für folgende Vertragsstaaten : BE, CH, DE, FR, GB, GR, IT, LI, LU, NL, SE**

**1.** Verfahren zur Bekämpfung von Gliederfüßler-, Pflanzenfadenwurm- oder Wurmschädlingen an bestimmten Orten oder Stellen durch Behandeln des betreffenden Orts bzw. der betreffenden Stelle mit einer wirksamen Menge einer Verbindung der allgemeinen Formel:

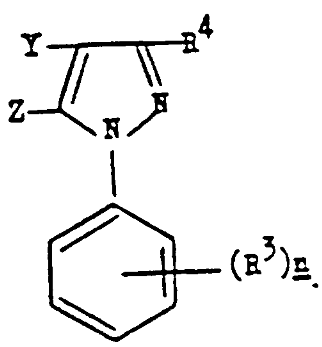

I

worin Y für ein Halogenatom, die Cyano- oder Nitrogruppe oder eine Gruppe $RSO_2$, RSO oder RS mit R gleich einer nicht-substituierten oder durch ein oder mehr Halogenatom(e) substituierten gerad- oder verzweigtkettigen Alkylgruppe mit 1 bis 6 Kohlenstoffatom(en), einer Cycloalkylgruppe mit 3 bis 5 Kohlenstoffatomen, einer gerad- oder verzweigtkettigen Alkenylgruppe mit 2 bis 6 Kohlenstoffatomen oder für die Thiocyanatgruppe, die nicht-substituierte oder durch eine oder mehr gerad- oder verzweigtkettige Alkylgruppe(n), die gleich oder verschieden sein können und 1 bis 6 Kohlenstoffatom(e) enthält (enthalten), substituierte Sulfamoylgruppe, die nicht-substituierte oder durch 1 oder 2 gerad- oder verzweigtkettige Alkylgruppe(n), die gleich oder verschieden sein können und 1 bis 6 Kohlenstoffatom-(e) enthalten, substituierte Carbamoylgruppe, eine gerad- oder verzweigtkettige Alkoxycarbonylgruppe

mit 2 bis 7 Kohlenstoffatomen, eine gerad- oder verzweigtkettige Alkanoylgruppe mit 2 bis 7 Kohlenstoffatomen oder eine gerad- oder verzweigtkettige Alkylgruppe mit 1 bis 6 Kohlenstoffatom(en), die nicht-substituiert oder durch ein oder mehr Halogenatom(e) substituiert ist, steht,

Z das Wasserstoffatom oder die Aminogruppe -$NR^1R^2$ mit $R^1$ und $R^2$, die gleich oder verschieden sein können, jeweils gleich einem Wasserstoffatom oder einer gerad- oder verzweigtkettigen Alkylgruppe (mit 1 bis 6 Kohlenstoffatom(en), die nicht-substituiert oder durch eine gerad- oder verzweigtkettige Alkoxycarbonylgruppe mit 2 bis 5 Kohlenstoffatomen substituiert ist), einer Cycloalkylgruppe mit 3 bis 6 Kohlenstoffatomen, einer Formylgruppe, einer gerad- oder verzweigtkettigen Alkanoylgruppe (mit 2 bis 7 Kohlenstoffatomen oder zusammen gleich einem 5- oder 6-gliedrigen cyclischen Imid mit dem Stickstoffatom, an dem sie hängen, wobei sie selbst nicht-substituiert oder durch ein oder mehrere Halogenatom(e) substituiert sind) oder einer Cycloalkylcarbonylgruppe (mit 4 bis 7 Kohlenstoffatomen) oder einer gerad- oder verzweigtkettigen Alkoxycarbonylgruppe (mit 2 bis 7 Kohlenstoffatomen, wobei sie selbst nicht-substituiert oder durch ein oder mehrere Halogenatom(e) substituiert sind) oder eine gerad- oder verzweigtkettige Alkylsulfenylaminogruppe mit 1 bis 4 Kohlenstoffatom(en), eine gerad- oder verzweigtkettige Alkoxymethylenaminogruppe mit 2 bis 5 Kohlenstoffatomen, die nicht-substituiert oder am Methylen durch eine gerad- oder verzweigtkettige Alkylgruppe mit 1 bis 4 Kohlenstoffatom(en) substituiert ist, oder ein Halogenatom, eine gerad- oder verzweigtkettige Alkylgruppe mit 1 bis 4 Kohlenstoffatom(en), die Carboxygruppe oder eine gerad- oder verzweigtkettige Alkylthio-, Alkylsulfinyl- oder Alkylsulfonylgruppe mit 1 bis 6 Kohlenstoffatom(en), die nicht-substituiert oder durch ein oder mehrere Halogenatom(e) substituiert ist, oder eine gerad- oder verzweigtkettige Trialkylsilylmethylgruppe mit 1 bis 6 Kohlenstoffatom(en) in der jeweiligen Alkylgruppe, wobei die Alkylgruppen gleich oder verschieden sein können, eine Trialkylsilylgruppe mit 1 bis 6 Kohlenstoffatom(en) in der jeweiligen Alkylgruppe, wobei die Alkylgruppen gleich oder verschieden sein können, oder die Cyano- oder Nitrogruppe bedeutet,

$R^3$ ein Halogenatom, eine gerad- oder verzweigtkettige Alkyl- oder Alkoxygruppe mit 1 bis 4 Kohlenstoffatom(en), die nicht-substituiert oder durch ein oder mehrere Halogenatom(e) substituiert ist, eine gerad- oder verzweigtkettige Alkylthio- oder Alkylsulfinylgruppe mit 1 bis 4 Kohlenstoffatom(en), die durch ein oder mehrere Halogenatom(e) substituiert ist, die Nitro- oder Cyanogruppe oder eine gerad- oder verzweigtkettige Alkylsulfonylgruppe mit 1 bis 4 Kohlenstoffatom(en), die nicht-substituiert oder durch ein oder mehrere Halogenatom(e) substituiert ist, darstellt,

$R^4$ einem Halogenatom, einer Cyano- oder Nitrogruppe entspricht und

n eine ganze Zahl von 1 bis einschließlich 5 bedeutet,

und im Falle, daß Z für eine Carboxygruppe steht, ihre Salze mit unter Pestizidgesichtspunkten akzeptablen Basen,

vorausgesetzt, daß

(A) $R^4$, Y und Z nicht gleichzeitig 3 Gruppen derselben Art, ausgewählt aus

(i) Nitro,

(ii) Cyano und

(iii) Halogen, bedeuten und

(B) ein Verfahren zur Behandlung des menschlichen oder tierischen Körpers, das von einem Arzt oder Tierarzt als Therapie durchgeführt wird, ausgeschlossen ist.

**2.** Verfahren nach Anspruch 1, wobei in der allgemeinen Formel I $(R^3)_n$ eine
2,4,6-Trichlor-,
2,3,5,6-Tetrachlor-,
2-Chlor-4-trifluormethyl-,
2,3,5,6-Tetrafluor-4-trifluormethyl-,
2,6-Dichlor-4-trifluormethylthio-,
2-Chlor-3,5,6-trifluor-4-trifluormethyl-,
2,6-Dichlor-3,5-difluor-4-trifluormethyl-,
2,6-Dichlor-4-nitro-,
2,6-Dichlor-4-trifluormethylsulfinyl-,
2,6-Dichlor-4-methansulfonyl- oder
2,6-Dichlor-4-trifluormethansulfonylsubstitution bedeutet.

**3.** Verfahren nach Anspruch 1, wobei in der allgemeinen Formel I $(R^3)_n$ eine
2,6-Dichlor-4-trifluormethyl oder
2,6-Dichlor-4-trifluormethoxysubstitution bedeutet.

4. Verfahren nach Anspruch 1 oder 3, wobei die Verbindung der allgemeinen Formel I
5-Amino-1-(2,6-dichlor-4-trifluormethylphenyl)-3,4-dicyanopyrazol oder
5-Amino-3-cyano-1-(2,6-dichlor-4-trifluormethylphenyl)-4-methansulfonylpyrazol bedeutet.

5. Verwendung einer Zubereitung mit mindestens einer Verbindung der allgemeinen Formel I, worin die zahlreichen Symbole die in Anspruch 1 angegebene Bedeutung besitzen, oder eines aus Pestizidgesichtspunkten akzeptablen Salzes derselben in Verbindung mit einem oder mehreren verträglichen Verdünnungsmittel(n) oder Träger(n) als Arthropodicid, Pflanzennematocid oder Wurmmittel, wobei gilt, daß

(1) wenn
$R^4$ für Chlor steht,
Y Nitro bedeutet,
Z Methyl entspricht und
$(R^3)_n$ für 4-Nitro steht,
die Zubereitung einen pharmazeutisch akzeptablen Hilfsstoff oder ein Futtermittel umfaßt oder im wesentlichen steril und pyrogenfrei ist oder in Einheitsdosisform vorliegt und

(2) Zusammensetzungen mit 1-(4-Nitrophenyl)-3-nitro-4-cyano-oder -carboxamidpyrazol ausgeschlossen sind.

6. Arthropodicide, pflanzennematodicide oder wurmabtreibende Zubereitung, umfassend mindestens eine Verbindung der allgemeinen Formel I, worin die verschiedenen Symbole die in Anspruch 1 angegebene Bedeutung besitzen, oder ein aus Pestizidgesichtspunkten akzeptables Salz derselben in Verbindung mit einem oder mehreren kompatiblen Verdünnungsmittel(n) oder Träger(n), wobei gilt, daß

(1) wenn
$R^4$ Chlor bedeutet,
Y Nitro entspricht,
Z Methyl darstellt und
$(R^3)_n$ für 4-Nitro steht,
die Zubereitung keine pflanzennematodicide Zubereitung ist und einen pharmazeutisch akzeptablen Hilfsstoff oder ein Futtermittel umfaßt oder im wesentlichen steril und pyrogenfrei ist und in Einheitsdosisform vorliegt, und

(2) Zubereitungen mit 1-(4-Nitrophenyl)-3-nitro-4-pyrazolcarbonitril oder -carboxamid ausgeschlossen sind.

7. Verbindung der allgemeinen Formel I, worin die verschiedenen Symbole die in Anspruch 1 angegebene Bedeutung besitzen, und ihre Salze, wobei die Verbindungen ausgeschlossen sind, bei denen
$R^4$ Chlor,
Y Nitro,
Z Methyl und $(R^3)_n$ 4-Nitro bedeuten, und
$R^4$ Nitro,
Y Cyano oder $CONH_2$,
Z Wasserstoff und
$(R^3)_n$ eine 4-Nitrosubstitution bedeuten,
$R^4$ und Y beide für eine Cyanogruppe stehen, Z Amino bedeutet und $(R^3)_n$ für eine 3- oder 4-Nitro oder 4-Chlorsubstitution steht,
$R^4$ Brom bedeutet, Y Methyl und Z Wasserstoff oder $R^4$ bedeuten und Y Brom und Z Methyl bedeuten und $(R^3)_n$ für eine 2,4-Dinitrophenyl- oder 2,4,6-Trinitrophenylsubstitution steht,
$R^4$ und Y Brom bedeuten und Z für Wasserstoff steht, $R^4$ und Y Chlor bedeuten und Z für Methyl steht und $(R^3)_n$ eine 2,4-Dinitrophenylsubstitution darstellt,
$R^4$ Chlor, Y Brom, Z Methyl und $(R^3)_n$ eine 2,4-Dinitrophenylsubstitution bedeuten,
und wobei die Verbindungen der Formel I ausgeschlossen sind, bei denen
$R^4$, Y bzw. Z Nitro, Methyl bzw. Wasserstoff bedeuten und $(R^3)_n$ 2,4,6-Trinitrophenyl entspricht,
$R^4$, Y bzw. Z Brom, Brom bzw. Wasserstoff bedeuten und $(R^3)_n$ 4-Bromphenyl entspricht,
$(R^3)_n$ 2,4-Dinitrophenyl entspricht und $R^4$, Y bzw. Z Chlor, Methyl bzw. Wasserstoff und Chlor, Brom bzw. Wasserstoff bedeuten,
$(R^3)_n$ für 4-Nitrophenyl steht und $R^4$, Y bzw. Z Brom, Methyl bzw. Wasserstoff, Nitro, Methyl bzw. Wasserstoff und Cyano, Methoxycarbonyl bzw. Amino bedeuten und
$R^4$, Y bzw. Z für Nitro, Methyl bzw. Nitro stehen und $(R^3)_n$ 2,4-Dinitrophenyl bedeutet.

**8.** Verbindung nach Anspruch 7, wobei in der allgemeinen Formel I $(R^3)_n$ eine
2,4,6-Trichlor-,
2,3,5,6-Tetrachlor-,
2-Chlor-4-trifluormethyl-,
2,3,5,6-Tetrafluor-4-trifluormethyl-,
2,6-Dichlor-4-trifluormethylthio-,
2-Chlor-3,5,6-trifluor-4-trifluormethyl-,
2,6-Dichlor-3,5-difluor-4-trifluormethyl-,
2,6-Dichlor-4-nitro-,
2,6-Dichlor-4-trifluormethylsulfinyl-,
2,6-Dichlor-4-methansulfonyl- oder
2,6-Dichlor-4-trifluormethansulfonylsubstitution bedeutet.

**9.** Verbindung nach Anspruch 7, wobei in der allgemeinen Formel I $(R^3)_n$ eine
2,6-Dichlor-4-trifluormethyl- oder
2,6-Dichlor-4-trifluormethoxysubstitution bedeutet.

**10.** Verbindung nach Anspruch 7, nämlich 5-Amino-1-(2,6-dichlor-4-trifluormethylphenyl)-3,4-dicyanopyrazol oder 5-Amino-3-cyano-1-(2,6-dichlor-4-trifluormethylphenyl)-4-methansulfonylpyrazol.

**11.** Verfahren zur Herstellung einer Verbindung der in Anspruch 1 angegebenen allgemeinen Formel I, worin die verschiedenen Symbole die in Anspruch 7 angegebene Bedeutung besitzen, umfassend
(A) wenn die Verbindung der allgemeinen Formel I der allgemeinen Formel (IA)

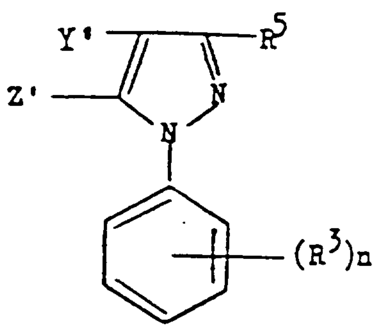

IA

worin Y' für die Cyano- oder Nitrogruppe oder eine Gruppe $RSO_2$, RSO oder RS mit R in der in Anspruch 1 angegebenen Bedeutung, eine gerad- oder verzweigtkettige Alkoxycarbonylgruppe mit 2 bis 7 Kohlenstoffatomen oder eine gerad- oder verzweigtkettige Alkylgruppe mit 1 bis 6 Kohlenstoffatom(en), die nicht-substituiert oder durch ein oder mehrere Halogenatom(e) substituiert ist, steht, Z' die nicht-substituierte Aminogruppe oder eine gerad- oder verzweigtkettige Alkylgruppe mit 1 bis 4 Kohlenstoffatom(en) bedeutet und $R^5$ einem Fluor-, Chlor- oder Bromatom oder der Cyanogruppe entspricht,
entspricht,
(i) die Reaktion einer Verbindung der allgemeinen Formel:

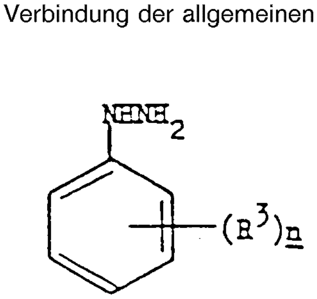

II

worin $R^3$ und n die in Anspruch 1 angegebene Bedeutung besitzen,
oder eines Säureadditionssalzes derselben mit (1), wenn $R^5$ in der Verbindung der allgemeinen Formel IA für ein Fluor-, Chlor- oder Bromatom steht, einer Verbindung der allgemeinen

Formel:

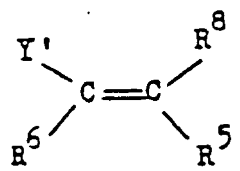

III

worin Y' und $R^5$ die oben angegebene Bedeutung besitzen, $R^6$ für die Cyanogruppe oder eine gerad- oder verzweigtkettige Alkanoylgruppe mit 2 bis 5 Kohlenstoffatomen steht und $R^8$ einer gerad- oder verzweigtkettigen Alkoxygruppe mit 1 bis 4 Kohlenstoffatom(en), der Hydroxygruppe oder einem Fluor-, Chlor- oder Bromatom entspricht, oder

(2) wenn $R^5$ in der Verbindung der allgemeinen Formel IA für die Cyanogruppe steht, Y' die Cyanogruppe bedeutet und Z' die nicht-substituierte Aminogruppe darstellt, mit Tetracyanoethylen,

gegebenenfalls Isolieren des durch Umsetzung einer Verbindung der allgemeinen Formel II mit einer Verbindung der allgemeinen Formel III oder Tetracyanoethylen gebildeten Zwischenprodukts der allgemeinen Formel:

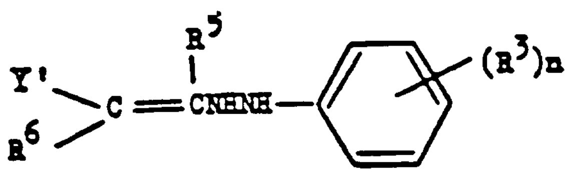

V

worin $R^3$, n, $R^5$, $R^6$ und Y' die oben angegebene Bedeutung besitzen,

vor seiner Cyclisierung zu der Verbindung der allgemeinen Formel IA;

(B) wenn Z' in der allgemeinen Formel IA für die nicht-substituierte Aminogruppe steht,

ein Umsetzen einer Verbindung der allgemeinen Formel $Y'CH_2CN$ mit einer Verbindung der allgemeinen Formel II in Gegenwart einer Verbindung der allgemeinen Formel $R^7C(R^o)_3$ mit $R^7$ gleich einer gerad- oder verzweigtkettigen Alkylgruppe mit 1 bis 4 Kohlenstoffatom(en), die nicht-substituiert oder durch ein oder mehrere Halogenatom(e) substituiert ist, oder einer Cycloalkylgruppe mit 3 bis 6 Kohlenstoffatomen und $R^0$ gleich einer Alkoxygruppe in einem inerten organischen Lösungsmittel bei einer Temperatur von Umgebungstemperatur bis Rückflußtemperatur;

(C) wenn Z' in der allgemeinen Formel IA für die nicht-substituierte Aminogruppe steht und $R^5$ die Cyanogruppe bedeutet,

die Umsetzung einer Verbindung der allgemeinen Formel:

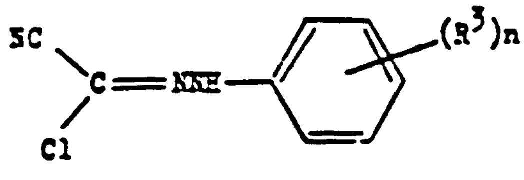

IV

worin $R^3$ und n die in Anspruch 1 angegebene Bedeutung besitzen,

mit einer Verbindung der allgemeinen Formel $Y'CH_2CN$ mit Y' in der oben angegebenen Bedeutung und

ein Herstellen weiterer Verbindungen der allgemeinen Formel I durch die Umwandlung - wie oben beschrieben - eines oder mehrerer Substituenten Y, Z, $R^3$ und $R^4$ oder diesen entsprechenden Substituenten in Substituenten Y, Z, $R^3$ und $R^4$ in der in Anspruch 1 angegebenen Bedeutung, und im Falle von Z gleich der Carboxygruppe, gegebenenfalls Umwandeln einer Verbindung der allgemeinen Formel I in ein Salz derselben.

**12.** Zwischenproduktverbindungen der allgemeinen Formel I, worin Y für das Wasserstoffatom, die Formyl- oder Carboxygruppe, eine gerad- oder verzweigtkettige Alkanoylgruppe mit 2 bis 6 Kohlenstoffatomen, die durch ein oder mehrere Halogenatom(e) substituiert ist, die Dithiogruppe (die zwei Pyrazolringe verbindet), die Aminogruppe, die $-SO_2Cl$-Gruppe, eine gerad- oder verzweigtkettige Carboxyalkylgruppe mit 2 bis 6 Kohlenstoffatomen steht, Z die Carbamoylgruppe oder eine gerad- oder verzweigtkettige Alkoxycarbonylgruppe mit 2 bis 7 Kohlenstoffatomen oder die Diphenoxycarbonylaminogruppe bedeutet, die $(R^3)_n$-Substitution die in Anspruch 2 oder 3 angegebene Bedeutung besitzt und $R^4$ die Amino-, Hydroxymethyl-, Carboxy- oder Carbamoylgruppe oder eine gerad- oder verzweigtkettige Alkoxycarbonyl- oder Alkoxycarbonylaminogruppe mit 2 bis 7 Kohlenstoffatomen bedeutet.

**13.** Verbindung der allgemeinen Formel I nach Anspruch 1 oder ein aus Pestizidgesichtspunkten akzeptables Salz derselben zur Verwendung bei der Herstellung eines Medikaments zur Behandlung einer Gliederfüßler- oder Wurminfektion.

**Patentansprüche für folgende Vertragsstaaten : AT, ES**

**1.** Verfahren zur Herstellung einer Verbindung der allgemeinen Formel:

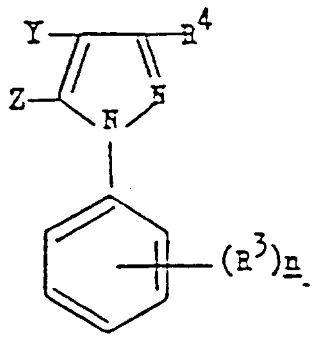

I

worin Y für ein Halogenatom, die Cyano- oder Nitrogruppe oder eine Gruppe $RSO_2$, RSO oder RS mit R gleich einer nicht-substituierten oder durch ein oder mehr Halogenatom(e) substituierten gerad- oder verzweigtkettigen Alkylgruppe mit 1 bis 6 Kohlenstoffatom(en), einer Cycloalkylgruppe mit 3 bis 5 Kohlenstoffatomen, einer gerad- oder verzweigtkettigen Alkenylgruppe mit 2 bis 6 Kohlenstoffatomen oder für die Thiocyanatgruppe, die nicht-substituierte oder durch eine oder mehr gerad- oder verzweigtkettige Alkylgruppe(n), die gleich oder verschieden sein können und 1 bis 6 Kohlenstoffatom(e) enthält (enthalten), substituierte Sulfamoylgruppe, die nicht-substituierte oder durch 1 oder 2 gerad- oder verzweigtkettige Alkylgruppe(n), die gleich oder verschieden sein können und 1 bis 6 Kohlenstoffatom(e) enthalten, substituierte Carbamoylgruppe, eine gerad- oder verzweigtkettige Alkoxycarbonylgruppe mit 2 bis 7 Kohlenstoffatomen, eine gerad- oder verzweigtkettige Alkanoylgruppe mit 2 bis 7 Kohlenstoffatomen oder eine gerad- oder verzweigtkettige Alkylgruppe mit 1 bis 6 Kohlenstoffatom(en), die nicht-substituiert oder durch ein oder mehr Halogenatom(e) substituiert ist, steht,

Z das Wasserstoffatom oder die Aminogruppe $-NR^1R^2$ mit $R^1$ und $R^2$, die gleich oder verschieden sein können, jeweils gleich einem Wasserstoffatom oder einer gerad- oder verzweigtkettigen Alkylgruppe (mit 1 bis 6 Kohlenstoffatom(en), die nicht-substituiert oder durch eine gerad- oder verzweigtkettige Alkoxycarbonylgruppe mit 2 bis 5 Kohlenstoffatomen substituiert ist), einer Cycloalkylgruppe mit 3 bis 6 Kohlenstoffatomen, einer Formylgruppe, einer gerad- oder verzweigtkettigen Alkanoylgruppe (mit 2 bis 7 Kohlenstoffatomen oder zusammen gleich einem 5- oder 6-gliedrigen cyclischen Imid mit dem Stickstoffatom, an dem sie hängen, wobei sie selbst nicht-substituiert oder durch ein oder mehrere Halogenatom(e) substituiert sind) oder einer Cycloalkylcarbonylgruppe (mit 4 bis 7 Kohlenstoffatomen) oder einer gerad- oder verzweigtkettigen Alkoxycarbonylgruppe (mit 2 bis 7 Kohlenstoffatomen, wobei sie selbst nicht-substituiert oder durch ein oder mehrere Halogenatom(e) substituiert sind) oder eine gerad- oder verzweigtkettige Alkylsulfenylaminogruppe mit 1 bis 4 Kohlenstoffatom(en), eine gerad- oder verzweigtkettige Alkoxymethylenaminogruppe mit 2 bis 5 Kohlenstoffatomen, die nicht-substituiert oder am Methylen durch eine gerad- oder verzweigtkettige Alkylgruppe mit 1 bis 4 Kohlenstoffatom(en) substituiert ist, oder ein Halogenatom, eine gerad- oder verzweigtkettige Alkylgruppe mit 1 bis 4 Kohlenstoffatom(en), die Carboxygruppe oder eine gerad- oder verzweigtkettige Alkylthio-, Alkylsulfinyl-

oder Alkylsulfonylgruppe mit 1 bis 6 Kohlenstoffatom(en), die nicht-substituiert oder durch ein oder mehrere Halogenatom(e) substituiert ist, oder eine gerad- oder verzweigtkettige Trialkylsilylmethylgruppe mit 1 bis 6 Kohlenstoffatom(en) in der jeweiligen Alkylgruppe, wobei die Alkylgruppen gleich oder verschieden sein können, eine Trialkylsilylgruppe mit 1 bis 6 Kohlenstoffatom(en) in der jeweiligen Alkylgruppe, wobei die Alkylgruppen gleich oder verschieden sein können, oder die Cyano- oder Nitrogruppe bedeutet,

$R^3$ ein Halogenatom, eine gerad- oder verzweigtkettige Alkyl- oder Alkoxygruppe mit 1 bis 4 Kohlenstoffatom(en), die nicht-substituiert oder durch ein oder mehrere Halogenatom(e) substituiert ist, eine gerad- oder verzweigtkettige Alkylthio- oder Alkylsulfinylgruppe mit 1 bis 4 Kohlenstoffatom(en), die durch ein oder mehrere Halogenatom(e) substituiert ist, die Nitro- oder Cyanogruppe oder eine gerad- oder verzweigtkettige Alkylsulfonylgruppe mit 1 bis 4 Kohlenstoffatom(en), die nicht-substituiert oder durch ein oder mehrere Halogenatom(e) substituiert ist, darstellt,

$R^4$ einem Halogenatom, einer Cyano- oder Nitrogruppe entspricht und

n eine ganze Zahl von 1 bis einschließlich 5 bedeutet,

und im Falle, daß Z für eine Carboxygruppe steht, ihre Salze mit unter Pestizidgesichtspunkten akzeptablen Basen,

vorausgesetzt, daß

(A) $R^4$, Y und Z nicht gleichzeitig 3 Gruppen derselben Art, ausgewählt aus

(i) Nitro,

(ii) Cyano und

(iii) Halogen, bedeuten und

wobei die Verbindungen ausgeschlossen sind, bei denen

$R^4$ Chlor,

Y Nitro,

Z Methyl und $(R^3)_n$ 4-Nitro bedeuten, und

$R^4$ Nitro,

Y Cyano oder $CONH_2$,

Z Wasserstoff und

$(R^3)_n$ eine 4-Nitrosubstitution bedeuten,

$R^4$ und Y beide für eine Cyanogruppe stehen, Z Amino bedeutet und $(R^3)_n$ für eine 3- oder 4-Nitro- oder 4-Chlorsubstitution steht,

$R^4$ Brom bedeutet, Y Methyl und Z Wasserstoff bedeuten oder $R^4$ und Y Brom und Z Methyl bedeuten und $(R^3)_n$ für eine 2,4-Dinitrophenyl- oder 2,4,6-Trinitrophenylsubstitution steht,

$R^4$ und Y Brom bedeuten und Z für Wasserstoff steht, $R^4$ und Y Chlor bedeuten und Z für Methyl steht und $(R^3)_n$ eine 2,4-Dinitrophenylsubstitution darstellt,

$R^4$ Chlor, Y Brom, Z Methyl und $(R^3)_n$ eine 2,4-Dinitrophenylsubstitution bedeuten,

und wobei die Verbindungen der Formel I ausgeschlossen sind, bei denen

$R^4$, Y bzw. Z Nitro, Methyl bzw. Wasserstoff bedeuten und $(R^3)_n$ 2,4,6-Trinitrophenyl entspricht,

$R^4$, Y bzw. Z Brom, Brom bzw. Wasserstoff bedeuten und $(R^3)_n$ 4-Bromphenyl entspricht,

$(R^3)_n$ 2,4-Dinitrophenyl entspricht und $R^4$, Y bzw. Z Chlor, Methyl bzw. Wasserstoff und Chlor, Brom bzw. Wasserstoff bedeuten,

$(R^3)_n$ für 4-Nitrophenyl steht und $R^4$, Y bzw. Z Brom, Methyl bzw. Wasserstoff, Nitro, Methyl bzw. Wasserstoff und Cyano, Methoxycarbonyl bzw. Amino bedeuten und

$R^4$, Y bzw. Z für Nitro, Methyl bzw. Nitro stehen und $(R^3)_n$ 2,4-Dinitrophenyl bedeutet,

welches Verfahren die folgenden Schritte umfaßt:

(A) wenn die Verbindung der allgemeinen Formel I der allgemeinen Formel (IA)

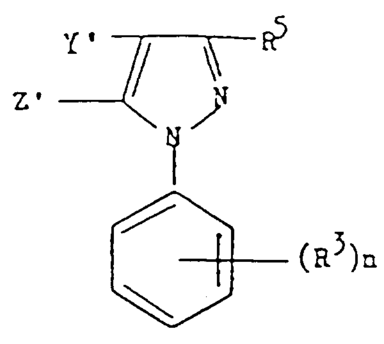

IA

worin Y' für die Cyano- oder Nitrogruppe oder eine Gruppe $RSO_2$, RSO oder RS mit R in der oben angegebenen Bedeutung, eine gerad- oder verzweigtkettige Alkoxycarbonylgruppe mit 2 bis 7 Kohlenstoffatomen oder eine gerad- oder verzweigtkettige Alkylgruppe mit 1 bis 6 Kohlenstoffatom(en), die nicht-substituiert oder durch ein oder mehrere Halogenatom(e) substituiert ist, steht, Z' die nicht-substituierte Aminogruppe oder eine gerad- oder verzweigtkettige Alkylgruppe mit 1 bis 4 Kohlenstoffatom(en) bedeutet und $R^5$ einem Fluor-, Chlor- oder Bromatom oder der Cyanogruppe entspricht,

entspricht,

(i) die Umsetzung einer Verbindung der allgemeinen Formel:

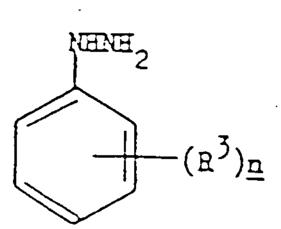

II

worin $R^3$ und n die oben angegebene Bedeutung besitzen,

oder eines Säureadditionssalzes derselben mit (1), wenn $R^5$ in der Verbindung der allgemeinen Formel IA für ein Fluor-, Chlor- oder Bromatom steht, einer Verbindung der allgemeinen Formel:

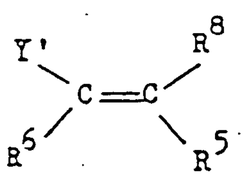

III

worin Y' und $R^5$ die oben angegebene Bedeutung besitzen, $R^6$ für die Cyanogruppe oder eine gerad- oder verzweigtkettige Alkanoylgruppe mit 2 bis 5 Kohlenstoffatomen steht und $R^8$ einer gerad- oder verzweigtkettigen Alkoxygruppe mit 1 bis 4 Kohlenstoffatom(en), der Hydroxygruppe oder einem Fluor-, Chlor- oder Bromatom entspricht, oder

(2) wenn $R^5$ in der Verbindung der allgemeinen Formel IA für die Cyanogruppe steht, Y' die Cyanogruppe bedeutet und Z' die nicht-substituierte Aminogruppe darstellt, mit Tetracyanoethylen,

gegebenenfalls Isolieren des durch Umsetzung einer Verbindung der allgemeinen Formel II mit einer Verbindung der allgemeinen Formel III oder Tetracyanoethylen gebildeten Zwischenprodukts der allgemeinen Formel:

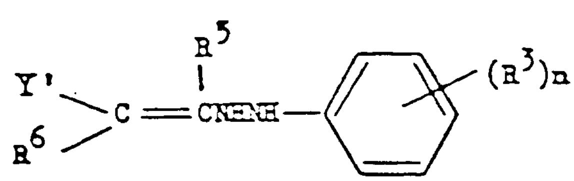

V

worin $R^3$, n, $R^5$, $R^6$ und Y' die oben angegebene Bedeutung besitzen,

vor seiner Cyclisierung zu der Verbindung der allgemeinen Formel IA;

(B) wenn Z' in der allgemeinen Formel IA für die nicht-substituierte Aminogruppe steht,

ein Umsetzen einer Verbindung der allgemeinen Formel $Y'CH_2CN$ mit einer Verbindung der allgemeinen Formel II in Gegenwart einer Verbindung der allgemeinen Formel $R^7C(R^o)_3$ mit $R^7$ gleich einer gerad- oder verzweigtkettigen Alkylgruppe mit 1 bis 4 Kohlenstoffatom(en), die nicht-substituiert oder durch ein oder mehrere Halogenatom(e) substituiert ist, oder einer Cycloalkylgruppe

mit 3 bis 6 Kohlenstoffatomen und $R^0$ gleich einer Alkoxygruppe in einem inerten organischen Lösungsmittel bei einer Temperatur von Umgebungstemperatur bis Rückflußtemperatur;
(C) wenn Z' in der allgemeinen Formel IA für die nicht-substituierte Aminogruppe steht und $R^5$ die Cyanogruppe bedeutet,
die Umsetzung einer Verbindung der allgemeinen Formel:

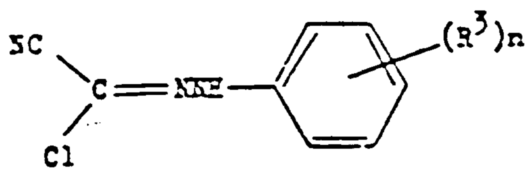

IV

worin $R^3$ und n die oben angegebene Bedeutung besitzen,
mit einer Verbindung der allgemeinen Formel $Y'CH_2CN$ mit Y' in der oben angegebenen Bedeutung und
ein Herstellen weiterer Verbindungen der allgemeinen Formel I durch die Umwandlung - wie oben beschrieben - eines oder mehrerer Substituenten Y, Z, $R^3$ und $R^4$ oder diesen entsprechenden Substituenten in Substituenten Y, Z, $R^3$ und $R^4$ in der oben angegebenen Bedeutung, und im Falle von Z gleich der Carboxygruppe, gegebenenfalls Umwandeln einer Verbindung der allgemeinen Formel I in ein Salz derselben.

**2.** Verfahren nach Anspruch 1, wobei in der allgemeinen Formel I $(R^3)_n$ eine
2,4,6-Trichlor-,
2,3,5,6-Tetrachlor-,
2-Chlor-4-trifluormethyl-,
2,3,5,6-Tetrafluor-4-trifluormethyl-,
2,6-Dichlor-4-trifluormethylthio-,
2-Chlor-3,5,6-trifluor-4-trifluormethyl-,
2,6-Dichlor-3,5-difluor-4-trifluormethyl-,
2,6-Dichlor-4-nitro-,
2,6-Dichlor-4-trifluormethylsulfinyl-,
2,6-Dichlor-4-methansulfonyl- oder
2,6-Dichlor-4-trifluormethansulfonylsubstitution bedeutet.

**3.** Verfahren nach Anspruch 1, wobei in der allgemeinen Formel I $(R^3)_n$ eine
2,6-Dichlor-4-trifluormethyl- oder
2,6-Dichlor-4-trifluormethoxysubstitution bedeutet.

**4.** Verfahren nach Anspruch 1 oder 3, wobei die Verbindung der allgemeinen Formel I
5-Amino-1-(2,6-dichlor-4-trifluormethylphenyl)-3,4-dicyanopyrazol oder
5-Amino-3-cyano-1-(2,6-dichlor-4-trifluormethylphenyl)-4-methansulfonylpyrazol bedeutet.

**5.** Arthropodicide, pflanzennematocide oder wurmabtreibende Zubereitung, umfassend mindestens eine Verbindung der allgemeinen Formel I,
worin Y für ein Halogenatom, die Cyano- oder Nitrogruppe oder eine Gruppe $RSO_2$, RSO oder RS mit R gleich einer nicht-substituierten oder durch ein oder mehrere Halogenatom(e) substituierten gerad- oder verzweigtkettigen Alkylgruppe mit 1 bis 6 Kohlenstoffatom(en), einer Cycloalkylgruppe mit 3 bis 5 Kohlenstoffatomen, einer gerad- oder verzweigtkettigen Alkenylgruppe mit 2 bis 6 Kohlenstoffatomen oder für die Thiocyanatgruppe, die nicht-substituierte oder durch ein oder mehr gerad- oder verzweigtkettige Alkylgruppen, die gleich oder verschieden sein können und 1 bis 6 Kohlenstoffatom(e) enthält (enthalten), substituierte Sulfamoylgruppe, die nicht-substituierte oder durch 1 oder 2 gerad- oder verzweigtkettige Alkylgruppen, die gleich oder verschieden sein können und 1 bis 6 Kohlenstoffatom(e) enthalten, substituierte Carbamoylgruppe, eine gerad- oder verzweigtkettige Alkoxycarbonylgruppe mit 2 bis 7 Kohlenstoffatomen, eine gerad- oder verzweigtkettige Alkanoylgruppe mit 2 bis 7 Kohlenstoffatomen oder eine gerad- oder verzweigtkettige Alkylgruppe mit 1 bis 6 Kohlenstoffatom(en), die nicht-substituiert oder durch ein oder mehrere Halogenatom(e) substituiert ist,

steht,

Z das Wasserstoffatom oder die Aminogruppe $-NR^1R^2$ mit $R^1$ und $R^2$, die gleich oder verschieden sein können, jeweils gleich einem Wasserstoffatom oder einer gerad- oder verzweigtkettigen Alkylgruppe (mit 1 bis 6 Kohlenstoffatom(en), die nicht-substituiert oder durch eine gerad- oder verzweigtkettige Alkoxycarbonylgruppe mit 2 bis 5 Kohlenstoffatomen substituiert ist), einer Cycloalkylgruppe mit 3 bis 6 Kohlenstoffatomen, einer Formylgruppe, einer gerad- oder verzweigtkettigen Alkanoylgruppe (mit 2 bis 7 Kohlenstoffatomen oder zusammen gleich einem 5- oder 6-gliedrigen cyclischen Imid mit dem Stickstoffatom, an dem sie hängen, wobei sie selbst nicht-substituiert oder durch ein oder mehrere Halogenatom(e) substituiert sind) oder einer Cycloalkylcarbonylgruppe (mit 4 bis 7 Kohlenstoffatomen) oder einer gerad- oder verzweigtkettigen Alkoxycarbonylgruppe (mit 2 bis 7 Kohlenstoffatomen, wobei sie selbst nicht-substituiert oder durch ein oder mehrere Halogenatom(e) substituiert sind) oder eine gerad- oder verzweigtkettige Alkylsulfenylaminogruppe mit 1 bis 4 Kohlenstoffatom(en), eine gerad- oder verzweigtkettigea Alkoxymethylenaminogruppe mit 2 bis 5 Kohlenstoffatomen, die nicht-substituiert oder am Methylen durch eine gerad- oder verzweigtkettige Alkylgruppe mit 1 bis 4 Kohlenstoffatom(en) substituiert ist, oder ein Halogenatom, eine gerad- oder verzweigtkettige Alkylgruppe mit 1 bis 4 Kohlenstoffatom(en), die Carboxygruppe oder eine gerad- oder verzweigtkettige Alkylthio-, Alkylsulfinyl- oder Alkylsulfonylgruppe mit 1 bis 6 Kohlenstoffatom(en), die nicht-substituiert oder durch ein oder mehrere Halogenatom(e) substituiert ist, oder eine gerad- oder verzweigtkettige Trialkylsilylmethylgruppe mit 1 bis 6 Kohlenstoffatom(en) in der jeweiligen Alkylgruppe, wobei die Alkylgruppen gleich oder verschieden sein können, eine Trialkylsilylgruppe mit 1 bis 6 Kohlenstoffatom(en) in der jeweiligen Alkylgruppe, wobei die Alkylgruppen gleich oder verschieden sein können, oder die Cyano- oder Nitrogruppe bedeutet,

$R^3$ ein Halogenatom, eine gerad- oder verzweigtkettige Alkyl- oder Alkoxygruppe mit 1 bis 4 Kohlenstoffatom(en), die nicht-substituiert oder durch ein oder mehrere Halogenatom(e) substituiert ist, eine gerad- oder verzweigtkettige Alkylthio- oder Alkylsulfinylgruppe mit 1 bis 4 Kohlenstoffatom(en), die durch ein oder mehrere Halogenatom(e) substituiert ist, die Nitro- oder Cyanogruppe oder eine gerad- oder verzweigtkettige Alkylsulfonylgruppe mit 1 bis 4 Kohlenstoffatom(en), die nicht-substituiert oder durch ein oder mehrere Halogenatom(e) substituiert ist, darstellt,

$R^4$ einem Halogenatom, einer Cyano- oder Nitrogruppe entspricht und

n eine ganze Zahl von 1 bis einschließlich 5 bedeutet,

und im Falle, daß Z für eine Carboxygruppe steht, ihre Salze mit unter Pestizidgesichtspunkten akzeptablen Basen,

vorausgesetzt, daß

(A) $R^4$, Y und Z nicht gleichzeitig 3 Gruppen derselben Art, ausgewählt aus

(i) Nitro,

(ii) Cyano und

(iii) Halogen, bedeuten;

oder ein aus Pestizidgesichtspunkten akzeptables Salz derselben in Verbindung mit einem oder mehreren kompatiblen Verdünnungsmittel(n) oder Träger(n), wobei gilt, daß

(1) wenn

$R^4$ für Chlor,

Y für Nitro,

Z für Methyl und

$(R^3)_n$ für 4-Nitro stehen die Zubereitung keine

pflanzennematocide Zubereitung ist und einen pharmazeutisch akzeptablen Hilfsstoff oder ein Futtermittel umfaßt oder im wesentlichen steril und pyrogenfrei ist oder in Einheitsdosisform vorliegt und

(2) die Zubereitungen mit 1-(4-Nitrophenyl)-3-nitro-4-pyrazolcarbonitril oder -carboxamid ausgeschlossen sind.

**6.** Verfahren zur Bekämpfung von Gliederfüßler-, Pflanzenfadenwurm- oder Wurmschädlingen an bestimmten Orten oder Stellen durch Behandeln des betreffenden Orts bzw. der betreffenden Stelle mit einer wirksamen Menge einer Verbindung der allgemeinen Formel I, worin die verschiedenen Symbole die in Anspruch 5 angegebene Bedeutung besitzen, oder eines aus Pestizidgesichtspunkten akzeptablen Salzes derselben in Verbindung mit einem oder mehreren Verdünnungsmittel(n) oder Träger(n), wobei gilt, daß

(1) wenn $R^4$ für Chlor,

Y für Nitro,

Z für Methyl und
$(R^3)_n$ für 4-Nitro stehen,

die Zubereitung einen pharmazeutisch akzeptablen Hilfsstoff oder ein Futtermittel umfaßt oder im wesentlichen steril und pyrogenfrei ist oder in einer Einheitsdosisform vorliegt, und
(2) Zubereitungen mit 1-(4-Nitrophenyl)-3-nitro-4-cyano- oder -carboxamidpyrazol und
(3) ein Verfahren zur Behandlung des menschlichen oder tierischen Körpers, das durch einen Arzt oder Tierarzt als Therapie durchgeführt wird, ausgeschlossen sind.

**7.** Verfahren nach Anspruch 1, wobei das Ausgangsmaterial aus einer ein Zwischenprodukt darstellenden Verbindung der allgemeinen Formel I, worin Y für das Wasserstoffatom, die Formyl- oder Carboxygruppe, eine gerad- oder verzweigtkettige Alkanoylgruppe mit 2 bis 6 Kohlenstoffatomen, die durch ein oder mehrere Halogenatom(e) substituiert ist, die Dithiogruppe (die zwei Pyrazolringe verbindet), die Aminogruppe, die $-SO_2Cl$-Gruppe, eine gerad- oder verzweigtkettige Carboxyalkylgruppe mit 2 bis 6 Kohlenstoffatomen steht, Z die Carbamoylgruppe oder eine gerad- oder verzweigtkettige Alkoxycarbonylgruppe mit 2 bis 7 Kohlenstoffatomen oder die Diphenoxycarbonylaminogruppe bedeutet, die $(R^3)_n$-Substitution die in Anspruch 2 oder 3 angegebene Bedeutung besitzt und $R^4$ die Amino-, Hydroxmethyl-, Carboxy- oder Carbamoylgruppe oder eine gerad- oder verzweigtkettige Alkoxycarbonyl- oder Alkoxycarbonylaminogruppe mit 2 bis 7 Kohlenstoffatomen bedeutet.

## Revendications

### Revendications pour les Etats contractants suivants : BE, CH, DE, FR, GB, GR, IT, LI, LU, NL, SE

**1.** Un procédé de lutte contre les arthropodes et les nématodes attaquant les plantes, et contre les helminthes, caractérisé en ce que l'on traite le lieu où se trouvent les dits arthropodes, nématodes ou helminthes avec une quantité efficace d'un composé de formule générale

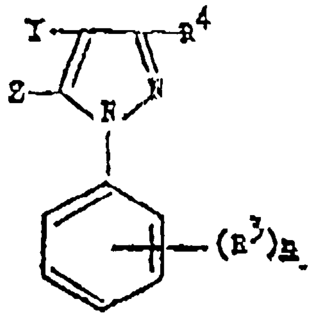

(I)

dans laquelle:
Y représente:
Un atome d'halogène,
un radical cyano, ou
un radical nitro, ou
Un radical $RSO_2$, RSO, ou RS, dans lesquels
R représente
un radical alkyl, linéaire ou ramifié, pouvant contenir de 1 à 6 atomes de carbone, éventuellement substitué par un ou plusieurs atomes d'halogènes ou,
Un radical cycloalkyl pouvant contenir de 3 à 5 atomes de carbone, ou,
Un radical alkènyl, linéaire ou ramifié, pouvant contenir de 2 à 6 atomes de carbone, ou,
Y représente:
Un radical thiocyanato ou,
un radical sulphamoyl substitué ou non par un ou deux radicaux alkyl linéaires ou ramifiés, identiques ou différents et pouvant contenir de 1 à 6 atomes de carbone,
Un radical carbamoyl, qui peut être substitué ou non par un ou deux radicaux alkyl, linéaires ou ramifiés, qui pouvent être identiques ou différents et contenir de 1 à 6 atomes de carbone ou,
Un radical alkoxycarbonyl, linéaire ou ramifié, pouvant contenir de 2 à 7 atomes de carbone, ou,
Un radical alkanoyl linéaire ou ramifié pouvant contenir de 2 à 7 atomes de carbone ou,
Un radical alkyl, linéaire ou ramifié, contenant de 1 à 6 atomes de carbone, substitué ou non par un

ou plusieurs atomes d'halogènes
Z représente:
Un atome d'hydrogène ou,
Un radical amino $-NR^1R^2$ dans lequel $R^1$ et $R^2$ , qui peuvent être identiques ou différents représentent chacun:
Un atome d'hydrogène ou,
Un radical alkyl, linéaire ou ramifié, contenant de 1 à 6 atomes de carbone, et qui est substitué ou non par un ou plusieurs radicaux alkoxycarbonyl, linéaires ou ramifiés, contenant de 2 à 5 atomes de carbone,
Un radical cycloalkyl contenant de 3 à 6 de carbone ou,
un radical formyl,
Un radical alkanoyl linéaire ou ramifié (pouvant contenir de 2 à 7 atomes de carbone, ou former une imide cyclique à 5 ou 6 chaînons avec l'atome d'azote auquel ils sont reliés, eux-mêmes étant substitués ou non substitués par un ou plusieurs atomes d'halogènes) ou,
Un radical cycloalkylcarbonyl pouvant contenir de 4 à 7 atomes de carbone ou,
Un radical alkoxycarbonyl, linéaire ou ramifié, pouvant contenir de 2 à 7 atomes de carbone qui sont eux-mêmes substitués ou non par un ou plusieurs atomes d'halogènes ou,
Z représente
Un radical alkylsulphénylamino, linéaire ou ramifié, contenant de 1 à 4 atomes de carbone,
un radical alkoxyméthylèneamino, linéaire ou ramifié, contenant de 2 à 5 atomes de carbone, substitué ou non sur le radical méthylène par un radical alkyl, linéaire ou ramifié, contenant de 1 à 4 atomes de carbone ou
Un atome d'halogène ou
un radical alkyl, linéaire ou ramifié, contenant de 1 à 4 atomes de carbone,
un radical carboxy ou,
un radical alkylthio, alkylsulphinyl ou alkylsulphonyl, linéaire ou ramifié, pouvant contenir de 1 à 6 atomes de carbone, et qui peut être substitué ou non par un ou plusieurs atomes d'halogènes ou représente,
un radical trialkylsilylmethyl, linéaire ou ramifié, contenant de 1 à 6 atomes de carbone et dans lequel chaque radical alkyl contient de 1 à 6 atomes de carbone et peut être identique ou différent,
un radical trialkylsilyl contenant de 1 à 6 atomes de carbone et dans lequel chaque radical alkyl peut être identique ou différent ou,
un radical nitro ou
un radical cyano:
$R^3$ représente:
un atome d'halogène,
un radical alkyl ou alkoxy, linéaire ou ramifié, contenant de 1 à 4 atomes de carbone, éventuellement substitué par un ou plusieurs atomes d'halogènes ou,
un radical thioalkyl ou thiosulphinyl, linéaire ou ramifié, contenant de 1 à 4 atomes de carbone, et qui est substitué par un ou plusieurs atomes d'halogènes ou,
un radical nitro ou
un radical cyano ou,
un radical alkylsulphonyl, linéaire ou ramifié, contenant de 1 a 4 atomes de carbone, substitué ou non par un ou plusieurs atomes d'halogènes.
$R^4$ représente:
Un atome d'halogène ou
Un radical nitro ou
Un radical cyano
n est un nombre entier pouvant prendre toutes les valeurs de 1 à 5,
et, lorsque Z représente un radical carboxy, les sels de ces différents composés qui sont obtenus par action sur des bases acceptables pour donner des composés antiparasitaires,
A condition que:
A) $R^4$, Y, et Z ne représentent pas simultanément 3 radicaux appartenant à l'une des mêmes familles suivantes :
( i ) nitro
( ii ) cyano,et
( iii ) halogène

B) Et à l'exclusion des méthodes de traitements des humains ou des animaux pratiqués par des médecins ou des vétérinaires à titre thérapeutique.

**2.** Un procédé selon la revendication 1) dans la formule générale ( I ) de laquelle $(R^3)_n$ représente les substituants suivants:
2, 4, 6- trichloro,
2, 3, 5, 6- tétrachloro,
2- chloro- 4- trifluoromethyl,
2, 3, 5, 6, -tétrafluoro-4-trifluoromethyl,
2, 6-dichloro-4- trifluoromethylthio,
2-chloro-3, 5, 6- trifluoro-4- trifluoromethyl
2, 6- dichloro-3, 5- difluoro-4- trifluoromethyl
2, 6- dichloro-4- nitro,
2, 6-dichloro-4- trifluoromethylsulphinyl,
2, 6-dichloro-4- methanesulphonyl ou,
2, 6-dichloro-4- trifluoro méthanesulphonyl.

**3.** Un procédé selon la revendication 1) dans la formule générale de laquelle $(R^3)_n$ représente un radical :
2, 6- dichloro-4- trifluoromethyl ou,
2, 6- dichloro-4- trifluoromethoxy

**4.** Un procédé selon, l'une quelconque des revendications 1 ou 3 dans laquelle le composé de formule ( I ) est:
Le: 5- amino-1- (2, 6- dichloro-4- trifluoromethyl phenyl)-3, 4- dicyano pyrazole, ou
Le: 5-amino-3- cyano-1- (2, 6- dichloro-4- trifluoromethyl phenyl)-4- methane sulphonyl pyrazole.

**5.** L'utilisation comme insecticides ou nématicides pour les plantes ou comme anthelminthiques de compositions contenant comme matière active au moins un composé répondant a la formule générale ( I ), dans laquelle la signification des différents symboles est celle donnée dans la revendication 1), ou de l'un des sels de ces composés utilisable comme antiparasitaire, en association avec un ou plusieurs diluants ou charges convenables, sous réserve que:
(1) Lorsque :
$R^4$ représente un atome de chlore,
Y représente un radical nitro, et
Z représente un radical methyl, et
$(R^3)_n$ représente un radical 4- nitro,
la composition contienne des adjuvants ou des aliments acceptables sur le plan pharmaceutique, ou soit essentiellement stérile et ne contienne pas de substance pyrogène, ou se présente sous forme d'une dose unitaire; et que,
(2) Ces compositions excluent celles qui contiennent comme matière active le 1-(4-nitrophenyl)-3-nitro-4- cyano ou -carboxamide pyrazole

**6.** Une composition insecticide ou nématicide pour les plantes ou une composition anthelminthique contenant comme matière active au moins un composé répondant a la formule générale ( I ), dans laquelle la signification des différents symboles est celle donnée dans la revendication 1), ou de l'un des sels de ces composés utilisable comme antiparasitaire, en association avec un ou plusieurs diluants ou charges convenables, sous réserve que:
(1) Lorsque :
$R^4$ représente un atome de chlore,
Y représente un radical nitro, et
Z représente un radical methyl, et
$(R^3)_n$ représente un radical 4- nitro,
la composition ne soit pas destinée à être utilisée comme nématicide pour les cultures et contienne des adjuvants ou des aliments acceptables sur le plan pharmaceutique, ou soit essentiellement stérile et ne contienne pas de substance pyrogène, ou se présente sous forme d'une dose unitaire; et que,
(2) Ces compositions excluent celles qui contiennent comme matière active le 1-(4-nitrophenyl)-3-nitro-4- pyrazole- carbonitrile ou carboxamide.

**7.** Un composé répondant a la formule générale I, dans laquelle la signification des différents symboles est celle donnée dans la revendication 1), ou de l'un des sols de ces composés a l'exclusion des composés dans lesquels:

$R^4$ représente le chlore,
Y représente un radical nitro, et
Z représente un radical methyl, et
$(R^3)_n$ représente un radical 4- nitro,

et de ceux dans lesquels

$R^4$ représente un radical nitro,
Y représente un radical cyano ou $CONH_2$, et
Z représente un atome d'hydrogène et
$(R^3)_n$ représente un radical 4- nitro,

et de ceux dans lesquels:

$R^4$ et Y représentent tous les deux un radical cyano, Z représente un radical amino et $(R^3)_n$ représente un radical 3- ou 4- nitro ou 4- chloro

et de ceux dans lesquels:

$R^4$ représente un atome de brome, Y représente un radical methyl et Z représente un atome d'hydrogène ou de ceux dans lesquels $R^4$ et Y représente un atome de brome et Z représente un radical methyl et $(R^3)_n$ représente un radical 2, 4-dinitrophenyl ou 2, 4, 6- trinitrophenyl

et de ceux dans lesquels:

$R^4$ et Y représentent un atome de brome et Z représente un atome d'hydrogène,ou $R^4$ et Y représentent un atome de chlore et Z représente un radical methyl, et $(R^3)_n$ représente un radical 2,4 dinitro phenyl

et de ceux dans lesquels:

$R^4$ représente un atome de chlore, Y représente un atome de brome et Z représente un radical methyl et $(R^3)_n$ représente un radical 2, 4- dinitro phenyl,

et à l'exclusion des composés de formule I dans laquelle:

$R^4$ , Y et Z représentent respectivement des radicaux nitro, methyl et un atome d'hydrogène et $(R^3)_n$ représente un radical 2, 4, 6- trinitrophenyl;

et de ceux dans lesquels:

$R^4$ , Y et Z représentent respectivement des atomes de brome, de brome, et d'hydrogène et $(R^3)_n$ représente un radical 4- bromophenyl

et de ceux dans lesquels:

$(R^3)_n$ représente un radical 2,4, dinitrophenyl et $R^4$ , Y et Z représentent respectivement un atome de chlore,un radical methyl ou un atome d'hydrogène; un atome de chlore de brome et d'hydrogène

et de ceux dans lesquels:

$(R^3)_n$ représente un radical 4- nitrophenyl et $R^4$ , Y et Z représentent respectivement le brome, un radical methyl et un atome d'hydrogène;ou des radicaux nitro, methyl, et un atome d'hydrogène; des radicaux cyano, methoxycarbonyl et amino;

et de ceux dans lesquels:

$R^4$ Y et Z représentent respectivement des radicaux nitro, methyl et nitro et $(R^3)_n$ représente un radical 2, 4- dinitrophenyl

**8.** Un composé selon la revendication 7) dans laquelle $(R^3)_n$ représente les substituants suivants:

2, 4, 6- trichloro,
2, 3, 5, 6- tétrachloro,
2- chloro-4- trifluoromethyl,
2, 3, 5, 6- tétrafluoro-4- trifluoromethyl,
2, 6-di chloro-4- trifluoromethylthio,
2, chloro-3, 5, 6- trifluoro-4- trifluoromethyl,
2, 6-di chloro-3, 5- difluoro-4- trifluoromethyl,
2, 6-di chloro-4- nitro,
2, 6-di chloro-4- trifluoromethylsulphinyl,
2, 6-di chloro-4- methanesulphonyl,
2, 6-di chloro-4- trifluoromethanesulphonyl.

**9.** Un composé selon la revendication 7) caractérisé en ce que $(R^3)_n$ représente les substituants suivants:
2, 6-di chloro-4- trifluoromethyl
2, 6-di chloro-4- trifluoromethoxy

**10.** Un compose selon la revendication 7) caractérisé en ce que le dit composé est le:
5- amino-1-(2, 6-di chloro-4- trifluoromethylphenyl)-3, 4 di-cyano pyrazole, ou le
5- amino-3- cyano-1-(2, 6-di chloro-4- trifluoromethylphenyl)-4- methane sulphonylepyrazole.

**11.** Un procédé de préparation des composés de fomule générale ( I ) décrite dans la revendication 1), et dans laquelle les différents symboles ont les définitions données dans la revendication 7), caractérisé en ce que:
( A ) Lorsque le composé de formule générale( I ) correspond à la formule générale

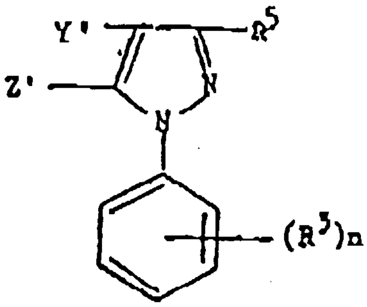

( IA )

dans laquelle
Y' représente
un radical nitro ou cyano ou
un radical $RSO_2$, RSO ou RS, dans laquelle R est défini comme dans la revendication 1)
un radical alkoxycarbonyl, linéaire ou ramifié, contenant de 2 a 7 atomes de carbone, ou
un radical alkyl, linéaire ou ramifié, contenant de 1 à 6 atomes de carbone, éventuellement substitué par un ou plusieurs atomes d'halogènes,
Z' représente
un radical amino non substitué ou
un radical alkyl, linéaire ou ramifié, contenant de 1 à 4 atomes de carbone, et
$R^5$ représente
un atome de fluor,
de chlore, ou
de brome, ou
un radical cyano,
le dit procédé comprend les étapes suivantes:
( 1 ) Faire réagir un composé de formule générale ( II )

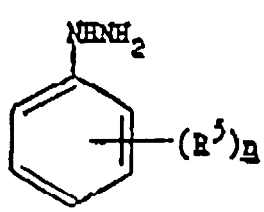

( II )

dans laquelle :
$R^3$ et n ont la définition de la revendication 1),
ou un sel d'addition sur un acide de ces composés avec:
( 1 ) Lorsque $R^5$ représente dans le composé de formule générale ( IA ), un atome de fluor, de brome ou de chlore,
un composé de formule générale ( III )

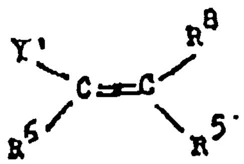

( III )

11

dans laquelle Y' et $R^5$ ont la définition donnée ci-dessus,

$R^6$ représente

un radical cyano ou

un radical alkanoyl, linéaire ou ramifié, contenant de 2 à 5 atomes de carbone , et $R^8$ représente

un radical alkoxy, linéaire ou ramifié, contenant de 1 à 4 atomes de carbone,

un radical hydroxy ou

un atome de fluor, de chlore ou de brome, ou

( 2 ) Lorsque dans le composé de formule générale ( IA )

$R^5$ , représente un radical cyano

Y' représente un radical cyano et

Z' représente un radical amino non substitué,

avec le tétracyanoéthylène,

et en isolant, éventuellement, avant sa cyclisation en un composé de formule IA, le composé intermédiaire de formule générale ( V )

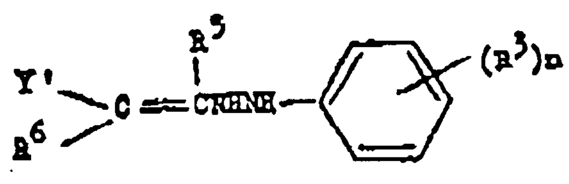

( V )

dans laquelleR$^3$, n, $R^5$, $R^6$, et Y' ont la définition donnée ci-dessus, obtenu par action d'un composé de formule générale ( II ) avec un composé de formule générale ( III ) , ou avec du tétracyano-éthylène;

( B ) Lorsque Z' dans la formule générale ( IA ) représente le radical amino non substitué,

en faisant réagir un composé de formule générale $Y'CH_2CN$ avec un composé de formule générale ( II ) en présence d'un composé de formule générale $R^7C(R^0)_3$ dans laquelle

$R^7$ représente

un radical alkyl, linéaire ou ramifié, contenant de 1 à 4 atomes de carbone, éventuellement substitué par un ou plusieurs atomes d'halogènes ou

un radical cycloalkyl contenant de 3 à 6 atomes de carbone et,

$R^0$ représente un radical alkoxy,

dans un solvant organique inerte à une température comprise entre la température ambiante et la température de reflux du mélange.

( C ) Lorsque Z' dans la formule générale ( IA ) représente un radical amino non substitué et que $R^5$ représente un radical cyano,

en faisant réagir un composé de formule générale ( IV )

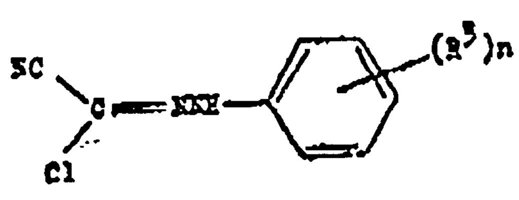

( IV )

dans laquelle $R^3$ et n ont la définition donnée dans la revendication 1 avec un un composé de formule générale $Y'CH_2CN$ dans laquelle Y' a la définition donnée ci-dessus ;

La préparation d'autres composés de formule générale ( I ) peut être réalisée par conversion, comme indiqué précédemment, de un ou plusieurs des substituants Y, Z, $R^3$ et $R^4$ ou des substituants leurs correspondants, en des substituants Y, Z, $R^3$ et $R^4$ tels que définis dans la revendication 1) et,

lorsque Z représente un radical carboxy, en transformant éventuellement le composé de formule générale ( I ) en l'un de ses sels.

**12.** Des composés intermédiaires de formule générale ( I ) dans laquelle Y représente:
un atome d'hydrogène,
le radical formyl ou carboxy,
un radical alkanoyl, linéaire ou ramifié, contenant de 2 à 6 atomes de carbone,et qui est substitué par un ou plusieurs atomes d'halogènes
le radical dithio (qui réunit deux cycles pyrazole) ,
le radical amino,
le radical $-SO_2Cl$,
un radical carboxyalkyl, linéaire ou ramifié, contenant de 2 à 6 atomes de carbone ,
Z représente
un radical carbamoyl ou
un radical alkoxycarbonyl , linéaire ou ramifié, contenant de 2 à 7 atomes de carbone, ou
le radical diphenoxy carbonyl amino,
$(R^3)_n$ a la définition donnée dans les revendications 2 ou 3 et
$R^4$ représente:
un radical amino un radical hydroxymethyl
un radical carboxy ou carbamoyl
un radical alkoxycarbonyl ou alkoxycarbonylamino, linéaire ou ramifié,
contenant de 2 à 7 atomes de carbone

**13.** L'utilisation d'un composé de formule générale ( I ) tel que défini dans la revendication 1), ou d'un sel de l'un de ces composés acceptable pour des utilisations antiparasitaires, pour la fabrication d'un médicament utilisable pour le traitement des infections dues aux arthropodes ou aux helminthes

**Revendications pour les Etats contractants suivants : AT, ES**

**1.** Un procédé de préparation de composés répondant à la formule générale ( I )

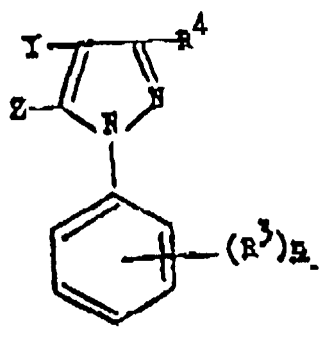

( I )

dans laquelle:
Y représente:
Un atome d'halogène,
un radical cyano, ou
un radical nitro, ou
Un radical $RSO_2$, RSO, ou RS, dans lesquels
R représente
un radical alkyl, linéaire ou ramifié, pouvant contenir de 1 à 6 atomes de carbone, éventuellement

substitué par un ou plusieurs atomes d'halogènes ou,

Un radical cycloalkyl pouvant contenir de 3 à 5 atomes de carbone, ou,

Un radical alkènyl, linéaire ou ramifié, pouvant contenir de 2 à 6 atomes de carbone, ou,

Y représente:

Un radical thiocyanato ou,

Un radical sulphamoyl substitué ou non par un ou deux radicaux alkyl linéaires ou ramifiés, identiques ou différents et pouvant contenir de 1 à 6 atomes de carbone,

Un radical carbamoyl, qui peut être substitué ou non par un ou deux radicaux alkyl, linéaires ou ramifiés, qui peuvent être identiques ou différents et contenir de 1 à 6 atomes de carbone ou,

Un radical alkoxycarbonyl, linéaire ou ramifié, pouvant contenir de 2 à 7 atomes de carbone, ou,

Un radical alkanoyl linéaire ou ramifié pouvant contenir de 2 à 7 atomes de carbone ou,

Un radical alkyl, linéaire ou ramifié, contenant de 1 à 6 atomes de carbone, substitué ou non par un ou plusieurs atomes d'halogènes

Z représente:

Un atome d'hydrogène ou,

Un radical amino $-NR^1R^2$ dans lequel $R^1$ et $R^2$, qui peuvent être identiques ou différents représentent chacun:

Un atome d'hydrogène ou,

Un radical alkyl, linéaire ou ramifié, contenant de 1 à 6 atomes de carbone,et qui est substitué ou non par un ou plusieurs radicaux alkoxycarbonyl, linéaires ou ramifiés, contenant de 2 à 5 atomes de carbone,

Un radical cycloalkyl contenant de 3 à 6 atomes de carbone ou,

un radical formyl,

Un radical alkanoyl linéaire ou ramifié ( pouvant contenir de 2 à 7 atomes de carbone, ou former une imide cyclique a 5 ou 6 chaînons avec l'atome d'azote auquel ils sont reliés, eux-mêmes étant substitués ou non substitués par un ou plusieurs atomes d'halogènes ) ou,

Un radical cycloalkylcarbonyl pouvant contenir de 4 à 7 atomes de carbone ou,

Un radical alkoxycarbonyl, linéaire ou ramifié, pouvant contenir de 2 à 7 atomes de carbone qui sont eux-mêmes substitués ou non par un ou plusieurs atomes d'halogènes ou,

Z représente

Un radical alkylsulphénylamino, linéaire ou ramifié, contenant de 1 à 4 atomes de carbone,

un radical alkoxyméthylèneamino, linéaire ou ramifié, contenant de 2 à 5 atomes de carbone, substitué ou non sur le radical méthylène par un radical alkyl, linéaire ou ramifié, contenant de 1 à 4 atomes de carbone ou

Un atome d'halogène ou

un radical alkyl, linéaire ou ramifié, contenant de 1 à 4 atomes de carbone,

un radical carboxy ou,

un radical alkylthio, alkylsulphinyl ou alkylsulphonyl, linéaire ou ramifié, pouvant contenir de 1 à 6 atomes de carbone, et qui peut être substitué ou non par un ou plusieurs atomes d'halogènes ou représente,

un radical trialkylsilylmethyl, linéaire ou ramifié, contenant de 1 à 6 atomes de carbone et dans lequel chaque radical alkyl contient de 1 à 6 atomes de carbone et peut être identique ou différent,

un radical trialkylsilyl contenant de 1 à 6 atomes de carbone et dans lequel chaque radical alkyl peut être identique ou différent ou

un radical nitro ou

un radical cyano:

$R^3$ représente:

un atome d'halogène,

un radical alkyl ou alkoxy, linéaire ou ramifié , contenant de 1 à 4 atomes de carbone, éventuellement substitué par un ou plusieurs atomes d'halogènes ou,

un radical thioalkyl ou thiosulphinyl, linéaire ou ramifié, contenant de 1 à 4 atomes de carbone, et qui est substitué par un ou plusieurs atomes d'halogènes ou,

un radical nitro ou

un radical cyano ou,

un radical alkylsulphonyl, linéaire ou ramifié, contenant de 1 à 4 atomes de carbone, substitué ou non par un ou plusieurs atomes d'halogènes.

$R^4$ représente:

Un atome d'halogène ou

Un radical nitro ou

Un radical cyano

n est un nombre entier pouvant prendre toutes les valeurs de 1 a 5,

et, lorsque Z représente un radical carboxy, les sels de ces différents composés qui sont obtenus par action sur des bases acceptables pour donner des composés antiparasitaires,

A condition que:

A) $R^4$, Y, et Z ne représentent pas simultanément 3 radicaux appartenant à l'une des mêmes familles suivantes :

( I ) nitro

( II ) cyano,et

( III ) halogène

à l'exclusion des composés dans lesquels:

$R^4$ représente le chlore,

Y représente un radical nitro, et

Z représente un radical methyl, et

$(R^3)_n$ représente un radical 4- nitro,

et de ceux dans lesquels

$R^4$ représente un radical nitro,

Y représente un radical cyano ou $CONH_2$, et

Z représente un atome d'hydrogène et

$(R^3)_n$ représente un radical 4- nitro,

et de ceux dans lesquels:

$R^4$ et Y représentent tous les deux un radical cyano, Z représente un radical amino et $(R^3)_n$ représente un radical 3- ou 4- nitro ou 4- chloro

et de ceux dans lesquels:

$R^4$ représente un atome de brome, Y représente un radical methyl et Z représente un atome d'hydrogène ou de ceux dans lesquels $R^4$ et Y représente un atome de brome et Z représente un radical methyl et $(R^3)_n$ représente un radical 2, 4-dinitrophenyl ou 2, 4, 6- trinitrophenyl

et de ceux dans lesquels:

$R^4$ et Y représentent un atome de brome et Z représente un atome d'hydrogène,ou $R^4$ et Y représentent un atome de chlore et Z représente un radical methyl, et $(R^3)_n$ représente un radical 2,4 dinitro phenyl

et de ceux dans lesquels:

$R^4$ représente un atome de chlore, Y représente un atome de brome et Z représente un radical methyl et $(R^3)_n$ représente un radical 2, 4- dinitro phenyl,

et à l'exclusion des composés de formule I dans laquelle:

$R^4$ , Y et Z représentent respectivement des radicaux nitro, methyl et un atome d'hydrogène et $(R^3)_n$ représente un radical 2, 4, 6- trinitrophenyl;

et de ceux dans lesquels:

$R^4$ , Y et Z représentent respectivement des atomes de brome, de brome, et d'hydrogène et $(R^3)_n$ représente un radical 4- bromophenyl

et de ceux dans lesquels:

$(R^3)_n$ représente un radical 2, 4, dinitrophenyl et $R^4$ , Y et Z représentent respectivement un atome de chlore,un radical methyl ou un atome d'hydrogène; un atome de chlore de brome et d'hydrogène

et de ceux dans lesquels:

$(R^3)_n$ représente un radical 4- nitrophenyl et $R^4$ , Y et Z représentent respectivement le brome, un radical methyl et un atome d'hydrogène;ou des radicaux nitro, methyl, et un atome d'hydrogène; des radicaux cyano, methoxycarbonyl et amino;

et de ceux dans lesquels:

$R^4$ , Y et Z représentent respectivement des radicaux nitro, methyl et nitro et $(R^3)_n$ représente un radical 2, 4- dinitrophenyl

caractérisé en ce que:

( A ) Lorsque le composé de formule générale( I ) correspond a la formule générale

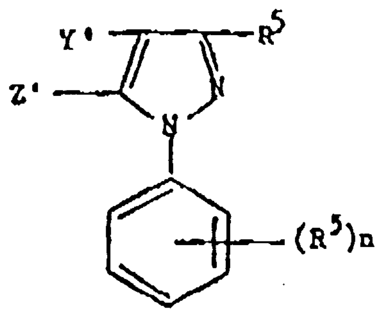

( IA )

dans laquelle
Y' représente
un radical nitro ou cyano ou
un radical $RSO_2$, RSO ou RS, dans laquelle R est défini comme dans la revendication 1)
un radical alkoxycarbonyl, linéaire ou ramifié, contenant de 2 à 7 atomes de carbone, ou
un radical alkyl, linéaire ou ramifié, contenant de 1 à 6 atomes de carbone, éventuellement substitué par un ou plusieurs atomes d'halogènes,
Z' représente
un radical amino non substitué ou
un radical alkyl, linéaire ou ramifié, contenant de 1 à 4 atomes de carbone, et
$R^5$ représente
un atome de fluor,
de chlore, ou
de brome , ou
un radical cyano,
le dit procédé comprend les étapes suivantes:
( 1 ) Faire réagir un composé de formule générale ( II )

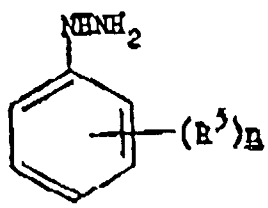

( II )

dans laquelle :
$R^3$ et n ont la définition de la revendication 1),
ou un sel d'addition sur un acide de ces composés avec:
( 1 ) Lorsque $R^5$ représente, dans le composé de formule générale ( IA ), un atome de fluor, de brome ou de chlore,
un composé de formule générale ( III )

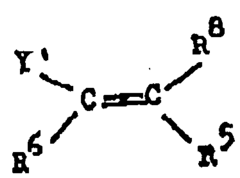

( III )

dans laquelle Y' et $R^5$ ont la définition donnée ci-dessus,
$R^6$ représente
un radical cyano ou
un radical alkanoyl, linéaire ou ramifié, contenant de 2 à 5 atomes de carbone, et

$R^8$ représente

un radical alkoxy, linéaire ou ramifié, contenant de 1 à 4 atomes de carbone,

un radical hydroxy ou

un atome de fluor, de chlore ou de brome, ou

( 2 ) Lorsque dans le composé de formule générale ( IA )

$R^5$ , représente un radical cyano

Y' représente un radical cyano et

Z' représente un radical amino non substitué,

avec le tétracyanoéthylène,

et en isolant, éventuellement, avant sa cyclisation en un composé de formule IA, le composé intermédiaire de formule générale ( V )

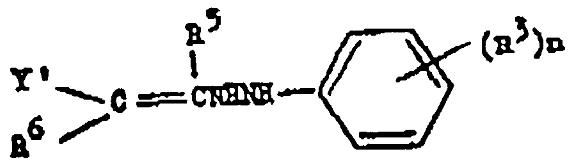

( V )

dans laquelle$R^3$, n, $R^5$, $R^6$, et Y' ont la définition donnée ci-dessus,

obtenu par action d'un composé de formule générale ( II ) avec un composé de formule générale ( III ) , ou avec du tétracyano- éthylène;

( B ) Lorsque Z' dans la formule générale ( IA ) représente le radical amino non substitué,

en faisant réagir un composé de formule générale $Y'CH_2CN$ avec un composé de formule générale ( II ) en présence d'un composé de formule générale $R^7C(R^0)_3$ dans laquelle

$R^7$ représente

un radical alkyl, linéaire ou ramifié, contenant de 1 à 4 atomes de carbone, éventuellement substitué par un ou plusieurs atomes d'halogènes ou

un radical cycloalkyl contenant de 3 à 6 atomes de carbone et,

$R^0$ représente un radical alkoxy,

dans un solvant organique inerte à une température comprise entre la température ambiante et la température de reflux du mélange.

( C ) Lorsque Z' dans la formule générale ( IA ) représente un radical amino non substitué et que $R^5$ représente un radical cyano,

en faisant réagir un composé de formule générale ( IV )

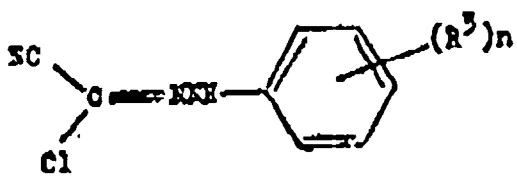

( IV )

dans laquelle $R^3$ et n ont la définition donnée dans la revendication 1 avec un un composé de formule générale $Y'CH_2CN$ dans laquelle Y' a la définition donnée ci-dessus ;

La préparation d'autres composés do formule générale ( I ) peut être réalisée par conversion, comme indiqué précédemment, de un ou plusieurs des substituants Y, Z, $R^3$ et $R^4$ ou des substituants leurs correspondants, en des substituants Y, Z, $R^3$ et $R^4$ tels que définis dans la revendication 1) et,

lorsque Z représente un radical carboxy, en transformant éventuellement le composé de formule générale ( I ) en l'un de ses sels.

**2.** Un procédé selon la revendication 1) dans la formule générale ( I ) dans laquelle le substituant $(R^3)_n$ représente:

2, 4, 6- trichloro,

2, 3, 5, 6- tétrachloro,

2- chloro- 4- trifluoromethyl,

2, 3, 5, 6, -tétrafluoro-4-trifluoromethyl,
2, 6-dichloro-4- trifluoromethylthio,
2-chloro-3, 5, 6- trifluoro-4- trifluoromethyl
2, 6- dichloro-3, 5- difluoro-4- trifluoromethyl
2, 6- dichloro-4- nitro,
2, 6-dichloro-4- trifluoromethylsulphinyl,
2, 6-dichloro-4- methanesulphonyl ou,
2, 6-dichloro-4- trifluoro méthanesulphonyl.

**3.** Un procédé selon la revendication 1) dans la formule générale de laquelle $(R^3)_n$ représente un radical :
2, 6- dichloro-4- trifluoromethyl ou,
2, 6- dichloro-4- trifluoromethoxy

**4.** Un procédé selon, l'une quelconque des revendications 1 ou 3 dans laquelle le composé de formule ( I ) est:
Le: 5- amino-1- (2, 6- dichloro-4- trifluoromethyl phenyl)-3, 4- dicyano pyrazole, ou
Le: 5-amino-3- cyano-1- (2, 6- dichloro-4- trifluoromethyl phenyl)-4- methane sulphonyl pyrazole.

**5.** Une composition insecticide ou nématicide pour les plantes ou une composition anthelminthique contenant comme matière active au moins un composé répondant à la formule générale ( I ),
dans laquelle:
Y représente:
Un atome d'halogène,
un radical cyano, ou
un radical nitro, ou
Un radical $RSO_2$, RSO, ou RS, dans lesquels
R représente
un radical alkyl, linéaire ou ramifié, pouvant contenir de 1 à 6 atomes de carbone, éventuellement substitué par un ou plusieurs atomes d'halogènes ou,
Un radical cycloalkyl pouvant contenir de 3 à 5 atomes de carbone, ou,
Un radical alkènyl, linéaire ou ramifié, pouvant contenir de 2 à 6 atomes de carbone, ou,
Y représente:
Un radical thiocyanato ou,
Un radical sulphamoyl substitué ou non par un ou deux radicaux alkyl linéaires ou ramifiés, identiques ou différents et pouvant contenir de 1 à 6 atomes de carbone,
Un radical carbamoyl, qui peut être substitué ou non par un ou deux radicaux alkyl, linéaires ou ramifiés, qui peuvent être identiques ou différents et contenir de 1 à 6 atomes de carbone ou,
Un radical alkoxycarbonyl, linéaire ou ramifié, pouvant contenir de 2 à 7 atomes de carbone, ou,
Un radical alkanoyl linéaire ou ramifié pouvant contenir de 2 à 7 atomes de carbone ou,
Un radical alkyl, linéaire ou ramifié, contenant de 1 à 6 atomes de carbone, substitué ou non par un ou plusieurs atomes d'halogènes
Z représente:
Un atome d'hydrogène ou,
Un radical amino $-NR^1R^2$ dans lequel $R^1$ et $R^2$ , qui peuvent être identiques ou différents représentent chacun:
Un atome d'hydrogène ou,
Un radical alkyl, linéaire ou ramifié, contenant de 1 à 6 atomes de carbone,et qui est substitué ou non par un ou plusieurs radicaux alkoxycarbonyl, linéaires ou ramifiés, contenant de 2 à 5 atomes de carbone,
Un radical cycloalkyl contenant de 3 à 5 atomes de carbone ou,
un radical formyl,
Un radical alkanoyl linéaire ou ramifié ( pouvant contenir de 2 à 7 atomes de carbone, ou former une imide cyclique à 5 ou 6 chaînons avec l'atome d'azote auquel ils sont reliés, eux-mêmes étant substitués ou non substitués par un ou plusieurs atomes d'halogènes ) ou,
Un radical cycloalkylcarbonyl pouvant contenir de 4 à 7 atomes de carbone ou,
Un radical alkoxycarbonyl, linéaire ou ramifié, pouvant contenir de 2 à 7 atomes de carbone qui sont eux-mêmes substitués ou non par un ou plusieurs atomes d'halogènes ou,
Z représente

un radical alkylsulphénylamino, linéaire ou ramifié, contenant de 1 à 4 atomes de carbone,

un radical alkoxyméthylèneamino, linéaire ou ramifié, contenant de 2 à 5 atomes de carbone, substitué ou non sur le radical méthylène par un radical alkyl, linéaire ou ramifié, contenant de 1 à 4 atomes de carbone ou

Un atome d'halogène ou

un radical alkyl, linéaire ou ramifié, contenant de 1 à 4 atomes de carbone,

un radical carboxy ou,

un radical alkylthio, alkylsulphinyl ou alkylsulphonyl, linéaire ou ramifié, pouvant contenir de 1 à 6 atomes de carbone, et qui peut être substitué ou non par un ou plusieurs atomes d'halogènes ou représente,

un radical trialkylsilylmethyl, linéaire ou ramifié, contenant de 1 à 6 atomes de carbone et dans lequel chaque radical alkyl contient de 1 à 6 atomes de carbone et peut être identique ou différent,

un radical trialkylsilyl contenant de 1 à 6 atomes de carbone et dans lequel chaque radical alkyl peut être identique ou différent ou,

un radical nitro ou

un radical cyano;

$R^3$ représente:

un atome d'halogène,

un radical alkyl ou alkoxy, linéaire ou ramifié , contenant de 1 à 4 atomes de carbone, éventuellement substitué par un ou plusieurs atomes d'halogènes ou,

un radical thioalkyl ou thiosulphinyl, linéaire ou ramifié, contenant de 1 à 4 atomes de carbone, et qui est substitué par un ou plusieurs atomes d'halogènes ou,

un radical nitro ou

un radical cyano ou,

un radical alkylsulphonyl, linéaire ou ramifié, contenant de 1 à 4 atomes de carbone, substitué ou non par un ou plusieurs atomes d'halogènes.

$R^4$ représente:

Un atome d'halogène ou

Un radical nitro ou

Un radical cyano

n est un nombre entier pouvant prendre toutes les valeurs de 1 à 5,

et, lorsque Z représente un radical carboxy, les sels de ces différents composés qui sont obtenus par action sur des bases acceptables pour donner des composés antiparasitaires,

A condition que: A) $R^4$, Y, et Z ne représentent pas simultanément 3 radicaux appartenant à l'une des mêmes familles suivantes :

( I ) nitro

( II ) cyano,et

( III ) halogène

ou de l'un des sels de ces composés utilisable comme antiparasitaire, en association avec un ou plusieurs diluants ou charges convenables, sous réserve que:

(1) Lorsque :

$R^4$ représente le chlore ,

Y représente un radical nitro, et

Z représente un radical methyl, et

$(R^3)_n$ représente un radical 4- nitro,

la composition ne soit pas destinée à être utilisée comme nématicide pour les cultures et contienne des adjuvants ou des aliments acceptables sur le plan pharmaceutique, ou soit essentiellement stérile et ne contienne pas de substance pyrogène, ou se présente sous forme d'une dose unitaire; et que,

(2) Ces compositions excluent celles qui contiennent comme matière active le 1-(4-nitrophenyl)-3- nitro-4- pyrazole- carbonitrile ou carboxamide.

**6.** Un procédé de lutte contre les arthropodes et les nématodes attaquant les plantes, et contre les helminthes, caractérisé en ce que l'on traite le lieu où se trouvent les dits arthropodes, nématodes ou helminthes avec une quantité biologiquement efficace d'un composé de formule générale ( I ) dans laquelle les différents substituants ont la définition donnée dans la revendication 5) ou un sel de ces composés utilisable pour des applications pesticides, associé à une ou plusieurs chages ou diluantsavec lesquels ils sont compatibles, sous la réserve que:

(1) Lorsque :

$R^4$ représente le chlore,

Y représente un radical nitro, et

Z représente un radical methyl, et

$(R^3)_n$ représente un radical 4- nitro,

la composition ne soit pas destinée à être utilisée comme nématicide pour les cultures et contienne des adjuvants ou des aliments acceptables sur le plan pharmaceutique, ou soit essentiellement stérile et ne contienne pas de substance pyrogène, ou se présente sous forme dune dose unitaire; et que,

(2) Ces compositions excluent celles qui contiennent comme matière active le 1-(4-nitrophenyl)-3-nitro-4- cyano ou carboxamide pyrazole

(3) Et à l'exclusiondes procédés de traitement thérapeutiques par des médecins ou des vétérinaires des être humains ou des animaux

**7.** Un procédé de préparation selon la revendication 1) caractérisé en ce que le produit de départ est obtenu à partir d'un intermédiaire de formule générale ( I )

dans laquelle

Y représente:

un atome d'hydrogène ,

le radical formyl ou carboxy,

un radical alkanoyl, linéaire ou ramifié, contenant de 2 à 6 atomes de carbone,et qui est substitué par un ou plusieurs atomes d'halogènes

le radical dithio (qui réunit deux cycles pyrazole) ,

le radical amino ,

le radical $-SO_2Cl$,

un radical carboxyalkyl, linéaire ou ramifié, contenant de 2 à 6 atomes de carbone ,

Z représente

un radical carbamoyl ou

un radical alkoxycarbonyl , linéaire ou ramifié, contenant de 2 à 7 atomes de carbone, ou

le radical diphenoxy carbonyl amino,

$(R^3)_n$ a la définition donnée dans les revendications 2 ou 3 et

$R^4$ représente:

un radical amino

un radical hydroxymethyl

un radical carboxy ou carbamoyl

un radical alkoxycarbonyl ou alkoxycarbonylamino, linéaire ou ramifié, contenant de 2 à 7 atomes de carbone